(19) Europäisches Patentamt
European Patent Office
Office européen des brevets

(11) EP 1 467 815 B1

(12) # EUROPÄISCHE PATENTSCHRIFT

(45) Veröffentlichungstag und Bekanntmachung des Hinweises auf die Patenterteilung:
**26.10.2005 Patentblatt 2005/43**

(21) Anmeldenummer: **03704410.4**

(22) Anmeldetag: **16.01.2003**

(51) Int Cl.7: **B01L 3/00**, B01L 3/14, G01N 33/531

(86) Internationale Anmeldenummer:
**PCT/EP2003/000408**

(87) Internationale Veröffentlichungsnummer:
**WO 2003/059516 (24.07.2003 Gazette 2003/30)**

(54) **REAKTIONSGEFÄSS ZUR DURCHFÜHRUNG VON ARRAY-VERFAHREN**

REACTION VESSEL FOR CARRYING OUT ARRAY PROCESSES

CUVE DE REACTION DESTINEE A LA MISE EN OEUVRE DE PROCEDES D'ANALYSE

(84) Benannte Vertragsstaaten:
**AT BE BG CH CY CZ DE DK EE ES FI FR GB GR HU IE IT LI LU MC NL PT SE SI SK TR**

(30) Priorität: **16.01.2002 DE 10201463**

(43) Veröffentlichungstag der Anmeldung:
**20.10.2004 Patentblatt 2004/43**

(73) Patentinhaber: **Clondiag Chip Technologies GmbH**
**07749 Jena (DE)**

(72) Erfinder:
- **SCHULZ, Torsten**
  **07743 Jena (DE)**
- **ERMANTRAUT, Eugen**
  **07745 Jena (DE)**
- **EHRICHT, Ralf**
  **07749 Jena (DE)**
- **MÖBIUS, Klaus, Peter**
  **07751 Zöllnitz (DE)**
- **WAGNER, Gerd**
  **07743 Jena (DE)**
- **FISCHER, Joachim**
  **07778 Porstendorf (DE)**
- **ELLINGER, Thomas**
  **07743 Jena (DE)**

(74) Vertreter: **Neuefeind, Regina**
**Maiwald Patentanwalts GmbH**
**Elisenhof**
**Elisenstrasse 3**
**80335 München (DE)**

(56) Entgegenhaltungen:
**EP-A- 0 344 578**
**DE-A- 3 717 209**
**US-A- 5 683 875**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 467 815 B1

## Beschreibung

Die Erfindung betrifft ein Reaktionsgefäß, eine Vorrichtung sowie ein Verfahren zum Nachweis von spezifischen Wechselwirkungen zwischen molekularen Target- und Sondenmolekülen.

Biomedizinische Tests basieren häufig auf dem Nachweis einer Wechselwirkung zwischen einem Molekül, das in bekannter Menge und Position vorhanden ist (der molekularen Sonde) und einem nachzuweisenden, unbekannten Molekül bzw. nachzuweisenden, unbekannten Molekülen (den molekularen Ziel- oder Targetmolekülen). Bei modernen Tests sind die Sonden in Form einer Substanzbibliothek auf Trägern, den so genannten Mikro-Arrays oder Chips abgelegt, so dass eine Probe parallel an mehreren Sonden gleichzeitig analysiert werden kann (D. J. Lockhart, E. A. Winzeler, Genomics, gene expression and DNA arrays; Nature 2000, 405, 827-836). Für die Herstellung der Mikro-Arrays werden die Sonden dabei üblicherweise in vorgegebener Art und Weise auf einer geeigneten, beispielsweise in WO 00/12575 beschriebenen Matrix immobilisiert (siehe z.B. US 5,412,087, WO 98/36827) bzw. synthetisch erzeugt (siehe z.B. US 5,143,854).

Der Nachweis einer Wechselwirkung zwischen der Sonde und dem Targetmolekül erfolgt üblicherweise folgendermaßen: Nach der Fixierung der Sonde bzw. der Sonden in vorgegebener Art und Weise an einer bestimmten Matrix in Form eines Mikro-Arrays werden die Targets in einer Lösung mit den Sonden in Kontakt gebracht und unter definierten Bedingungen inkubiert. Infolge der Inkubation findet zwischen Sonde und Target eine spezifische Wechselwirkung statt. Die dabei auftretende Bindung ist deutlich stabiler als die Bindung von Targetmolekülen an Sonden, die für das Targetmolekül nicht spezifisch sind. Zum Entfernen von Targetmolekülen, die nicht spezifisch gebunden worden sind, wird das System mit entsprechenden Lösungen gewaschen oder erwärmt.

Der Nachweis der spezifischen Wechselwirkung zwischen einem Target und seiner Sonde kann dann durch eine Vielzahl von Verfahren erfolgen, die in der Regel von der Art des Markers abhängen, der vor, während oder nach der Wechselwirkung des Targetmoleküls mit dem Mikro-Array in Targetmoleküle eingebracht worden ist. Typischerweise handelt es sich bei solchen Markern um fluoreszierende Gruppen, so dass spezifische Target-Sonden-Wechselwirkungen mit hoher Ortsauflösung und im Vergleich zu anderen herkömmlichen Nachweismethoden, vor allem massensensitiven Methoden, mit geringem Aufwand fluoreszenzoptisch ausgelesen werden können (A. Marshall, J. Hodgson, DNA chips: An array ofpossibilities, Nature Biotechnology 1998, 16, 27-31; G. Ramsay, DNA Chips: State of the art, Nature Biotechnology 1998, 16,, 40-44).

Abhängig von der auf dem Mikro-Array immobilisierten Substanzbibliothek und der chemischen Natur der Targetmoleküle können anhand dieses Testprinzips Wechselwirkungen zwischen Nukleinsäuren und Nukleinsäuren, zwischen Proteinen und Proteinen sowie zwischen Nukleinsäuren und Proteinen untersucht werden (zur Übersicht siehe F. Lottspeich, H. Zorbas, 1998, Bioanalytik, Spektrum Akademischer Verlag, Heidelberg Berlin).

Als Substanzbibliotheken, die auf Mikro-Arrays oder Chips immobilisiert werden können, kommen dabei Antikörper-Bibliotheken, Rezeptor-Bibliotheken, PeptidBibliotheken und Nukleinsäure-Bibliotheken in Frage.

Die Nukleinsäure-Bibliotheken nehmen die mit Abstand wichtigste Rolle ein. Es handelt sich dabei um Mikro-Arrays, auf denen Desoxyribonukleinsäure- (DNA) Moleküle oder Ribonukleinsäure- (RNA) Moleküle immobilisiert sind.

Voraussetzung für die Bindung eines beispielsweise mit einer Fluoreszenzgruppe markierten Targetmoleküls in Form eines DNA- oder RNA-Moleküls an eine Nukleinsäuresonde des Mikro-Arrays ist, dass sowohl Targetmolekül als auch Sondenmolekül in Form einer einzelsträngigen Nukleinsäure vorliegen. Nur zwischen solchen Molekülen kann eine effiziente und spezifische Hybridisierung stattfinden. Einzelsträngige Nukleinsäureziel- und Nukleinsäuresondenmoleküle erhält man in der Regel durch Hitzedenaturierung und optimale Wahl von Parametern wie Temperatur, Ionenstärke und Konzentration helixdestabilisierender Moleküle. Somit wird gewährleistet, dass nur Sonden mit nahezu perfekt komplementären, d.h. einander entsprechenden Sequenzen mit der Zielsequenz gepaart bleiben (A.A. Leitch, T. Schwarzacher, D. Jackson, I. J. Leitch, 1994, In vitro Hybridisierung, Spektrum Akademischer Verlag, Heidelberg Berlin Oxford).

Ein typisches Beispiel für die Verwendung von Mikro-Arrays in biologischen Testverfahren ist der Nachweis von Mikroorganismen in Proben in der biomedizinischen Diagnostik. Dabei macht man sich die Tatsache zunutze, dass die Gene für ribosomale RNA (rRNA) ubiquitär verbreitet sind und über Sequenzabschnitte verfügen, die für die jeweilige Spezies charakteristisch sind. Diese Spezies-charakteristischen Sequenzen werden in Form von einzelsträngigen DNA-Oligonukleotiden auf ein Mikro-Array aufgebracht. Die zu untersuchenden Target-DNA-Moleküle werden zunächst aus der zu untersuchenden Probe isoliert und mit Markern, beispielsweise fluoreszierenden Markern versehen. Anschließend werden die markierten Target-DNA-Moleküle in einer Lösung mit den auf dem Mikro-Array aufgebrachten Sonden inkubiert, unspezifisch auftretende Wechselwirkungen werden durch entsprechende Waschschritte entfernt und spezifische Wechselwirkungen durch fluoreszenzoptische Auswertung nachgewiesen. Auf diese Art und Weise ist es möglich, mit einem einzigen Test in einer Probe gleichzeitig z. B. mehrere Mikroorganismen nachzuweisen. Die Anzahl der nachweisbaren Mikroorganismen hängt bei diesem Testverfahren theoretisch nur von der Anzahl der spezifischen Sonden ab, die auf dem Mikro-Array aufgebracht worden sind.

Zur praktischen Durchführung dieser Tests werden die Mikro-Arrays oder Chips in geschlossenen Kammern fixiert, die über Ein- und Auslässe zum Wechsel der für die Waschschritte und Hybridisierungsschritte notwendigen Flüssigkeiten verfügen.

Solche Systeme sind z.B. in US 6,287,850 und WO 01/02094 beschrieben. In DE 199 40 750 ist ein Träger für Analytbestimmungsverfahren beschrieben, der sich nach leichten konstruktiven Änderungen zur Verwendung für Array-Anwendungen auch im Rahmen dieser Erfindung eignet.

Zur Sequenzanalyse von DNA werden üblicherweise oberflächengebundene DNA-Bibliotheken verwendet, die auf Objektträgern aufgebracht sind. Zur Durchführung der Hybridisierungsreaktion auf diesen Objektträgern werden bislang spezielle Hybridisierungskammern oder Inkubationskammern verwendet. Um die Temperierung und das Vermischen der Hybridisierungslösung in diesen bislang bekannten Kammern zu gewährleisten, ist eine für die verwendete Vorrichtung speziell angepasste und deshalb aufwändige und teure Ausstattung erforderlich.

In DE 101 49 684.2 ist eine Durchflusszelle beschrieben, die zur Durchführung einer PCR sowie von Hybridisierungsreaktionen auf DNA-Chips geeignet ist. Die dort beschriebene Durchflusszelle ist ein komplexes Bauteil, welches mit einer Reihe von technischen Merkmalen versehen ist, die eine Benutzung einer üblicherweise in Laboratorien verwendeten Ausstattung wie beispielsweise einem Thermomixer (Eppendorf, Deutschland, Hamburg) oder einer Laborzentrifuge (Heraeus, Hanau, Deutschland) ausschließen.

In WO 01/02094 ist eine Kartusche beschrieben, die einen DNA-Chip umfasst. In dieser Kartusche kann sowohl eine PCR als auch eine Hybridisierungsreaktion auf einem DNA-Chip durchgeführt werden. In WO 95/33846 ist ein Körper mit einer Vertiefung beschrieben, in die ein Substrat mit Nukleinsäuremolekülen bekannter Sequenz auf definierten Bereichen eingebaut wird. Der Körper weist einen abgeschlossenen Hohlraum auf, in den die Probenflüssigkeit injiziert werden kann. Die Einfüllkanäle werden über Septen abgedichtet und mit geeigneten Einstechkanülen geöffnet, um den Körper bzw. die Kartusche zu befüllen. Die Verwendung der vorstehend beschriebenen Kartuschen erfordert ebenfalls speziell dafür vorgesehene Vorrichtungen.

In US 5,856,174 ist eine miniaturisierte integrierte Nukleinsäurediagnostik-Vorrichtung beschrieben. Diese Vorrichtung ermöglicht das Sammeln einer oder mehrerer Proben, deren Präparierung und die anschließende Durchführung mehrerer Probenanalysen. Eine derartige Vorrichtung dient zur automatischen Durchführung einer DNA-Chip basierten Analyse durch Vereinigung und Miniaturisierung aller anfallenden Schritte auf einer Kartusche. Die Bereitstellung einer derartigen Vorrichtung ist äußerst aufwändig und teuer.

In US 5,545,531 ist ein Verfahren zur Herstellung von Mikrotiterplatten beschrieben, deren Boden ein Wafer ist, der an jeder Stelle, an der sich eine Vertiefung in der Mikrotiterplatte befindet, eine Probenmatrize aufweist. In US 5,874,219 ist ein Verfahren für die konkurrierende Durchführung von biologischen Tests beschrieben, bei dem eine Vielzahl von Reaktionsgefäßen zusammenhängend nebeneinander angeordnet sind und jedes Gefäß mit einer molekularen Probenmatrize versehen ist. Diese biologische Chip-Reaktionsgefäßplatte ist so ausgestaltet, dass die Wechselwirkungen an der molekularen Probenmatrize mit entsprechenden Lesegeräten ausgelesen werden können. Auf diese Weise können eine Reihe von biologischen Proben nebeneinander und parallel mit molekularen Probenmatrizen untersucht werden. Für die Durchführung von einzelnen Tests ist die dort beschriebene Reaktionsgefäßplatte nicht geeignet. Die Herstellung einer derartigen Reaktionsgefäßplatte ist in US 5,545,531 beschrieben.

Anhand des vorstehend beschriebenen Standes der Technik wird deutlich, dass ein großer Bedarf an Vorrichtungen besteht, die zum einen auf einfache und kostengünstige Weise bereitgestellt werden können und zum anderen eine einfache Durchführung von auf Mikro-Arrays basierenden Nachweistests ermöglichen. Insbesondere besteht ein Bedarf an Vorrichtungen zur Durchführung von auf Mikro-Arrays basierenden Tests, die die Verwendung von typischen im Laboralltag verwendeten Geräten und Instrumenten erlaubt. Allgemein besteht ein Bedarf an Geräten zur Durchführung von auf Mikro-Arrays basierenden Tests, die sich durch eine einfache Konstruktion, eine leichte Handhabbarkeit, das Vermeiden von Kontaminationsquellen, eine reproduzierbare Durchführbarkeit der Tests und niedrige Herstellungskosten auszeichnen.

Analysen basierend auf Sonden-Arrays werden somit zum derzeitigen Zeitpunkt in der Regel fluoreszenzoptisch ausgelesen (siehe A. Marshall und J. Hodgson, DNA Chips: An array of possibilities, Nature Biotechnology, 16, 1998, 27-31; G. Ramsay, DNA Chips: State of the Art, Nature Biotechnology, 16, Jan. 1998, 40-44). Nachteilig an den herkömmlichen Detektionsverfahren ist jedoch der teilweise erhebliche technische Aufwand sowie die hohen Kosten, die mit den Nachweisverfahren verbunden sind.

In letzter Zeit wurden eine Reihe von Array-Verfahren entwickelt, die mit relativ geringem technischen Aufwand einen qualitativ und/oder quantitativen Nachweis der Wechselwirkung zwischen Sonden und Targets erlauben.

In DE 100 33 334.6 und WO 02/02810 sind Verfahren zum quantitativen und qualitativen Nachweis von molekularen Wechselwirkungen auf Sonden-Arrays durch zeitlich aufgelöste Fällungsreaktionen sowie die zugehörigen Geräte und Einwegartikel beschrieben.

In WO 99/35499 wird eine Vorrichtung beschrieben, bei der Substanzbibliotheken adressierbar auf einer Scheibe angebracht sind, die einer modernen Compact Disk (CD) ähnlich ist. Durch Rotation der Scheibe kann eine bestimmte Substanz auf der Scheibe angefahren werden. Der Lesekopf des Lesegerätes ist dann entlang des Radius

der Scheibe bewegbar. Die Position der Scheibe kann durch integrierte mit einem Standard CD-Player auslesbare Spuren bestimmt werden. Die Wechselwirkung der Probe mit einer Targetsubstanz kann durch Absorptionsmessungen und Fluoreszenzmessungen im Durch- oder Auflicht erfolgen. Ebenso können magnetische Partikel abgelegt werden, die mit einem magnetischem Lesekopf detektiert werden können. Zur Visualisierung der Wechselwirkungsreaktion wird insbesondere eine Anfärbung durch ein Silberpräzipitat vorgeschlagen, dessen Abscheidung über ein Streptavidin/Biotin-Goldkonjungat vermittelt wird.

In WO 00/72018 wird ein Verfahren zur Silberabscheidung auf Objektträgern zur Visualisierung von Wechselwirkungsreaktionen von Substanzbibliotheken offenbart. Des Weiteren werden dazu notwendige Lesegeräte beschrieben.

Der vorliegende Erfindung liegt somit die Aufgabe zugrunde, eine Vorrichtung zur Durchführung von Array-Verfahren bereitzustellen, die sich durch einfache Konstruktion, leichte Handhabbarkeit und damit kostengünstige Herstellung auszeichnet. Eine weitere Aufgabe der vorliegenden Erfindung ist es, ein Reaktionsgefäß zur Verfügung zu stellen, das in einer derartigen Vorrichtung verwendet werden kann und sich ebenfalls durch eine leichte Handhabbarkeit sowie durch eine Kompatibilität mit üblicherweise in Laboratorien verwendeten Geräten wie beispielsweise Tischzentrifugen und Pipetten auszeichnet. Ferner ist es eine Aufgabe der vorliegenden Erfindung, ein Reaktionsgefäß zur Verfügung zu stellen, die die Durchführung von Mikro-Array-basierten Tests in einem Ein-Kammer-System unter Vermeidung von Kontaminationsquellen erlaubt. Des Weiteren ist eine Aufgabe der vorliegenden Erfindung, eine Vorrichtung zur Durchführung von Array-Verfahren bereitzustellen, die den Einsatz von Detektionsverfahren mit relativ geringem technischen Aufwand erlaubt.

Diese und weitere Aufgaben der vorliegenden Erfindung werden durch die Bereitstellung der in den Patentansprüchen angegebenen Gegenstände gelöst. Bevorzugte Ausführungsformen sind in den Unteransprüchen definiert.

Die Aufgaben werden erfindungsgemäß dadurch gelöst, dass ein Reaktionsgefäß bereitgestellt wird, welches ein Tube ist und eine für ein Tube typische Form und typische Größe und ein Füllvolumen von 100 µl bis 2,5 ml aufweist und bei dem auf einer seiner Grundflächen ein Trägerelement mit darauf auf vorbestimmten Bereichen immobilisierten Sondenmolekülen angeordnet ist.

Die Verwendung des erfindungsgemäßen Reaktionsgefäßes für den Nachweis von spezifischen Wechselwirkungen zwischen molekularen Target- und Sondenmolekülen bietet den wesentlichen Vorteil, dass die Anschaffung von zusätzlichen Geräten bzw. einer zusätzlichen Ausstattung für die Durchführung der Nachweisreaktionen nicht erforderlich ist, da die üblicherweise in Laboren, insbesondere in biologischen Laboren verwendeten Geräte für Standard-Laborreaktionsgefäße, wie beispielsweise Tischzentrifugen und Pipetten, zum Einsatz kommen können. Ein weiterer Vorteil des erfindungsgemäßen Reaktionsgefäßes ist, dass eine getrennte Inkubationskammer überflüssig ist, da das Reaktionsgefäß auch als Hybridisierungskammer dient. Zusätzlich wird die Oberfläche des Trägers mit den darauf immobilisierten Sondenmolekülen durch die für herkömmliche Laborreaktionsgefäße typische Deckelverriegelung, beispielsweise die Safe-Lock-Deckelverriegelung bei Eppendorf-Reaktionsgefäßen, vor Kontaminationen und anderen nachteiligen äußeren Einflüssen geschützt.

Unter Laborreaktionsgefäßen mit einer typischen Form und Größe werden im Rahmen der vorliegenden Erfindung Reaktionsgefäße verstanden, die als Einweg-Reaktionsgefäße, in der Standardausführung 1,5 ml fassend, in, insbesondere biologischen bzw. molekularbiologischen, Laboratorien üblicherweise verwendet werden. Derartige Laborreaktionsgefäße werden auch als Tubes und, nach dem bedeutendsten Hersteller, insbesondere als Eppendorf-Tubes oder "Eppis" (Hamburg, Deutschland) bezeichnet. So werden Laborreaktionsgefäße mit einer typischen Form und Größe von Eppendorf als Standard-Reaktionsgefäße oder Safe-Lock-Reaktionsgefäße angeboten. Selbstverständlich können im Rahmen der vorliegenden Erfindung auch Reaktionsgefäße von Herstellern wie Greiner (Frickenhausen, Deutschland), Millipore (Eschborn, Deutschland), Heraeus (Hanau, Deutschland) und BIOplastics (Landgraaf, Niederlande) sowie anderen Herstellern eingesetzt werden, die eine Form und Größe aufweisen, wie sie für Laborreaktionsgefäße insbesondere von Eppendorf typisch ist. Beispiele für Laborreaktionsgefäße mit einer typischen Form und Größe sind in Abbildung 21 gezeigt.

Unter Labonreaktionsgefäßen typischer Form und Größe werden im Rahmen der vorliegenden Erfindung insbesondere nicht Rundkolben oder andere Kolben wie Erlenmeyerkolben, Bechergläser oder Messzylinder verstanden.

Das erfindungsgemäße Reaktionsgefäß unterscheidet sich von den vorstehenden genannten Reaktionsgefäßen dadurch, dass auf einer seiner Grundflächen ein Trägerelement mit darauf auf vorbestimmten Bereichen immobilisierten Sondenmolekülen angeordnet ist. Ein derartiges Trägerelement mit darauf auf vorbestimmten Bereichen immobilisierten Sondenmolekülen wird im Folgenden auch als Chip oder Affinitätsmatrix bezeichnet. Die vorbestimmten Bereiche auf dem Träger werden im Folgenden auch als Array-Elemente bezeichnet.

Trotz der Modifizierung eines herkömmlichen Laborreaktionsgefäßes durch Einbau eines derartigen Chips weist das Reaktionsgefäß eine für ein Laborreaktionsgefäß typische Form und/oder Größe auf. Das erfindungsgemäße Reaktionsgefäß weist somit eine rotationssymmetrische Form, insbesondere eine zylindrische bzw. im Wesentlichen

zylindrische Form auf. Von den für herkömmliche Laborreaktionsgefäße typischen Formen und damit für das erfindungsgemäße Reaktionsgefäß denkbaren Formen ist ferner eine von der zylindrischen Grundform abweichende konische Form umfasst, wobei die Verjüngung vorzugsweise in Richtung der Affinitätsmatrix auftritt. Typische Formen sind ferner Kombinationen von zylindrischen bzw. im Wesentlichen zylindrischen Bereichen und konischen Bereichen (siehe u.a. Abbildungen 1-4 und 21). Aufgrund der für Laborreaktionsgefäße typischen Form und Größe ist das erfindungsgemäße Reaktionsgefäß insbesondere mit üblichen Tischzentrifugen wie beispielsweise von Herstellern wie Eppendorf oder Heraeus kompatibel, d.h. das erfindungsgemäße Reaktionsgefäß ist zur Zentrifugation in üblichen Tischzentrifugen geeignet. Übliche maximale Außendurchmesser für Standard-Laborreaktionsgefäße und damit auch für das erfindungsgemäße Reaktionsgefäß liegen im Bereich von 0,8 cm bis 2 cm, vorzugsweise 1,0 cm bis 1,5 cm und besonders bevorzugt 1,1 cm bis 1,3 cm. Weitere bevorzugte Außendurchmesser sind bis 0,9 cm, bis 1,2 cm, bis 1,4 cm, bis 1,6 cm und bis 1,7 cm. Die Höhe des erfindungsgemäßen Reaktionsgefäßes beträgt üblicherweise 1,5 cm bis 5,0 cm, vorzugsweise 2,0 cm bis 4,0 cm, besonders bevorzugt 2,5 cm bis 3,5 cm, und am meisten bevorzugt 2,8 cm bis 3,2 cm. Weitere bevorzugte Höhen sind bis 2,6 cm, bis 2,7 cm, bis 2,9 cm, bis 3,0 cm, bis 3,1 cm, bis 3,3 cm und bis 3,4 cm. In speziellen Ausgestaltungen kann die Höhe auch 1,0 cm oder mehr betragen. Das erfindungsgemäße Reaktionsgefäß ist in üblichen Tischzentrifugen zentrifugierbar und kann somit beispielsweise in herkömmlichen Tischzentrifugen wie einer Standard-Tischzentrifuge mit Standard-Rotor von Eppendorf sowie auch in üblichen Racks und Haltern für Reaktionsgefäße wie beispielsweise einem Tube-Rack von Eppendorf eingesetzt werden. Zum Einbringen der zu untersuchenden Probe sowie anderer zur Durchführung der Nachweisreaktion erforderlicher Reagenzien in das Reaktionsgefäß können übliche Pipetten oder Spritzen wie beispielsweise variable und Fixvolumen-Pipetten von Eppendorf verwendet werden.

Die Anordnung des Chips in dem erfindungsgemäßen Reaktionsgefäß ermöglicht die Detektion der Wechselwirkungsreaktion zwischen Target- und Sondenmolekülen durch übliche Verfahren wie beispielsweise durch Fluoreszenzdetektion oder radiochemische Methoden. Als besonders vorteilhaft hat sich die Anwendung von Absorptionsmessungen erwiesen, da diese besonders kostengünstig durchzuführen sind. Eine derartige Absorptionsmessung kann durch Verwendung einer reaktiven Färbemethode, die an den Oberflächenbereichen stattfindet, an denen eine Wechselwirkungsreaktion stattgefunden hat, wesentlich verbessert und verbilligt werden. Hier hat sich insbesondere die Abscheidung von Silber an mit Goldnanokügelchen markierten Targetmolekülen bewährt (siehe DE 100 33 334.6 und WO 02/02810). Zum Nachweis der Silberabscheidung kann ein Gerät verwendet werden, das eine oder mehrere Leuchtdioden beliebiger Emissionswellenlänge als Lichtquelle verwendet und beispielsweise eine CCD-Kamera zur ortsaufgelösten Detektion der Wechselwirkungsreaktion auf den vorbestimmten Bereichen des Chips aufweist.

Zur Beschreibung der vorliegenden Erfindung werden unter anderem folgende Definitionen verwendet:

Unter einem Sonden-Array wird im Rahmen der vorliegenden Erfindung eine Anordnung von molekularen Sonden auf einer Oberfläche verstanden, wobei die Position einer jeden Sonde separat bestimmt ist. Vorzugsweise umfasst der Array definierte Stellen bzw. vorbestimmte Bereiche, so genannte Array-Elemente, die besonders bevorzugt in einem bestimmten Muster angeordnet sind, wobei jedes Array-Element üblicherweise nur eine Spezies an Sonden beinhaltet.

Unter einer Sonde bzw. einem Sondenmolekül wird im Rahmen der vorliegenden Erfindung ein Molekül verstanden, das zum Nachweis anderer Moleküle durch ein bestimmtes, charakteristisches Bindungsverhalten bzw. eine bestimmte Reaktivität verwendet wird. Für die auf dem Array angeordneten Sonden kommt jede Art von Molekülen in Frage, die sich an feste Oberflächen koppeln lassen und eine spezifische Affinität aufweisen. In einer bevorzugten Ausführungsform handelt es sich um Biopolymere aus den Klassen der Peptide, Proteine, Nukleinsäuren und/oder deren Analoga. Besonders bevorzugt sind die Sonden Nukleinsäuren und/oder Nukleinsäureanaloga. Als Nukleinsäuren können sowohl DNA- als auch RNA-Moleküle verwendet werden. Beispielsweise kann es sich bei den

Oligonukleatidsonden um Oligonukleotide mit einer Länge von 10 bis 100 Basen, vorzugsweise 15 bis 50 Basen und besonders bevorzugt von 20 bis 30 Basen Länge handeln, die auf der Array-Oberfläche immobilisiert sind.

Unter einem Target bzw. einem Targetmolekül wird im Rahmen der vorliegenden Erfindung das mit einer molekularen Sonde nachzuweisende Molekül verstanden. Bei einer bevorzugten Ausführungsform der vorliegenden Erfindung handelt es sich bei den zu detektierenden Targets um Nukleinsäuren. Der erfindungsgemäße Sonden-Array kann jedoch analog zum Nachweis von Protein/Sonden-Wechselwirkungen, Antikörper/Sonden-Wechselwirkungen, usw. eingesetzt werden.

Unter einem Array-Element bzw. einem vorbestimmten Bereich wird im Rahmen der vorliegenden Erfindung ein für die Deposition einer molekularen Sonde bestimmtes Areal auf einer Oberfläche verstanden, die Summe aller belegten Array-Elemente ist das Sonden-Array.

Eine Markierung bezeichnet im Rahmen der vorliegenden Erfindung eine detektierbare Einheit, beispielsweise ein Fluorophor oder eine Ankergruppe, an die eine detektierbare Einheit gekoppelt werden kann.

Unter einem Substrat wird im Rahmen der vorliegenden Erfindung ein im Reaktionsmedium gelöst vorliegendes Molekül bzw. eine Kombination von Molekülen verstanden, die mit Hilfe eines Katalysators bzw. eines Kristallisationskeimes und/oder eines umsetzenden Agens lokal abgeschieden wird. Das umsetzende Agens kann beispielsweise ein reduzierenden Agens wie bei der Silberabscheidung oder ein oxidierendes Agens wie bei der Erzeugung eines Farbstoffs durch enzymatische Oxidation sein.

Unter einem Trägerelement bzw. Träger wird im Rahmen der vorliegenden Erfindung ein Festkörper verstanden, auf dem das Sonden-Array aufgebaut ist. Das Trägerelement mit den darauf angeordneten Sonden wird im Folgenden auch als Chip bezeichnet und kann bei speziellen Ausgestaltungen der vorliegenden Erfindung auch ein Grundelement umfassen, auf dem der eigentliche Chip angeordnet ist.

Das Trägerelement kann in dem erfindungsgemäßen Reaktionsgefäß angeordnet sein, indem es einfach in ein Laborreaktionsgefäß eingesetzt bzw. eingeklemmt wird, vorzugsweise indem die Chipfläche derart gestaltet ist, dass sie passgenau in ein Laborreaktionsgefäß, beispielsweise in dessen Deckel, eingesetzt bzw. eingeklemmt werden kann. Alternativ ist eine Grundfläche des Laborreaktionsgefäßes derart abgeflacht, dass das Trägerelement darauf angebracht werden kann. Sollten es technische Gründe erfordern, so kann der Träger bzw. der Chip auch in die Seitenwände des erfindungsgemäßen Reaktionsgefäßes eingebaut werden.

Bei einer vorteilhaften Ausgestaltung weist die Grundfläche, vorzugsweise der Boden des Laborreaktionsgefäßes jedoch eine Aussparung zur Aufnahme des Trägers auf. In diese Aussparung kann der Träger bzw. Chip beispielsweise von innen und/oder von außen eingeklebt und/oder eingeklemmt und/oder geschraubt und/oder geschweißt, insbesondere durch Laser-Schweißen, und/oder eingerastet werden. Bei diesen Ausgestaltungen weist das erfindungsgemäße Reaktionsgefäß eine für ein Laborreaktionsgefäß typische Form und Größe und einen als Einfassung geformten Durchbruch zur Aufnahme der Affinitätsmatrizen, insbesondere von oberflächengebundenen Substanzbibliotheken auf. Beispiele für derartige Ausführungsformen des erfindungsgemäßen Reaktionsgefäßes sind in den Abbildungen 1 bis 4 angegeben. Neben den dort gezeigten Varianten sind selbstverständlich weitere Kombinationen der Art der Befestigung des Trägers denkbar.

Der Einbau des Trägers bzw. Chips von innen hat den Vorteil, dass der Träger bzw. Chip auch bei höherem Innendruck, z.B. bei Verwendung in einer Zentrifuge bzw. bei Erwärmung der Probenflüssigkeit auf Temperaturen nahe des Siedepunktes, im Reaktionsgefäß nicht aus seiner Befestigung nach außen gedrückt werden kann. Allerdings erfordert die Montage einen höheren Aufwand als der Einbau von außen.

Klemmverbindungen bzw. Gewinde bzw. Rasterungen sorgen für eine kraftschlüssige und flüssigkeitsundurchlässige Verbindung zwischen Reaktionsgefäß und Träger bzw. Chip. Durch derartige Varianten werden die Vorteile des Einsetzen des Trägers bzw. Chips von innen in das Reaktionsgefäß mit denen einer vereinfachten Montage vereint. Nachteilig wirkt sich eine weitere Verbindungsstelle, z.B. die Klemmverbindung sowie die höhere Anzahl an Bauteilen aus.

Bei der Herstellung des erfindungsgemäßen Reaktionsgefäßes wird üblicherweise von einem spritzgussgefertigten Standard-Laborreaktionsgefäß insbesondere von einem der vorstehend genannten Hersteller ausgegangen werden. Dieses wird an der Unterseite gekappt und dann in einer speziell dafür vorgesehenen Vorrichtung umgeschmolzen. Ein derartiges Verfahren eignet sich insbesondere für kleinere Stückzahlen. Bei großen Stückzahlen bietet es sich an, das Reaktionsgefäß direkt in einer der vorstehend genannten Ausgestaltungen spritzzugießen.

Um die Affinitätsmatrix von außen vor Dreck zu schützen, ist es vorteilhaft, auf die Unterseite des Reaktionsgefäßes eine Schutzfolie aufzuspannen bzw. aufzukleben, die kurz vor der Verwendung des Reaktionsgefäßes abgezogen wird.

Üblicherweise ist die Grundfläche, auf der das Trägerelement mit den darauf auf vorbestimmten Bereichen immobilisierten Sondenmolekülen angeordnet ist, der Boden des erfindungsgemäßen Reaktionsgefäßes.

Alternativ kann der Träger auch im Deckel des Reaktionsgefäßes angebracht sein. Der Einbau des Trägerelements bzw. der Chipfläche in den Deckel des Reaktionsgefäßes ist insbesondere dann vorteilhaft, wenn die Affinitätsmatrix empfindlich gegenüber den Bedingungen bei einem oder mehreren der Reaktionsschritte zur Vorbereitung und/oder Durchführung der Nachweisreaktion reagiert. Derartige Reaktionsschritte können in dieser Ausgestaltung des erfindungsgemäßen Reaktionsgefäßes in dem aufrecht stehenden Reaktionsgefäß durchgeführt werden, wodurch die Affinitätsmatrix bzw. der Chip nicht mit den Reaktions- und Probenlösungen in Kontakt kommt und so geschützt wird. Zur Durchführung der Nachweisreaktion wird das erfindungsgemäße Reaktionsgefäß dann auf den Deckel gedreht bzw. gestellt, so dass die Probe mit den oberflächengebundenen Sonden in Berührung kommt. Auf diese Weise wird die thermische und chemische Belastungen der Affinitätsmatrix bzw. des Chips verringert.

Das Trägerelement des erfindungsgemäßen Reaktionsgefäßes ist vorzugsweise im Bereich der Detektionsfläche optisch durchlässig und/oder oder nicht fluoreszierend. Unter Detektionsfläche ist der Bereich des Trägerelements zu verstehen, auf dem auf vorbestimmten Bereichen Sondenmoleküle immobilisiert sind. In einer bevorzugten Ausführungsform der Erfindung sind die Sondenmoleküle direkt auf dem Trägerelement aufgebracht, ohne dass das Trägerelement ein weiteres Grundelement umfasst.

In anderen bevorzugten Ausführungsformen sind die Sondenmoleküle auf einem vorzugsweise optisch durchlässigen und/oder nicht fluoreszierenden Chip aufgebracht, der wiederum fest mit einem Grundelement, das vorzugsweise mindestens in dem durch den Chip definierten Detektionsbereich optisch durchlässig und/oder nicht fluoreszierend ist, verbunden ist. Die Dimensionen des Chips sind dabei kleiner als die Dimensionen des Grundelements. In

diesem Fall bilden der die Sondenmoleküle tragende Chip und das Grundelement zusammen das Trägerelement.

Bei einer bevorzugten Ausgestaltung des erfindungsgemäßen Reaktionsgefäßes ist die der Detektionsfläche des Trägerelements gegenüberliegende Grundfläche in dem der Detektionsfläche entsprechenden Bereich ebenfalls optisch durchlässig und/oder nicht fluoreszierend.

Allgemein umfasst ein Sonden-Array gemäß der vorliegenden Erfindung einen Träger, der die Bildung von Arrays mit Sonden auf seiner Oberfläche erlaubt. Ein derartiger Träger kann unter anderem hergestellt werden aus Materialien, ausgewählt aus der Gruppe, bestehend aus Glas, Filtern, elektronischen Vorrichtungen, Polymeren, metallischen Materialien u.dgl. sowie beliebigen Kombinationen dieser Materialien.

Das Trägerelement besteht vorzugsweise aus optisch durchlässigen und/oder nicht fluoreszierenden Materialien. Bei derartigen Materialien handelt es sich beispielsweise um Glas, Borofloat 33 (beispielsweise erhältlich von Schott, Jena, Deutschland), Quarzglas, einkristallines $CaF_2$ (beispielsweise erhältlich von Schott), einkristallines Silizium, Phenylmethylmethacrylat und/oder Polycarbonat

Sind die Sondenmoleküle nicht direkt auf dem Trägerelement, sondern auf einem Chip aufgebracht, besteht der Chip ebenfalls vorzugsweise aus optisch durchlässigen und/oder nicht fluoreszierenden Materialien. Bei den Materialien handelt es sich insbesondere um Glas, Borofloat 33, Quarzglas, einkristallines $CaF_2$, einkristallines Silizium, Phenylmethylmethacrylat und/oder Polycarbonat.

Neben den vorstehend beschriebenen optisch durchlässigen oder nicht fluoreszierenden Materialien für das Trägerelement bzw. der Chip sind herkömmliche Filter-, Keramik-, Metall-, Halbmetall- und/oder Kunststoffmaterialien denkbar. So können beispielsweise auch speziell für DNA-Bibliotheken gefertigte Nylonmembranen als Trägermaterialien eingesetzt werden.

Das Material des Behälters des Reaktionsgefäßes entspricht den üblicherweise für Laborreaktionsgefäße verwendeten Materialien und ist beispielsweise ausgewählt aus der Gruppe bestehend aus Glas, Glaskeramik, kunststoffbeschichtetem Glas und Kunststoffen bzw. organischen Polymeren wie Polypropylen, Polyethylen, Polystyrol, Polycarbonat, PVC, Polymethylmethacrylat, Silikonkunststoff, Kautschuk, Polytetrafluorethylen und/oder Nylon. Für spezielle Ausführungsformen sind als Materialien auch Metalle, insbesondere nicht rostende Stähle, Platin und/oder Aluminium, denkbar.

Das Reaktionsgefäß weist eine für ein Laborreaktionsgefäß typische Größe auf. Typische Füllvolumina liegen im Bereich von 100 µl bis 2,5 ml. Besonders bevorzugt hat das Reaktionsgefäß ein für ein Standard-Eppendorf-Tube übliches Füllvolumen von bis zu 1,5 ml. Weitere bevorzugte Füllvolumina sind bis 0,4 ml, bis 0,5 ml, bis 0,7 ml, bis 1,0 ml oder bis 2,0 ml.

Üblicherweise handelt es sich bei den immobilisierten Sondenmolekülen auf dem Trägerelement um eine Substanzbibliothek. Unter einer Substarazbibliothek wird im Rahmen der vorliegenden Erfindung eine Ansammlung von unterschiedlichen Substanzen, die auf einer Oberfläche immobilisiert sind, verstanden. Die Anordnung der Substanzen auf der Oberfläche erfolgt derart, dass jeder Substanz ein bestimmter, eindeutig zu identifizierender Ort zugeordnet ist und dass jede Substanz vollkommen getrennt von den anderen immobilisiert ist.

Bei den Substanzbibliotheken kann es sich um Proteinsubstanzbibliotheken, Peptidsubstanzbibliotheken und Nukleinsäuresubstanzbibliotheken handeln. Bei Proteinsubstanzbibliotheken kann es sich insbesondere um Antikörper-, Rezeptormolekül- und Membranproteinbibliotheken handeln. Denkbare Peptidbibliotheken sind insbesondere Rezeptorligandenbibliotheken, pharmakologisch aktive Peptidbibliotheken und Peptidhormonbibliotheken.

Nukleinsäuresubstanzbibliotheken sind insbesondere DNA- und RNA-Molekülbibliotheken. Bei DNA-Molekülbibliotheken können besonders bevorzugt ribosomale DNA-Sequenzen von Mikroorganismen auf dem Trägerelement angebracht sein. Ferner kann es sich um Nukleinsäuresubstanzbibliotheken zur SNP-Analyse handeln. Ebenfalls denkbar sind Protein- oder Nukleinsäuresubstanzbibliotheken, die ein so genanntes "expression profiling" erlauben. Eine weitere Alternative sind kombinatorische Substanzbibliotheken.

Die Substanzbibliotheken sind dabei so auf das Trägerelement aufgebracht, dass sie den Probenraum des Reaktionsgefäßes kontaktieren. Das Trägerelement des Reaktionsgefäßes ist somit vorzugsweise dadurch gekennzeichnet, dass es auf seiner Oberfläche eine Detektionsfläche mit einer Substanzbibliothek aufweist und mindestens in dem Detektionsbereich optisch durchlässig ist.

Eine weitere vorteilhafte Ausgestaltung der Erfindung ist, dass das entstehende Reaktionsgefäß dicht ist und wässrige Proben auf Temperaturen von bis zu 100°C über Stunden erhitzt werden können, ohne dass es zu einem Austritt von Flüssigkeit kommt oder die Proben verdampfen. D.h. die erfindungsgemäßen Reaktionsgefäße können in weiten Temperaturbereichen eingesetzt werden und sind beim Einfrieren in flüssigem Stickstoff (bei -196°C) ebenso funktionsfähig wie im siedendem Wasserbad. Ebenso sind sie beispielsweise geeignet, um 15-minütiges Autoklavieren bei 121°C auszuhalten. Ferner sind die erfindungsgemäßen Reaktionsgefäße vorzugsweise chemisch resistent gegen Säuren, Basen und organische Lösungsmittel wie Alkohole, Aceton oder Phenol.

Das erfindungsgemäße Reaktionsgefäß weist vorzugsweise die üblicherweise in Labor-Reaktionsgefäßen verwendeten Deckel bzw. Deckelverriegelungen auf, wie beispielsweise die Systeme Easy-to-Open (Biozym, Oldendorf, Deutschland), Safe-Lock (Eppendorf) und dergleichen. Auf diese Weise ist die Dichtheit sowie das leichte, insbesondere einhändige Öffnen des Reaktionsgefäßes gewährleistet.

Falls das Trägerelement in eine Aussparung des Reaktionsgefäßes eingesetzt wird, wird eine ausreichende Dichtheit des Reaktionsgefäßes üblicherweise dadurch erreicht, dass das Trägerelement in die Aussparung eingeklebt und/oder eingeklemmt wird und gegebenenfalls anschließend ein Dichtmaterial an den erforderlichen Bereichen aufgebracht wird.

Die Klebstoffe werden üblicherweise mit handelsüblichen Dispensiergeräten aufgetragen. Als Klebstoff kommen vorzugsweise platinvernetzende Polydimethylsiloxane wie Sylgard 182 oder Sylgard 184 (Dow Corning, Midland, Michigan, USA) in Betracht. Alternativ können andere Klebstoffe wie beispielsweise Silikonkleber, Polyurethankleber, Epoxidharzkleber, Cyanacrylatkleber, Acrylkleber und/oder Heizkleber verwendet werden. Als Dichtmaterialien können unterschiedliche Kautschuke wie Silikonkautschuk und/oder Gummimaterialen und dergleichen eingesetzt werden.

Vorzugsweise ist das in dem Reaktionsgefäß angeordnete Trägerelement ein so genannter DNA-Chip. Bei einem derartigen DNA-Chip handelt es sich um eine beispielsweise auf einer Glasoberfläche gebundene DNA-Bibliothek mit eindeutiger Zuordnung der DNA-Sequenzen zu vorbestimmten Bereichen der Oberfläche.

Die im Rahmen der vorliegenden Erfindung eingesetzten Sonden-Arrays mit an definierten Stellen immobilisierten Sonden können allgemein nach herkömmlich bekannten Verfahren hergestellt werden. DNA-Chips werden vorzugsweise durch allgemein übliche Spotting-Verfahren oder durch spezielle ortsaufgelöste Synthese-Verfahren hergestellt. Für die Herstellung kommen auch Alternativ-Verfahren wie Synthese-Verfahren über eine lichtgeführte DNA-Synthese in Frage. Verfahren zur Herstellung von Sonden-Arrays bzw. Chips, speziell von DNA-Chips sind dem Fachmann bekannt und unter anderem in DE 197 06 570, EP 0 969 918 und WO 98/36827 beschrieben.

Vorzugsweise werden die Sonden-Arrays alternativ nach zwei prinzipiell unterschiedlichen Verfahren hergestellt.

Bei einer Methode werden separat synthetisierte Sonden, beispielsweise Oligonukleotide, mit Hilfe von Automaten, so genannten Spöttern, die das ortsspezifische Ablegen kleinster Flüssigkeitsmengen gewährleisten, auf Oberflächen aufgebracht und kovalent oder nicht kovalent mit dieser verknüpft. Das Verfahren arbeitet seriell. Jeder Spot wird individuell mit der Sonde bestückt.

Alternativ werden Sonden-Arrays durch ortsspezifische *in situ*-Synthese der Sonden, beispielsweise der Oligonukleotidsonden erzeugt. Die Synthese erfolgt parallel, beispielsweise im Wafer-Maßstab. Geeignete Reagenzien zur Aktivierung der Array-Oberfläche bzw. geeignete Schutzgruppen für die Sondensynthese auf der Array-Oberfläche sind dem Fachmann bekannt.

Die Immobilisierung von Molekülen auf der Array-Oberfläche kann entweder spezifisch oder unspezifisch erfolgen. Die spezifische Immobilisierung setzt eine Selektivität der Wechselwirkung bestimmter chemischer Funktionen des zu immobilisierenden Moleküls mit der Oberfläche des Substrats voraus. Ein Beispiel für eine spezifische, nicht kovalente Immobilisierung ist die Bindung von Biotinmarkierter Nukleinsäure an ein mit Streptavitin beschichtetes Substrat. Aminomodifizierte Nukleinsäuren lassen sich spezifisch über die Reaktion der Aminogruppe mit einem Epoxid, einer Carboxy-Funktion oder einem Aldehyd immobilisieren. Vorzugsweise wird die Immobilisierung über eine endständige Phosphatgruppe der Sonde bzw. des Monomer-Bausteins einer Biopolymer-Sonde an einer aminierten Oberfläche durchgeführt.

Die ortsspezifische Immobilisierung erfolgt über eine Vielzahl von Mechanismen und chemischen Funktionen und kann sowohl kovalent als auch nicht kovalent sein. Beispiel hierfür ist die Immobilisierung von Nukleinsäuren auf einer mit Poly-L-Lysin modifizierten Substratoberfläche, aber auch die Immobilisierung von chemisch nicht modifizierten Nukleinsäuren auf epoxidierten, aminierten oder mit Aldehyd-Funktionen besetzten Substratoberflächen.

Für das Ablegen geringer Mengen Material an vorgesehenen Stellen auf einem Substrat zur Herstellung eines Sonden-Arrays, der in das erfindungsgemäße Reaktionsgefäß eingesetzt wird, können ebenfalls dem Fachmann bekannte Verfahren eingesetzt werden. Eine Reihe derartiger Verfahren ist beispielsweise in D.J. Lockhart, E.A. Winzeler; Genomics, gene expression and DNA arrays; Nature, 405, Seiten 827-836, 2000 beschrieben.

Im Folgenden werden spezielle Ausführungsformen des erfindungsgemäßen Reaktionsgefäß anhand von Abbildungen detailliert beschrieben.

Das Reaktionsgefäß (1) in Abbildung 1 weist im Wesentlichen die Dimensionen von marktüblichen Reaktionsgefäßen auf und ähnelt vorzugsweise einem 1,5 ml Standard-Reaktionsgefäß aus Polypropylen, wie es in hohen Stückzahlen gefertigt wird. Es weist einen Durchbruch (im Rahmen der vorliegenden Erfindung auch als Aussparung bezeichnet) (8,10) auf, der mit einer Fassung versehen ist, in die die Affinitätsmatrix (100) eingelegt werden kann. Die Fassung umfasst im Allgemeinen eine Auflagefläche (6,11) für die Affinitätsmatrix (100), einen Flüssigkeitsdurchbruch (8), um die oberflächengebundene Substanzbibliothek (102) mit der Probe in Kontakt bringen zu können, sowie einen Sichtdurchbruch (10), um die Durchlichtdetektion zu ermöglichen. Des Weiteren kann der Durchbruch (8,10) mit einem Kleberand (7, 9) versehen sein, um die Verklebung der Affinitätsmatrix in das Reaktionsgefäß (2) zu vereinfachen. Um die Affinitätsmatrix (100) nicht in das Reaktionsgefäß einkleben zu müssen, können spezielle Schnellverschlüsse vorgesehen werden. Dabei kann die Dichtwirkung durch Dichtungen erreicht werden. Das Reaktionsgefäß kann durch einen Deckel (3) verschließbar sein.

Die in die Fassung eingearbeitete Affinitätsmatrix (100) kann sich an verschiedenen Positionen im Reaktionsgefäß (2) befinden. So ist es möglich, die Affinitätsmatrizen wie in den Abbildungen 1 bis 4 dargestellt am Boden des Reaktionsgefäßes zu befestigen. In diesem Fall ist die Affinitätsmatrix (100) immer den Probensubstanzen ausgesetzt.

Sollte die Affinitätsmatrix (100) empfindlich gegenüber einem oder mehreren Prozessschritten reagieren, so kann die Matrix auch in den Deckel (3) eingebaut werden. Die notwendigen Konditionierungsschritte der Probe werden dann bei aufrecht stehendem Reaktionsgefäß (1) durchgeführt, wobei die Affinitätsmatrix nicht mit der Probe in Berührung kommt und so geschützt wird. Zur Detektionsreaktion wird das Reaktionsgefäß dann auf den Kopf gestellt, so dass die Probe mit der oberflächengebundenen Substanzbibliothek (102) in Berührung kommt. Dies würde die thermischen und chemischen Belastungen der Affinitätsmatrix (100) verringern.

Die Affinitätsmatrizen (100) können auf unterschiedlich Weise mit dem Reaktionsgefäß (2) verbunden werden. Bei der in Abbildung 1 dargestellten Variante wird die Affinitätsmatrix von außen in einen Flüssigkeitsdurchbruch (8) auf eine Auflage (6) gelegt. In einen Kleberand (7) wird Klebstoff gegeben, der durch Fließ- und Kapillarwirkung die Affinitätsmatrix (100) umschließt und mit dem Reaktionsgefäß (2) flüssigkeitsdicht und temperaturstabil verbindet Alternativ kann die Affinitätsmatrix (100) auch mit dem Reaktionsgefäß (2) beispielsweise durch Laser-Schweißen verschweißt werden. Die Affinitätsmatrix (100) wird so in das Reaktionsgefäß eingebaut, dass die oberflächengebundene Substanzbibliothek (102) mit der Probenlösung in Kontakt treten kann. Nach dem Einbau der Affinitätsmatrix (100) ist eine optische Durchlichtdetektion mit den Lesegeräten (1000) entsprechend den Abbildungen 5 bis 15 möglich.

Alternativ kann die Affinitätsmatrix (100) auch von innen eingebaut werden, wie in Abbildung 2 dargestellt. Bei dieser Variante liegen die Auflagefläche und der Kleberand im Reaktionsraum des Reaktionsgefäß. Das hat den Vorteil, dass die Affinitätsmatrix (100) auch bei höherem Innendruck, z.B. bei Verwendung in einer Zentrifuge bzw. bei Erwärmung der Probenflüssigkeit auf Temperaturen nahe des Siedepunktes, des Reaktionsraum nicht aus ihrer Halterung gedrückt werden kann. Allerdings erfordert die Montage einen höheren Aufwand.

In Abbildung 3 wird eine Variante gezeigt, bei der die Affinitätsmatrix (100) in einen Chipträger eingebaut wird. Anschließend wird der Chipträger (200) auf das Reaktionsgefäß (2) gedrückt. Eine Klemmverbindung oder ein Gewinde (201) sorgen für eine kraftschlüssige und flüssigkeitsundurchlässige Verbindung zwischen Reaktionsgefäß (2) und Chipträger (200). Diese Variante vereint die Vorteile einer von innen eingesetzten Affinitätsmatrix (100) in das Reaktionsgefäß mit denen einer vereinfachten Montage. Nachteilig wirkt sich eine weitere Verbindungsstelle wie z.B. die Klemmverbindung sowie die höhere Anzahl an Bauteilen aus.

In Abbildung 4 ist eine Variante dargestellt, bei der die Affinitätsmatrix (100) durch eine von innen eingesetzte Klemmhülse (300) zwischen Reaktionsgefäß (2) und Klemmhülse (300) so eingeklemmt wird, das eine Verklebung der Affinitätsmatrix (100) mit dem Reaktionsgefäß (2) überflüssig wird. Jedoch erfordert diese Ausführung eine sehr hohe Fertigungspräzision von Klemmhülse (300) und Reaktionsgefäß (2), um eine flüssigkeitsdichte Verklemmung der Affinitätsmatrix (100) zu gewährleisten. Die Klemmhülse (300) kann in einer weiteren Variante in das Reaktionsgefäß (2) eingeschraubt werden.

Bei einem weiteren Aspekt der vorliegenden Erfindung wird ein Verfahren zum Nachweis der spezifischen Wechselwirkung zwischen molekularen Target- und Sondenmolekülen bereitgestellt, das die folgenden Schritte umfasst:

a) Bereitstellung eines vorstehend beschriebenen erfindungsgemäßen Reaktionsgefäßes;
b) Wechselwirkung des Targets mit den auf vorbestimmten Bereichen (Array-Elementen) angeordneten Sonden; und
c) Detektion der Wechselwirkung.

Die zu untersuchenden Targets können in jeder Art von Probe, vorzugsweise in einer biologischen Probe vorliegen. Vorzugsweise werden die Targets vor ihrer Detektion und Quantifizierung durch das erfindungsgemäß Verfahren isoliert, gereinigt, kopiert und/oder amplifiziert.

Die Amplifikation erfolgt üblicherweise durch herkömmliche PCR-Methoden. Vorzugsweise wird die Amplifikation als Multiplex-PCR in einem zweistufigen Prozess ausgeführt (siehe auch WO 97/45559). In einer ersten Stufe wird eine Multiplex-PCR durchgeführt, indem Fusionsprimer eingesetzt werden, deren 3'-Enden genspezifisch sind und deren 5'-Enden eine universelle Region darstellen. Letztere ist bei allen in der Multiplex-Reaktion eingesetzten forward- und reverse-Primem gleich. In dieser ersten Stufe ist die Primermenge limitierend. Dadurch können alle Multiplex-Produkte bis zu einem einheitlichen molaren Niveau amplifiziert werden, vorausgesetzt, dass die Zyklenzahl hinreichend ist, um für alle Produkte Primerlimitation zu erreichen. In einer zweiten Stufe werden universelle Primer zugegen, die identisch mit den 5'-Regionen der Fusionsprimer sind. Es erfolgt Amplifikation bis zur gewünschten DNA-Menge.

Der Nachweis erfolgt bei dem erfindungsgemäßen Verfahren vorzugsweise dadurch, dass die gebundenen Targets mit mindestens einer Markierung versehen sind, die in Schritt c) detektiert wird.

Bei einer alternativen Ausführungsform umfassen die auf dem Sonden-Array angeordneten Sondenmoleküle, die dem Nachweis der molekularen Wechselwirkungen mit dem Targetmolekülen dienen, mindestens eine Markierung sowie mindestens eine Sollbruchstelle, d.h. eine labile bzw. selektiv spaltbare Bindung, die spezifisch destabilisiert bzw. gespalten werden kann. Die zwischen der Markierung und der Position der Verknüpfung der Sonden mit der Array-Oberfläche angeordnete selektiv spaltbare Bindung ermöglicht, dass die Markierung bzw. detektierbare Einheit auch zum spezifischen Nachweis der molekularen Wechselwirkung zwischen Sonden und Targets eingesetzt werden kann. Dabei ist die Sollbruchstelle bzw. selektive spaltbare Bindung innerhalb des Sondenmoleküls so positioniert, dass ein Bruch der Bindung zum Ablösen der detektierbaren Einheit bzw. der Ankergruppe mit der detektierbaren Einheit von der Array-Oberfläche führt. Dagegen bleiben solche Markierungen mit der Array-Oberfläche verknüpft, deren Sonden-Moleküle spezifisch mit Target-Molekülen wechselgewirkt haben, da das mit der Markierung verknüpfte Spaltprodukt der Sonde bzw. das Sondenfragment über die Wechselwirkung mit dem Target mit dem zweiten Spaltprodukt der Sonde, das auf der Oberfläche des Arrays immobilisiert ist, gekoppelt bleibt Die Bereitstellung derartiger Sonden-Moleküle, die mindestens eine Sollbruchstelle umfassen, ist ausführlich in der deutschen Patentanmeldung DE 10142 643.7 beschrieben.

Die selektiv spaltbaren Bindungen sind in dieser Ausführungsform vorzugsweise derart beschaffen, dass sie auch dann effektiv spaltbar sind, wenn die Sonden an der Array-Oberfläche immobilisiert sein. Die selektiv spaltbare Bindung kann vorzugsweise durch chemische und/oder physikalische Methoden selektiv gespalten werden. Eine effiziente Spaltung an der Oberfläche ist insbesondere durch Agenzien mit geringem Ausmaß wie Atome und Ionen gewährleistet. Vorzugsweise ist die labile Bindung deshalb durch einfache chemische Agenzien selektiv spaltbar, beispielsweise durch Zugabe von Ionen, besonders bevorzugt von Säureanionen, Basekationen, Fluorid- und/oder Schwermetallionen wie Quecksilber- und/oder Silberionen.

Im Falle der Herstellung der Arrays durch Immobilisierung von separat synthetisierten Oligonukleotiden ist die selektiv spaltbare Bindung unter den Bedingungen, die bei der Immobilisierung der Sonden auf der Array-Oberfläche angewendet werden, stabil. Erfolgt die Herstellung der Sonden in situ durch ortsspezifische Synthese auf der Array-Oberfläche, ist es bevorzugt, dass die labile Bindung im Rahmen des Syntheseverlaufes effizient erzeugt werden kann. Besonders bevorzugt ist die Bereitstellung der labilen Bindung mittels Phosphoramiditchemie. Gleiches gilt im Übrigen für den Einbau der detektierbaren Einheit.

Folglich ist es bevorzugt, dass die selektiv spaltbare Bindung in einer Nukleinsäure vorliegt, die durch herkömmliche DNA- oder RNA-Synthese hergestellt werden kann. Besonders bevorzugt umfassen die Sondenmoleküle des erfindungsgemäßen Sonden-Arrays eine Nukleinsäure der Formel $A_1$-S-$A_2$, wobei S eine Nukleinsäure bzw. ein Nukleotidbaustein ist, die bzw. der mindestens eine selektiv spaltbare Bindung umfasst, und $A_1$ und $A_2$ beliebige Nukleinsäuren oder Nukleinsäureanaloga sind. Über eine der beiden Nukleinsäuren oder Nukleinsäureanaloga $A_1$ und $A_2$ ist das Sondenmolekül auf der Oberfläche des erfindungsgemäßen Sonden-Arrays immobilisiert, während die andere mindestens eine Markierung aufweist. S ist vorzugsweise ein Nukleotid-Dimer, das durch eine selektiv spaltbare Bindung verbrückt ist.

Beispiele für besonders bevorzugte DNA-Nukleotid-Bausteine S, die eine selektiv spaltbare Bindung umfassen, sind in der folgenden Formel I angegeben:

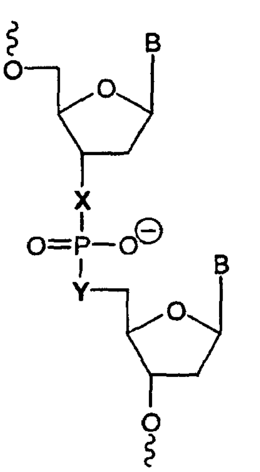

I

Dabei kann X und Y unabhängig voneinander ausgewählt werden aus der Gruppe vorzugsweise bestehend aus O, NH und S, wobei X und Y nicht gleichzeitig O sind.

B steht für eine Nucleobase wie die Purin-Derivate Adenin und Guanin sowie die Pyrimidine Cytosin und Thymin.

Die selektiv spaltbare Bindung innerhalb der Nukleotidsequenz derartiger Oligonukleotidsonden ist vorzugsweise eine Phosphothioatbindung oder eine Phosphoramidatbindung. Besonders bevorzugt ersetzt die Phosphothioatbindung, d.h. eine Zucker-O-P-S-Zucker-Bindung eine Phosphodiesterbindung, d.h. eine Zucker-O-P-O-Zucker-Bindung eines unmodifizierten Oligonukleotids. In dieser Ausführungsform sind zwei Nukleoside durch eine Phosphothioatbindung verbunden.

Alternativ kann die selektiv spaltbare Bindung innerhalb der Nukleotidsequenz auch eine andere Schwefel- oder Stickstoff-modifizierte Esterbindung wie beispielsweise eine Phosphonothioat-Bindung sein.

Weitere Beispiele für die Bereitstellung von selektiv spaltbaren Bindungen in den Sondenmolekülen des erfindungs gemäßen Sonden-Arrays sind Amid-, 1,2-Diol-, Disulfid- und/oder Sulfonyl-Gruppen sowie weitere Gruppen, die in US 5,118,605 beschrieben sind und unter den dort genannten Bedingungen spaltbar sind. Diese Gruppen sind allerdings weniger bevorzugt, da u.a. ihre Inkorporation in Oligonukleotidsonden mittels herkömmlicher Nukleinsäure-Synthese nicht möglich ist.

Alternativ können auch physikalische Methoden zur Spaltung der selektiv spaltbaren Bindung in den Sondenmolekülen eingesetzt werden. So kann die selektiv spaltbare Bindung beispielsweise photolytisch selektiv gespalten werden. Nukleotidbausteine, die eine photolytisch selektiv spaltbare Bindung umfassen und für die Synthese der Sondenmoleküle des erfindungsgemäßen Sonden-Arrays eingesetzt werden können, sind beispielsweise in US 5,367,066, US 5,552,538 und US 5,578,717 beschrieben.

Weitere Beispiele für besonders bevorzugte RNA-Nukleotid-Bausteine, die eine chemisch oder physikalisch selektiv spaltbare Bindung umfassen, sind in der folgenden Formel II angegeben:

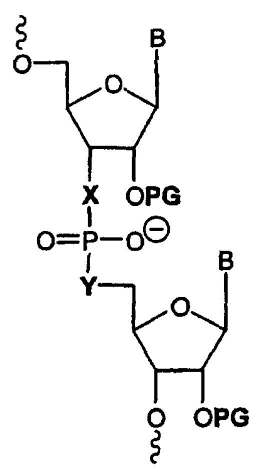

II

Dabei kann X und Y unabhängig voneinander ausgewählt werden aus der Gruppe vorzugsweise bestehend aus O, NH und S, wobei X und Y nicht gleichzeitig O sind, wenn PG keine labile Schutzgruppe ist.

PG wird vorzugsweise ausgewählt der Gruppe bestehend aus H und labilen Schutzgruppen wie

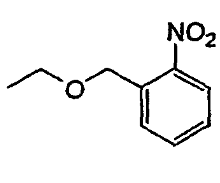 (photolabil) oder 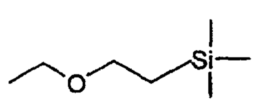 (labil z.B. gegen Fluorionen).

B steht in Formel II für eine Nucleobase wie die Purin-Derivate Adenin und Guanin sowie die Pyrimidine Cytosin und Uracil.

Bevorzugt sind allerdings Sondenmoleküle mit selektiv spaltbaren Bindungen, die unter normalen atmosphärischen, Temperatur-, und Licht-Bedingungen stabil sind.

Bei einer alternativen Ausführungsform ist die labile Bindung selektiv durch enzymatische Methoden spaltbar. Beispiele für Nukleotidbausteine, die derartige labile Bindungen umfassen, sind in US 4,775,619 und US 4,876,187 beschrieben. Enzymatische Methoden zur Spaltung der selektiv spaltbaren Bindung sind allerdings im Rahmen der vorliegenden Erfindung weniger bevorzugt, da enzymatische Aktivitäten durch die Nähe der selektiv spaltbaren Bindung zur Oberfläche aufgrund der Immobilisierung der Sondenmoleküle stark behindert werden. Folglich weist eine enzymatische Spaltungsreaktion nur eine sehr niedrige Effizienz auf, was einen unerwünschten hohen Signalhintergrund durch fälschlicherweise positive Messergebnisse zur Folge hat. Somit kann bei einer bevorzugten Ausführungsform des erfindungsgemäßen Sonden-Arrays die selektiv spaltbare Bindung nicht durch enzymatische Methoden selektiv gespalten werden.

Bei einer weiteren bevorzugten Ausführungsform des erfindungsgemäßen Sonden-Arrays befindet sich die selektiv spaltbare Bindung ungefähr in der Mitte zwischen der Stelle der Immobilisierung der Sonde auf der Array-Oberfläche und der Position der Markierung der Sonde. Somit wird gewährleistet, dass die Wahrscheinlichkeit der Wechselwirkung des Targets mit dem immobilisierten Sondenfragment, welches dem nach der Bindungsspaltung an der Oberfläche verbleibenden Rest der Sonde entspricht, wesentlich vermindert bzw. nahezu ausgeschlossen ist. Befindet sich die selektiv spaltbare Bindung dagegen zu nahe an der Array-Oberfläche, so ist der Komplex aus Sonden- und Targetmolekül nach der Spaltung nicht mehr ausreichend stabilisiert, da die Hybridisierung des Targets mit dem an der Array-Oberfläche immobilisierten Sondenfragments nicht stabil genug ist. Dies würde zu fälschlicherweise negativen Messergebnissen führen.

Wie bereits vorstehend erwähnt, ist die Markierung, die an die Targets oder Sonden gekoppelt ist, vorzugsweise eine detektierbare Einheit oder eine über eine Ankergruppe an die Targets oder Sonden gekoppelte detektierbare Einheit. Hinsichtlich der Möglichkeiten der Detektion bzw. der Markierung ist das erfindungsgemäße Verfahren äußerst flexibel. So ist das erfindungsgemäße Verfahren mit einer Vielzahl physikalischer, chemischer oder biochemischer Detektionsverfahren kompatibel. Voraussetzung ist lediglich, dass die zu detektierende Einheit bzw. Struktur direkt an eine Sonde oder ein Target, beispielsweise ein Oligonukleotid gekoppelt bzw. über eine mit dem Oligonukleotid koppelbare Ankergruppe verknüpft werden kann.

Die Detektion der Markierung kann auf Fluoreszenz, Magnetismus, Ladung, Masse, Affinität, enzymatischer Aktivität, Reaktivität, einer Goldmarkierung u.dgl. beruhen. So kann die Markierung beispielsweise auf der Verwendung von Fluorophormarkierten Strukturen bzw. Bausteinen basieren. In Verbindung mit der FluoreszenzDetektion kann die Markierung ein beliebiger an Targets oder Sonden während oder nach deren Synthese koppelbarer Farbstoff sein. Beispiele hierfür sind Cy-Farbstoffe (Amersham Pharmacia Biotech, Uppsala, Schweden), Alexa-Farbstoffe, Texas-Rot, Fluorescein, Rhodamin (Molecular Probes, Eugene, Oregon, USA), Lanthanide wie Samarium, Ytterbium und Europium (EG&G, Wallac, Freiburg, Deutschland).

Neben Fluoreszenz-Markern können im Rahmen der vorliegenden Erfindung als Markierung bzw. als Detektiereinheit, die mit den Targets bzw. Sonden gekoppelt ist, auch Lumineszenz-Marker, Metall-Marker, Enzym-Marker, radioaktive Marker und/oder polymere Marker eingesetzt werden.

Ebenso kann eine Nukleinsäure als Markierung (Tag) genutzt werden, die durch Hybridisierung mit einer markierten Reporter detektiert werden kann (Sandwich-Hybridisierung). Einsatz zum Nachweis des Tags finden diverse molekularbiologische Nachweisreaktionen wie Primer-Extension, Ligation und RCA.

Bei einer alternativen Ausführungsform des erfindungsgemäßen Verfahrens ist die detektierbare Einheit über eine Ankergruppe mit den Targets oder Sonden gekoppelt. Bevorzugt verwendete Ankergruppen sind Biotin, Digoxygenin u.dgl. Die Ankergruppe werden in einer anschließenden Reaktion mit spezifisch bindenden Komponenten, beispielsweise Streptavidin-Konjugaten oder Antikörper-Konjugaten umgesetzt, die selbst detektierbar sind oder eine detektierbare Reaktion auslösen. Bei Einsatz von Ankergruppen kann die Umsetzung der Ankergruppen in detektierbare Einheiten vor, während oder nach Zugabe der Probe umfassend die Targets bzw. ggf. vor, während oder nach der Spaltung der selektiv spaltbarer Bindung in den Sonden erfolgen.

Die Markierung kann erfindungsgemäß auch durch Wechselwirkung eines markierten Moleküls mit den Sonden-Molekülen erfolgen. Beispielsweise kann die Markierung durch Hybridisierung eines wie vorstehend beschrieben markierten Oligonukleotids mit einer Oligonukleotid-Sonde bzw. einem Oligonukleotid-Target erfolgen.

Weitere im Rahmen der vorliegenden Erfindung geeignete Markierungsverfahren und Nachweissysteme sind beispielsweise in Lottspeich und Zorbas, Bioanalytik, Spektrum Akademischer Verlag, Heidelberg, Berlin, 1998, Kapitel 23.3 und 23.4 beschrieben.

Bei einer bevorzugten Ausführungsform des erfindungsgemäßen Verfahrens werden Nachweisverfahren eingesetzt, die im Ergebnis ein Addukt mit einem bestimmten Löslichkeitsprodukt, das eine Präzipitation zur Folge hat, liefern. Zur Markierung werden insbesondere Substrate eingesetzt, die in ein schwer lösliches, üblicherweise gefärbtes Produkt umgesetzt werden können. Beispielsweise können bei dieser Markierungsreaktion Enzyme verwendet werden, die den Umsatz eines Substrats in ein schwer lösliches Produkt katalysieren. Eine Reihe von in Frage kommenden Reaktionen, die geeignet sind, um zu einem Niederschlag an Array-Elementen zu führen, sowie Möglichkeiten für die Detektion des Niederschlags sind beispielsweise in der internationalen Patentanmeldung WO 00/72018 und in der

internationalen Patentanmeldung WO 02/02810 beschrieben, auf deren Inhalt hiermit ausdrücklich Bezug genommen wird.

Bei einer besonders bevorzugten Ausführungsform des erfindungsgemäßen Verfahrens sind die gebundenen Targets mit einer Markierung versehen, die die Reaktion eines löslichen Substrats zu einem schwer löslichen Niederschlag auf dem Array-Element katalysiert, an dem einen Sonden/Target-Wechselwirkung stattgefunden hat bzw. die als Kristallisationskeim für die Umwandlung eines löslichen Substrats zu einem schwer löslichen Niederschlag auf dem Array-Element wirkt, an dem eine Sonden/Target-Wechselwirkung stattgefunden hat.

Der Einsatz des erfindungsgemäßen Verfahrens erlaubt auf diese Weise die simultane qualitative und quantitative Analyse einer Vielzahl von Sonden/Target-Wechselwirkungen, wobei einzelne Array-Elemente mit einer Größe von ≤1000 µm, vorzugsweise von ≤100 µm und besonders bevorzugt von ≤50 µm realisiert werden können.

In der Immunzytochemie und bei immunologischen Mikrotiterplatten-basierten Tests ist der Einsatz von enzymatischen Markierungen bekannt (siehe E. Lidell und I. Weeks, Antibody Technology, BIOS Scientific Publishers Limited, 1995). So katalysieren beispielsweise Enzyme den Umsatz eines Substrats in ein schwerlösliches, in aller Regel gefärbtes Produkt.

Eine weitere Möglichkeit des Nachweises molekularer Wechselwirkungen auf Arrays besteht im Einsatz von Metallmarkierungen. Hierbei werden beispielsweise kolloidales Gold oder definierte Goldcluster mit den Targets gekoppelt, ggf. über bestimmte Vermittlermoleküle wie Streptavidin. Die durch die Goldmarkierung entstehende Färbung wird vorzugsweise durch nachfolgende Reaktion mit unedleren Metallen wie z.B. Silber verstärkt, wobei die mit den Targets gekoppelte Goldmarkierung als Kristallisationskeim bzw. Katalysator beispielsweise für die Reduktion von Silberionen zu einem Silberniederschlag wirkt. Die mit Goldmarkierungen gekoppelten Targets werden im Folgenden auch als Goldkonjugate bezeichnet.

Bei dieser Ausführungsform des erfindungsgemäßen Verfahrens kann auch eine relative Quantifizierung der Sonden-/Target-Wechselwirkung erfolgen. Die relative Quantifizierung der Konzentration der gebundenen Targets auf einem Sonden-Array durch Nachweis eines Präzipitats bzw. eines Niederschlags erfolgt über die Konzentration der mit den Targets gekoppelten Markierungen, die die Reaktion eines löslichen Substrats zu einem schwerlöslichen Niederschlag auf dem Array-Element, an dem eine Sonden/Target-Wechselwirkung stattgefunden hat, katalysieren bzw. als Kristallisationskeim für derartige Reaktionen wirken. Beispielsweise beträgt im Fall von mit Nanogold markierten HPLC-gereinigten Oligonukleotidsonden das Verhältnis von gebundenem Target zu Goldpartikel 1:1. In anderen Ausführungsformen der vorliegenden Erfindung kann es ein Vielfaches oder auch einen Bruchteil davon betragen.

Die Detektion bei dieser Ausführungsform des erfindungsgemäßen Nachweisverfahrens erfolgt somit durch Messung der Durchlichtabsorption oder Auflichtreflektion, die durch den Niederschlag hervorgerufen wird, der auf den Array-Elementen, an denen einen Sonden/Target-Wechselwirkung stattgefunden, durch die katalytische Wirkung der mit den gebundenen Targets gekoppelten Markierung erzeugt wird.

Die Lichtabsorption wird im Fall der Kopplung von kolloidalem Gold oder definierten Goldcluster mit den Targets bereits durch die Gegenwart dieser metallischen Markierungen hervorgerufen. Zur Verstärkung der Lichtabsorption wird allerdings vorzugsweise katalytisch durch derartige Wechselwirkungshybride, d.h. die mit einer Markierung wie beispielsweise kolloidalem Gold oder definierten Goldclustern versehene Targets, ein nicht transparentes Präzipitat abgeschieden. Als besonders vorteilhaft hat sich im Fall von Goldkonjugaten die Verwendung von Silber als Präzipitat herausgestellt.

Im Folgenden wird der qualitative und/oder quantitative Nachweis der Sonden-/Target-Wechselwirkung durch Messung der Durchlichtabsorption anhand eines Beispiel erläutert. Selbstverständlich ist die im Folgenden beschriebene Vorgehensweise nicht auf die vorstehend beschriebene Silber/Gold-Färbung beschränkt, sondern kann entsprechend auf alle Nachweisverfahren angewendet werden, bei denen die gebundenen Targets mit einer Markierung versehen sind, die die Reaktion eines löslichen Substrats zu einem schwer löslichen Niederschlag auf dem Array-Element katalysiert, an dem einen Sonden/Target-Wechselwirkung stattgefunden hat bzw. die als Kristallisationskeim für die Umwandlung eines löslichen Substrats zu einem schwer löslichen Niederschlag auf dem Array-Element wirkt, an dem eine Sonden/Target-Wechselwirkung stattgefunden hat.

Zunächst wird das Targetmolekül z.B. mittels PCR biotiniliert. Das PCR-Produkt wird gegen eine Substanzbibliothek (102), beispielsweise eine DNA-Bibliothek, hybridisiert. Anschließend werden dem erfindungsgemäßen Reaktionsgefäß (1) Streptavidin-funktionalisierte Goldkügelchen zugegeben, die mit den biotinilierten Hybriden, beispielsweise DNA-Hybriden reagieren. An den nun spezifisch an der Oberfläche gebundenen Goldkügelchen kann ein Silberniederschlag erzeugt werden, indem beispielsweise Silbernitrat mit Hydrochinon unter der katalytischen Wirkung von Gold reduziert wird (siehe u.a. WO 00/72018, DE 100 33 334.6, M.A. Hayat, Immunogold-Silver Staining, CRC Press, New York, 1995).

Die Lichtabsorption durch das Silberpräzipitat hängt von der Menge des abgeschiedenen Silbers ab. Folglich kann die Lichtintensität I, die das Präzipitat durchstrahlt, nach einem dem Lambert-Beerschen Gesetz ähnlichen Funktion berechnet werden:

$$I = I_0 * \exp(-a * b) \quad \text{(I)}$$

Dabei ist I die Lichtintensität nach der Absorption, $I_0$ die Lichtintensität vor der Absorption, *a* ein Absorptionskoeffizient multipliziert mit der Abschattung pro Flächeneinheit *b* durch das Silberpräzipitat. Als Messgrößen stehen die Intensität I und die Zeit t zur Verfügung. Diese Messgrößen erhält man, indem das Trägerelement mit der Substanzbibliothek beleuchtet wird und das durchstrahlte Licht mit einer Kamera aufgenommen wird (siehe Abbildung 19). Diese Aufnahme wird in regelmäßigen Abständen wiederholt, während die Silberabscheidung durchgeführt wird. Die Helligkeitswerte der einzelnen Bibliotheksregionen (Spots) werden für jede Aufnahme ausgewertet, wodurch die Intensität I eines jeden Spot erhalten wird. Diese Helligkeitswerte können mittels Standardsoftware, wie zum Beispiel IconoClust® (Clondiag, Jena, Deutschland) automatisch berechnet werden. Durch Auftragen von $I/I_0$ gegen die Zeit t erhält man die in Abbildung 20 dargestellten Messkurven. Das Lambert-Beersche Gesetz entsprechend der Gleichung (I) kann mit den derart erhaltenen Silberabscheidungszeitreihen auf folgende Weise in Zusammenhang gebracht werden.

Die Fläche *F*, die ein Silberkügelchen abschattet, beträgt:

$$F = \pi * r^2 \quad \text{(II)}$$

Da die Abscheidungsgeschwindigkeit pro Flächenelement für einen Spot als konstant angenommen werden kann, wächst der Radius *r* der Silberkügelchen ebenfalls mit konstanter Geschwindigkeit:

$$r = \mathrm{d}r/\mathrm{dt} * t \quad \text{(III)}$$

Die Abschattung pro Flächeneinheit *b* durch das Silberpräzipitat ist proportional zur der Zahl der Silberkügelchen pro Flächeneinheit N, der Abschattungsfläche pro Silberkügelchen F und einer Konstante *k*.

$$b = N * F * k \quad \text{(III)}$$

Damit berechnet sich die Funktion für die Intensität I in Abhängigkeit von der Zeit t zu

$$I = I_0 * \exp(-a' * t^2), \quad \text{(IV)}$$

wobei *a'* eine unbekannte zusammengesetzte Silberabsorptionskonstante ist.

Für jede Silberabscheidungsreaktion muss eine eigene Abscheidungsrate angenommen werden, so auch für die unspezifische Reaktion an der Oberfläche:

$$I = I_0 * \exp(-a'_H * t^2), \quad \text{(V)}$$

wobei $a'_H$ die unspezifische Silberabsorptionskonstante ist.

An jedem Spot *i*, findet also sowohl eine spezifische Abscheidungsreaktion als auch eine unspezifische Abscheidungsreaktion statt:

$$I_i = I_{0i} * (\exp(-a'_i * t^2) + \exp(-a'_H * t^2)) + O_i, \quad \text{(VI)}$$

wobei *O* ein geräteabhängiger Offsetwert ist.

Die Zahl der Goldkügelchen, die pro Flächeneinheit abgeschieden werden, hängt einerseits von der Menge an mit Goldkügelchen markierten Targets und andererseits von der Bindungsstärke der Targets, beispielsweise der Target-DNA, mit den Sonden, beispielsweise der Spot-DNA, ab. Liegt kein Target in der Probe vor, das mit einer Sonde auf dem entsprechenden Array-Element wechselwirkt, so kommt es auch nicht zur Abscheidung von Goldkügelchen auf der Oberfläche dieses Array-Elements bzw. Spots. Ist die Bindung zwischen Sonde und Target schwach, so werden sich nur sehr wenige Goldkügelchen an der Oberfläche dieses Array-Elements absetzen.

Da *b* und damit *a'* direkt proportional zur Goldkügelchen - und damit Silberkügelchenanzahl *N* ist, stellt *a'* ein Maß für die Konzentration der Target-DNA und der Bindungsstärke der Target-DNA am Array-Element bzw. Spot i da.

In Abbildung 20 sind die Silberabscheidungszeitreihen zweier Spots dargestellt, die gegen eine Target-DNA hybridisiert werden. Die DNA-Sonden der Spots unterscheiden sich um eine Base, so dass die Target-DNA zu der Sonde des einen Spot perfekt komplementär ist, während sie zu der Sonde des anderen Spots einen Mismatch aufweist. Aus den beiden Messkurven lässt sich durch nichtlineare Regression mit Hilfe der Gleichung (VI) die Silberabsorptionskonstante *a'* berechnen. Im Falle des Perfect Match beträgt sie 2,566 x $10^{-6}$ $sek^{-2}$ und im Falle des Mismatch 4,83 x $10^{-7}$ $sek^{-2}$ (siehe auch Beispiel 2). Die beiden Konstanten unterscheiden sich damit um fast eine Zehnerpotenz. Damit kann die Berechnung der Konstanten *a'* als signifikante Messgröße für die Bindungsstärke und die Konzentration der Target-DNA an einem Spot genutzt werden. Des Weiteren kann man aus der Konstanten *a'* die Zeitkonstante τ der Abscheidungsreaktion bestimmen:

$$\tau_I = (1 / a'_i)^{0.5} \qquad \text{(VII)}$$

Durch die Regression können als weitere Parameter $I_0$ und *O* ermittelt werden. Mit der Lichtintensität $I_0$ können Beleuchtungsinhomogenitäten der DNA-Bibliothek korrigiert werden. Alternativ kann mittels des Abbildes der gesamten DNA-Bibliothek zum Zeitpunkt t = 0 eine Flatfieldkorrektur aller weiteren Bilder, die für die Zeitreihe zu einem späteren Zeitpunkt aufgenommen wurden, vorgenommen werden. Anhand des Parameters *O* lässt sich die Validität einer Messung abschätzen.

Da die korrekte Anpassung einer Exponentialfunktion an die Messwerte einen nichtlinearen Regressionsalgorithmus, beispielsweise eine nicht-lineare Regression nach Marquardt (H.R. Schwarz, Numerische Mathematik, Teubner Verlag, Stuttgart, Deutschland, 1998) erfordert, kann es vorteilhaft sein, auf eine weniger genaue, aber dafür robustere und daher lineare Methode zur Bestimmung der Messwerte aus den Zeitreihen zuzugreifen. Dazu werden die Zeitwerte quadriert und die Intensitätsmesswerte logarithmiert. Die so erhaltenen Werte werden dann an eine lineare Gleichung 1. Gerades angepasst. Die dabei erhaltenen Regressionsparameter können als Messwert und zur Testung der Validität herangezogen werden. Beleuchtungsinhomogenitäten lassen sich bei Verwendung einer linearen Methode über $I_0$ nicht mehr korrigieren, es verbleibt aber die Möglichkeit der Flatfieldkorrektur.

Eine weitere Variante der Auswertung besteht darin, nach einer festgelegten Zeit die Grauwerte der einzelnen Spots direkt als Messwerte zu verwenden. Dieses Verfahren hat allerdings die Nachteile, dass nicht im Voraus beurteilt werden kann, welcher Zeitpunkt zur Auswertung optimal ist und dass die Messwerte eine geringere statistische Sicherheit aufweisen. Darüber hinaus lässt sich eine etwaige Beleuchtungsinhomogenität nur über eine Flatfieldkorrektur durchführen.

Bei einer weiteren bevorzugten Ausführungsform des erfindungsgemäßen Verfahrens wird somit in Schritt c) der zeitliche Verlauf der Niederschlagsbildung an den Array-Elementen in Form von Signalintensitäten detektiert. Auf diese Weise kann eine genaue Bestimmung der relativen quantitativen Menge an gebundenen Targets gewährleistet werden. Eine derartige Vorgehensweise ist ausführlich in der internationalen Patentanmeldung WO 02/02810 beschrieben.

Wie bereits vorstehend erwähnt, sind die Targets vorzugsweise mit Markierungen versehen, die die Reaktion eines löslichen Substrats zu einem schwer löslichen Niederschlag auf dem Array-Element, an dem eine Sonden/Target-Wechselwirkung stattgefunden hat, katalysieren, bzw. Kristallisationskeim für derartige Reaktionen wirken.

Dabei können bei einer Ausführungsform der vorliegenden Erfindung die Targets direkt mit derartigen Markierungen versehen sein.

Alternativ wird auf eine direkte Markierung der Targets verzichtet und die Markierung erfolgt über Sandwich-Hybridisierung oder Sandwich-Reaktionen mit der mit dem Target wechselwirkenden Sonde und einer markierten Verbindung. Beispiele für eine derartige Vorgehensweise sind:

- Sandwich-Hybridisierung mit einem zur Targetsequenz komplementären markierten Oligonukleotid.
- Sandwich-Hybridisierung von in Kettenform mit der Targetsequenz hybridisierenden markierten Oligonukleotiden: Unter in Kettenform mit der Targetsequenz hybridisierenden markierten Oligonukleotiden wird im Rahmen der vorliegenden Erfindung ein Satz von markierten Oligonukleotiden verstanden, von denen mindestens eines Komplementarität sowohl zur Targetsequenz als auch zu einem weiteren Oligonukleotid aufweist. Die anderen Oligonulcleotide sind selbstkomplementär bzw. wechselseitig zueinander komplementär, so dass während der Hybridisierung eine Kette von an der Targetsequenz gebundenen markierten Oligonukleotiden entsteht.
- Sandwich-Hybridisierung mit einem zur Targetsequenz komplementären Oligonukleotid, das mit einer vielfach

markierten Struktur, beispielsweise einem Dendrimer, beschrieben z.B. in WO 99/10362, gekoppelt ist.

Eine weitere bevorzugte Möglichkeit der Kopplung der Targets mit einer Markierung stellt das synthetische oder enzymatische Anfügen eines homopolymeren Bereichs, beispielsweise einer polyA-Sequenz, an die Targets unter Bildung einer fortlaufenden Sequenz dar, wie es beispielsweise in US 6,103,474 beschrieben ist. Bei dieser Ausführungsform erfolgt die Markierung vorzugsweise über Sandwich-Hybridisierung mit einem zur Homopolymer-Sequenz komplementären markierten Oligonukleotid mit den vorstehend beschriebenen Variationen.

Bei einer weiteren bevorzugten Ausführungsform der vorliegenden Erfindung erfolgt eine Signalamplifikation durch Amplifikation von Teilen der an die Targets angefügten Homopolymersequenz bei gleichzeitigem Einbau von markierten Basen, insbesondere bevorzugt über einen RCA-Mechanismus durch Verwendung eines zirkulären einzelsträngigen Templates, das Komplementarität zur Homopolymersequenz aufweist.

Die nachfolgende Tabelle 1 gibt, ohne den Anspruch zu erheben, vollständig zu sein, einen Überblick über eine Reihe von in Frage kommenden Reaktionen, die geeignet sind, um zu einem Niederschlag an Array-Elementen zu führen, an denen eine Wechselwirkung zwischen Target und Sonde erfolgt ist:

Tabelle 1

| Katalysator bzw. Kristallisationskeim | Substrat |
|---|---|
| Meerrettichperoxidase | DAB (3,3'-Diaminobenzidin)<br>4-CN (4-Chlor-1-Napthol)<br>ABC (3-Amino-9-Ethylcarbazol)<br>HYR (p-Phenylendiamin-HCl und Pyrocatechol)<br>TMB (3,3',5,5'-Tetramethylbenzidin)<br>Naphtol/Pyronin |
| Alkalische Phosphatase | Bromchlorindoylphosphat (BCIP) und Nitrotetrazoliumblau (NBT) |
| Glucoseoxidase | t-NBT und m-PMS<br>(Nitrotetrazoliumblauchlorid und Phenazinmethosulfat |
| Goldpartikel | Silbernitrat<br>Silbertartrat |

Die Markierung von biologischen Proben mit Enzymen bzw. Gold, insbesondere nanokristallinem Gold ist hinlänglich beschrieben (siehe u.a. F. Lottspeich und H. Zorbas, Bioanalytik, Spektrum Akademischer Verlag, Heidelberg, Berlin, 1998; E. Lidell und I. Weeks, Antibody Technology, BIOS Scientific Publishers Limited, 1995).

Weitere Möglichkeiten für den Nachweis der Sonde/Target-Wechselwirkungen über unlösliche Präzipitate bei dem erfindungsgemäßen Verfahren sind beschreiben in: Immunogold-Silver Staining, Principles, Methods and Applications, Hrsg.: M.A.Hayat, 1995, CRC Press; Eur J Immunogenet 1991 Feb-Apr;18(1-2):33-55 HLA-DR, DQ and DP typing using PCR amplification and immobilized probes. Erlich H, Bugawan T, Begovich AB, Scharf S, Griffith R, Saiki R, Higuchi R, Walsh PS. Department of Human Genetics, Cetus Corp., Emeryville, Californis 94608; Mol Cell Probes 1993 Jun;7(3):199-207 A combined modified reverse dot-blot and nested PCR assay for the specific non-radioactive detection of Listeria monocytogenes. Bsat N, Batt CA. Department of Food Science, Comell University, Ithaca, NY 14853. Immunogenetics 1990;32(4):231-41 Erratum in: Immunogenetics 1991;34(6):413 Rapid HLA-DPB typing using enzymatically amplified DNA and nonradioactive sequence-specific oligonucleotide probes. Bugawan TL, Begovich AB, Erlich HA. Department of Human Genetics, Cetus Corporation, Emeryville, CA 94608. Hum Immunol 1992 Dec; 35(4):215-22 Generic HLA-DRB1 gene oligotyping by a nonradioactive reverse dot-blot methodology. Eliaou JF, Palmade F, Avinens O, Edouard E, Ballaguer P, Nicolas JC, Clot J. Laboratory of Immunology, Saint Eloi Hospital, CHU Montpellier, France. J Immunol Methods 1984 Nov 30;74(2):353-60 Sensitive visualization of antigen-antibody reactions in dot and blot immune overlay assays with immunogold and immunogold/silver staining. Moeremans M, Daneels G, Van Dijck A, Langanger G, De Mey J. Histochemistry 1987;86(6):609-15 Non-radioactive in situ hybridization. A comparison of several immunocytochemical detection systems using reflection-contrast and electron microscopy. Cremers AF, Jansen in de Wal N, Wiegant J, Dirks RW, Weisbeek P, van der Ploeg M, Landegent JE.

Im Rahmen der vorliegenden Erfindung sind u.a. folgende Varianten für den Nachweis der Sonde/Target-Wechselwirkungen über unlösliche Präzipitate denkbar.

Bei einer Ausführungsform der vorliegenden Erfindung werden die Targets mit einem Katalysator, vorzugsweise einem Enzym versehen, das die Umwandlung eines löslichen Substrats in ein unlösliches Produkt katalysiert. Die Reaktion, die zur Bildung eines Niederschlags an den Array-Elementen führt, ist in diesem Fall die Umwandlung eines löslichen Substrats in ein unlösliches Produkt in Gegenwart eines mit den Targets gekoppelten Katalysators, vorzugsweise Enzyms. Das Enzym wird vorzugsweise ausgewählt aus der Gruppe, bestehend aus Meerettichperoxidase, alkalischer Phosphatase und Glucoseoxidase. Das lösliche Substrat wird vorzugsweise ausgewählt aus der Gruppe, bestehend aus 3,3'-Diaminobenzidin, 4-Chlor-1-naphthol, 3-Amino-9-ethylcarbazol, p-Phenylendiamin-HCl/Pyrocatechol, 3,3',5,5'-Tetramethylbenzidin, Naphthol/Pyronin, Bromchlorindoylphosphat, Nitrotetraazoliumblau und Phenazinmethosulfat. Beispielsweise wird ein farbloser löslicher Wasserstoffdonor, z.B. 3,3'-Diaminobenzidin, in Anwesenheit von Wasserstoffperoxid in ein unlösliches farbiges Produkt umgewandelt. Das Enzym Meerrettichperoxidase überträgt Wasserstoffionen aus den Donoren auf Wasserstoffperoxid unter Bildung von Wasser.

Bei einer bevorzugten Ausführungsform der vorliegenden Erfindung ist die Reaktion, die zur Bildung eines Niederschlags an den Array-Elementen führt, die Bildung eines metallischen Niederschlags. Besonders bevorzugt ist die zur Bildung eines Niederschlags an den Array-Elementen führende Reaktion die chemische Reduktion einer Silberverbindung, vorzugsweise Silbernitrat, Silberlactat, Silberacetat oder Silbertartrat, zu elementarem Silber. Als Reduktionsmittel werden vorzugsweise Formaldehyd und/oder Hydrochinon eingesetzt.

Besonders bevorzugt erfolgt die Ausfällung der metallischen Verbindung in Gegenwart von mit den Targets gekoppelten Metallclustern bzw. kolloidalen Metallpartikeln, insbesondere Goldclustern oder kolloidalen Goldpartikeln. D.h. in diesem Fall stellen die Metallcluster bzw. kolloidalen Metallpartikel die mit den Targets gekoppelten Markierungen dar. Beispielsweise wird Silbernitrat in elementares Silber umgesetzt, wobei sich Silberionen aus der Lösung an Gold als Kristallisationskeim anlagern und in einem zweiten Schritt mit Hilfe eines Reduktionsmittels wie z.B. Formaldehyd oder Hydrochinon reduziert werden. Hierbei entsteht ein unlöslicher Niederschlag elementaren Silbers.

Bei einer alternativen Ausführungsform erfolgt die Ausfällung der metallischen Verbindung in Gegenwart von mit den Targets gekoppelten Polyanionen. Wenn es sich beim Target selbst nicht um ein Poly-Anion handelt, dann besteht die Möglichkeit, ein solches zur Keimbildung einzusetzen. Das mit einem Poly-Anion markierte Target wird beispielweise einer Silbernitratlösung ausgesetzt. Dabei lagern sich die Silber-Kationen am Poly-Anion selektiv an. Danach werden mit einem Reduktionsmittel Silberionen in elementares Silber umgewandelt.

Die Kopplung der Enzyme bzw. Katalysatoren bzw. Metallcluster bzw. kolloidalen Metallpartikel bzw. Polyanionen an die Targets kann direkt oder über an die Targets gekoppelte Ankermoleküle erfolgen. Grundsätzlich besteht keine Notwendigkeit, das Target direkt mit den oben beschriebenen Markierungen zu versehen. Es besteht auch die Möglichkeit, über geeignete Ankermoleküle, z.B. Streptavidin, die an das Target gekoppelt werden, eine nachfolgende Ankopplung der Markierung zu erreichen.

Ein Konjugat bestehend aus dem jeweiligen Katalysator bzw. Kristallisationskeim und einem spezifischen Bindungspartner für das Ankermolekül erlaubt ebenfalls die Durchführung der oben beschriebenen Prozeduren. Die Reaktion, die zur Bildung eines Niederschlags an den Array-Elementen führt, ist dann die Bindung eines spezifischen Bindungspartners an ein an das Target gekoppeltes Ankermolekül darstellt.

Derartige Bindungspartner/Ankermolekül-Paare werden vorzugsweise ausgewählt aus der Gruppe, bestehend aus Biotin/Avidin bzw. Streptavidin bzw. Anti-Biotin-Antikörper, Digoxigenin/Antidigoxigenin-Immunoglobulin, FITC/Anti-FITC-Immunoglobulin und DNP/Anti-DNP-Immunoglobulin.

In jeder der vorstehend beschriebenen Ausführungsformen wird katalytisch ein lösliches Substrat in ein unlösliches und ausfallendes Produkt umgesetzt. Aufgrund der Nähe zur Oberfläche wird das Produkt unmittelbar an der Oberfläche abgeschieden und bildet einen festen, für diverse Wäschen unempfindlichen Niederschlag.

Ferner ist es im Rahmen der vorliegenden Erfindung möglich, dass die Markierungen, insbesondere die Enzyme bzw. Metallcluster bzw. kolloidalen Metallpartikel bzw. Polyanionen, an die Targets vor, während oder nach der Wechselwirkung mit den Sonden gekoppelt werden.

Bei einer bevorzugten Ausführungsform der vorliegenden Erfindung ist die Wechselwirkung zwischen dem Target und der Sonde eine Hybridisierung zwischen zwei Nukleotidsequenzen. Die Hybridisierung der Targets mit den auf einem Sonden-Array angeordneten Sonden findet nach einem der bekannten Standardprotokolle statt (siehe u.a. Lottspeich und Zorbas, 1998).

Die entstandenen Hybride können durch kovalente Bindung, beispielsweise über Psoralen-Interkalation und nachfolgendes "Cross-Linking", oder wie in US 4,599,303 beschrieben, durch nichtkovalente Bindung, beispielsweise durch Bindung von Interkalatoren, stabilisiert werden.

Im Anschluss an die Hybridisierung der Targets mit den auf einem Sonden-Array angeordneten Sonden bzw. der Markierung der hybridisierten Targets erfolgt üblicherweise ein Waschschritt, mit dem unspezifisch und dadurch schwächer gebundene Komponenten entfernt werden.

Alternativ ist die Wechselwirkung zwischen dem Target und der Sonde eine Reaktion zwischen einer Antigenstruktur und dem entsprechenden Antikörper oder einem hypervariablen Abschnitt davon oder eine Reaktion zwischen einem Rezeptor und einem entsprechenden Liganden.

Die Bindung oder Erkennung der Targets durch spezifische Sonden ist üblicherweise ein spontane nicht-kovalente Reaktion unter optimalen Bedingungen. Davon umfasst sind ebenfalls nicht-kovalente chemische Bindungen. Die Zusammensetzung des Mediums sowie weitere chemische und physikalische Faktoren beeinflussen die Geschwindigkeit und Stärke der Bindung. So erniedrigen beispielsweise bei der Nukleinsäure-Erkennung eine niedrigere Stringenz und höhere Temperaturen die Rate und Stärke der Bindung zwischen zwei nicht perfekt komplementären Strängen. Die Optimierung der Bindungsbedingungen ist für Antigen/Antikörper- oder Ligand/Rezeptor-Wechselwirkungen ebenfalls erforderlich, die Bindungsbedingungen sind aber üblicherweise weniger spezifisch.

Der Nachweis der Gegenwart eines Niederschlags auf einem Array-Element erfolgt bei einer Ausführungsform der vorliegenden Erfindung durch Reflexion, Absorption oder Diffusion eines Lichtstrahls, vorzugsweise eines Laserstrahls oder einer Leuchtdiode, durch den Niederschlag. Aufgrund seiner granularen Form modifiziert der Niederschlag die Reflexion eines Lichtstrahls. Ferner führt der Niederschlag zu einer starken Lichtdiffusion die durch herkömmliche Detektionsvorrichtungen aufgezeichnet werden kann. Falls der Niederschlag wie beispielsweise der Silberniederschlag als dunkle Oberfläche erscheint, kann auch die Absorption von Licht detektiert und aufgezeichnet werden. Die Auflösung der Detektion hängt dann von der Anzahl der Pixel der Kamera ab. Bei einer besonders bevorzugten Ausführungsform erfolgt die Detektion der Gegenwart eines Niederschlags auf einem Array-Element, in dem die Lichtquelle die Array-Elemente scannend mit einer Abtastgeschwindigkeit homogen beleuchtet, wobei die Abtastgeschwindigkeit besonders bevorzugt gewährleistet, dass eine Belichtungszeit der aufnehmenden Kamera alle Signale der Probe zu einem Bild vereinigt.

Beispielsweise kann der Nachweis der durch die spezifische Reaktion verstärkten Bereiche mittels eines sehr einfachen optischen Aufbaus im Durchlicht (Kontrast durch Abschattung) bzw. Auflicht (Kontrast durch Reflexion) erfolgen. Die detektierte Intensität des abgeschatteten Bereiches ist direkt proportional zur Belegungsdichte mit Markierungen wie beispielsweise Gold-Partikeln und dem Keimbildungszustandes der Partikel.

Bei Verwendung eines Niederschlags, der elektrisch leitfähig ist oder dessen Dielektrizitätskonstante sich von der Umgebung unterscheidet, ist der Nachweis der Reaktion bei einer alternativen Ausführungsform der vorliegenden Erfindung auch elektrisch möglich.

Die elektrischen Messungen können über Leitfähigkeitsmessungen mittels Mikroelektrodenarray-Anordungen oder über eine Anordnung von Mikrokapazitätssensoren oder über Potentialmessungen mittels Feld-Effekt-Transistoren-Arrays (FET-Arrays) erfolgen. Bei Leitfähigkeitsmessungen mittels Mikroelektroden wird die Änderung des elektrischen Widerstandes zwischen zwei Elektroden bei einer Abscheidungsreaktion verfolgt (E. Braun, Y. Eichen, U. Sivan, G. Ben-Yoseph, Nature, 775, vol 391, 1998). Bei Dielektrizitätsmessungen mit Mikrokapazitätssensoren wird die Änderung der Kapazität zweier zueinander angeordneten Elektroden gemessen (M. Madou, Fundamentals of Microfabrication, CRC Press, Boca Raton, 1997). Bei Potentialmessungen mittels FET-Arrays wird die Änderung des Potentials auf den Sensoroberflächen gemessen (M. Madou, Fundamentals of Microfabrication, CRC Press, Boca Raton, 1997).

Bei Verwendung eines Substrates, das radioaktiv ist, oder radioaktiv markiert ist, kann der Nachweis der Gegenwart eines Niederschlags auf einem Array-Element durch Autoradiographie, Fluorographie und/oder indirekte Autoradiographie erfolgen. So wird bei der Autoradiographie eine mit strahlendem Präzipitat bedeckte Fläche direkt mit einem Röntgenfilm in Kontakt gebracht. Bei der Fluorographie wird eine mit strahlendem Präzipitat bedeckte Fläche mit fluoreszierenden Chemikalien wie z.B. Natriumsalicylat überschichtet, die die radioaktive Strahlungsenergie in Fluoreszenz umwandeln. Bei der indirekten Autoradiographie mit Verstärkungsfolien (intensifier screens) wird eine mit β-strahlendem Präzipitat bedeckte Fläche auf eine Verstärkerfolie gelegt, die die Strahlung in blaues Licht umwandelt. (siehe F. Lottspeich, H. Zorbas, siehe oben). Auf Radioaktivität basierende Detektionsverfahren sind jedoch aufgrund der gesundheitlichen Risiken und der deswegen zu erfüllenden Sicherheitsvorschriften häufig unerwünscht

Bei weiteren alternativen Ausführungsformen der vorliegenden Erfindung erfolgt der Nachweis der Gegenwart eines Niederschlags auf einem Array-Element durch Rasterelektronenmikroskopie, Elektronensonden-Mikroanalyse (EPMA), magnetooptische Kerr-Mikroskopie, magnetic force-Mikroskopie (MFM), atomic force-Mikroskopie (AFM), Messung des Mirage-Effekts, Rastertunnelmiktoskopie (STM) und/oder Ultraschall-Reflexions-Tomographie.

Nahezu unabhängig von der Art des Substrates ist der Nachweis der Reaktion mittels SEM und/oder EPMA. Bei der Rasterelektronen-Mikroskopie (scanning electron microscopy (SEM)) tastet ein fokussierter Elektronenstrahl die Probe ab (J. Goldstein et al. Scanning Electron Microscopy and X-Ray Microanalysis, Plenum, New York, 1981). Bei der electron probe microanalysis (EPMA) werden die Sekundärprozesse, die durch einen fokussierten Elektronenstahl ausgelöst werden, zur ortsaufgelösten Analyse genutzt (J. Goldstein et al. Scanning Electron Microscopy and X-Ray Microanalysis, Plenum, New York, 1981).

Bei Verwendung eines Substrates, das magnetisch ist, oder mit Magnetpartikeln markiert ist, kann der Nachweis der Reaktion durch Magnetooptische Kerr-Mikroskopie oder MFM erfolgen. Bei der Magnetooptischen Kerr-Mikroskopie wird die Drehung der Polarisationsebene des Lichtes durch magnetische Felder (Kerr- und Faradayeffekt) ausgenutzt (A. Hubert, R. Schäfer, Magnetic Domains, Springer, 1998).

Die Änderung der optischen Dichte durch das Substrat auf der Oberfäche infolge der Reaktion kann mittels des Mirrage-Effektes detektiert werden. Beim Mirrage-Effekt wird die lokale Erwärmung einer Oberfläche durch Absorption eines gebündelten Laserstrahls über die damit verbundene Brechungsindexänderung gemessen. Durch das Abrastern der Oberfläche erhält man ein Bild von den lokalen Oberflächenabsorptionseigenschaften (A. Mandelis, Progress in Photothermal and Photoacoustic Science and Technology, Volume 1, Elsevier, New York 1992). Ein weiteres thermisches ortsaufgelöstes Verfahren zum Nachweis der Wechselwirkungsreaktion durch das Substrat ist eine Array-Anordung von Mikrothermophiles, die die Kristallisations- bzw. Abscheidungsenthalpien der Substratabscheidungen messen (J.M. Köhler, M. Zieren, Thermochimica acta, 25, vol 310, 1998).

Ebenfalls geeignet zum Nachweis der Reaktion mittels Substrat sind STM und AFM. Beim atomic force microscope (AFM) tastet eine Mikro- oder Nanospitze die Oberflächen ab, wodurch die Oberflächentopographie vermessen wird (E. Braun, Y. Eichen, U. Sivan, G. Ben-Yoseph, Nature, 775, vol 391, 1998). Das magnetic force microscope MFM detektiert über eine Nanospitze lokale magnetische Suszeptibilitätsunterschiede (A.Hubert, R.Schäfer, Magnetic Domains, Springer, 1998). Beim scanning tunneling microscop STM wird über eine Nanospitze der Tunnelstrom gemessen, um die Nanooberflächentopographie zu ermitteln (O. Marti, M. Amrein, STM and SFM in biology, Academic Press Inc., San Diego, 1993)

Exotischere Verfahren wie die Ultraschall-Reflexions-Tomographie können ebenfalls zur Anwendung kommen. Bei den Tomografien handelt es sich um Verfahren, bei denen scheibchenweise ein 3-dimensionales Bild zusammengetragen wird (F. Natterer, Mathematische Methoden der Computer-Tomographie, Westdt. Vlg., Wiesbaden, 1997). Im Falle der Ultraschall-Reflexions-Tomographie wird die Messung der Ultraschall - Reflexion ausgenutzt, um das Tomogramm zu erzeugen (V. Fleischer, F. Bergner, DGZfP NDT Conference Dresden 1997).

Ein weiterer Aspekt der vorliegenden Erfindung betrifft eine Vorrichtung zur Durchführung des vorstehend beschriebenen Verfahrens, umfassend:

a) mindestens ein vorstehend beschriebenes erfindungsgemäßes Reaktionsgefäß; und
b) eine Detektionsvorrichtung zum Nachweis der spezifischen Wechselwirkung.

Bei Verwendung einer reaktiv verstärkend wirkenden Färbemethode der Wechselwirkung wie der vorstehend beschriebenen Iramunogold/Silber-Anfärbung kann die Detektion im Gegensatz zu den üblicherweise eingesetzten Fluoreszenzmessungen durch einfache Absorptions- bzw. Reflexionsmessungen erfolgen, so dass die Gesamtkosten für die erfindungsgemäße Vorrichtung sehr niedrig sind.

Vorzugsweise wird die erfindungsgemäße Vorrichtung bei einem Nachweisverfahren eingesetzt, bei dem wie vorstehend beschrieben die Detektion über eine Reaktion erfolgt, die zu einem Niederschlag an den Array-Elementen führt, an denen eine Wechselwirkung zwischen Sonden und Targets stattgefunden hat. In dem Reaktionsgefäß, das in die Detektionsvorrichtung bzw. das Lesegerät eingesetzt werden kann, bilden sich auf einigen Array-Elementen aufgrund der spezifischen Wechselwirkung der Probe bzw. des Targets mit den Sonden Wechselwirkungsprodukte in Form eines Niederschlags. Diese Wechselwirkungsprodukte weisen einen anderen Absorptionskoeffizienten als die reinen Substanzen auf. Durch geeignete Reaktionen kann dieser Effekt wie vorstehend beschrieben erheblich verstärkt werden.

Die Detektionsvorrichtung ist vorzugsweise eine Kamera, insbesondere eine CCD- oder CMOS-Kamera oder ähnliche Kameras, die üblicherweise den gesamten Bereich des Sonden-Arrays aufzeichnet. Alternativ können auch scannende Verfahren für die Detektionsvorrichtung zum Auslesen des Reaktionsgefäßes eingesetzt werden.

Bei speziellen Ausgestaltungen der erfindungsgemäßen Vorrichtung umfasst die Detektionsvorrichtung zusätzlich eine Ausleseoptik.

Bei einer bevorzugten Ausführungsform umfasst die erfindungsgemäße Vorrichtung zusätzlich mindestens eine Lichtquelle. Eine Lichtquelle im Rahmen der vorliegenden Erfindung gewährleistet vorzugsweise eine homogene Beleuchtung des Trägers. Besonders bevorzugt wird die Lichtquelle ausgewählt aus der Gruppe, bestehend aus Lasern, Licht-emittierenden Dioden (LED), Flächenstrahlern und Hochdrucklampen.

Neben punktförmigen Lichtquellen können auch Lichtquellen in Form von Beleuchtungsarrays in der erfindungsgemäßen Vorrichtung Verwendung finden. Eine homogene Beleuchtung des Trägers kann in dieser Ausgestaltung beispielsweise dadurch gewährleistet werden, dass die Lichtquelle mehrere diffus strahlende Lichtquellen umfasst, deren Überlagerung in einer homogenen Beleuchtung resultiert. So ermöglichen beispielsweise diffus streuende LED, die matrixförmig angeordnet sind, auf kurze Entfernungen zur Probe eine homogene Beleuchtung.

Die Homogenität der Beleuchtung kann auch durch eine entsprechende Strukturierung des Deckels des erfindungsgemäßen Reaktionsgefäßes erreicht werden. Auf diese Weise übernimmt der Deckel des erfindungsgemäßen Reaktionsgefäßes die Funktion einer Streuscheibe.

Bei einer weiteren bevorzugten Ausführungsform ist die Lichtquelle in einen Schwenkarm eingebaut, der zum Einführen des Reaktionsgefäßes in die Vorrichtung zur Seite geschwenkt werden kann. Danach ist er über das Reaktionsgefäß schwenkbar, um die Affinitätsmatrix zu beleuchten. Auf diese Weise kann das Reaktionsgefäß in einen Reaktionsgefäßhalter gedrückt werden, so dass die Substanzbibliothek in der richtigen Abbildungsebene liegt. Eine Nachfokussierung der Abbildungsoptik bzw. Ausleseoptik wird dadurch überflüssig. Alternativ kann das Lesegerät bzw.

die Detektionsvorrichtung mit einer Fokussierungseinrichtung ausgestattet werden, um unterschiedliche Typen von Reaktionsgefäßen aufnehmen zu können.

Bei einer weiterem Ausgestaltung befindet sich in der erfindungsgemäßen Vorrichtung eine Justiervertiefung, in die der Deckel des Reaktionsgefäßes gedrückt werden kann. Dadurch wird eine Verdrehung der Affinitätsmatrix vermieden.

Die Komponenten eines beispielhaften Aufbaus einer erfindungsgemäßen Vorrichtung zum optischen Nachweis einer Niederschlagsbildung bestehen aus einer leistungsschwachen (500 mcd) Lichtquelle, z.B. einer LED, zur homogenen Beleuchtung und einem Detektor, z.B. einer CCD-Kamera. Aufgrund des Verstärkungseffekts über die katalytische Abscheidung des Substrats, insbesondere bei der Verwendung eines Gold/Silber-Systems, sind die Änderungen der optischen Eigenschaften der Oberfläche derart ausgeprägt, dass ein einfacher Flachbettscanner, ein Diascanner oder ein vergleichbares Gerät zur Detektion des Niederschlags ausreicht.

Typische Detektionszeiten liegen deutlich unter einer Sekunde, während vergleichbare sensitive CCD-Systeme zur Detektion von Fluoreszenz etwa 10-18 Sekunden benötigen, so dass preiswerte Consumer-Kameras Verwendung finden können, deren Signalübertragung der Videonorm entspricht.

Bei einer weiteren vorteilhaften Ausgestaltung der erfindungsgemäßen Vorrichtung umfasst die Vorrichtung zusätzlich optische Filter. Derartige Filter ermöglichen zum einen die spektrale Eingrenzung der homogenen Beleuchtung und zum anderen die Beleuchtung der Proben mit verschiedenen Wellenlängen. Bei einer weiteren Variante umfasst die erfindungsgemäße Vorrichtung zusätzlich Filterwechsler. Mit diesen Filterwechslern können die optischen Filter schnell gewechselt werden und somit mögliche Fehlinformationen, die z.B. durch Verunreinigungen auftreten, eindeutig erkannt und eliminiert werden.

Bei einer weiteren bevorzugten Ausgestaltung, insbesondere wenn die Reflektivität des Trägerelements gemessen wird, umfasst die Vorrichtung zusätzlich einen halbdurchlässigen Spiegel zwischen Lichtquelle und Trägerelement. Bei dieser Ausführungsform gelangt das Licht der Lichtquelle durch einen halbdurchlässigen Spiegel auf die Probe und das Bild wird in Reflexion durch den halbdurchlässigen Spiegel und ggf. die Ausleseoptik auf eine Kamera abgebildet.

Bei einer bevorzugten Ausführungsform zur Messung der Reflektivität befindet sich zusätzlich ein Oberflächenspiegel auf der Unterseite des Trägerelements. Der Nachteil der schlechten Reflexion der Probe wird in dieser Ausgestaltung durch Transmissionseffekte ergänzt, indem das Beleuchtungslicht über eine Spiegelschicht hinter der Probe, entweder als eigenständiger Spiegel oder als auf der Rückseite des Probenträgers aufgetragene Schicht, reflektiert wird. Dabei kann beispielsweise ein Flächenstrahler auf der gegenüberliegenden Seite des Trägerelements und damit auch zum Sensor beispielsweise einer CCD-Kamera angeordnet werden. Auf diese Weise wird eine sehr kompakte Anordnung ermöglicht.

Bei einer weiteren Ausführungsform befindet sich die Probe in einer Küvette, die mittels einer Ausleseoptik unmittelbaren Kontakt zum Sensor einer CCD-Kamera hat. In diesem Fall ist die Ausleseoptik bzw. das Koppelmedium zwischen Küvette und Kamera vorzugsweise eine Faserplatte, erhältlich beispielsweise von der Firma Schott, oder ein Bildleitkabel. In dieser Ausführungsform ist das erfindungsgemäße Reaktionsgefäß eine Küvette aus zwei planparallelen Platten mit dazwischenliegendem Probenvolumen. Der Chip mit der oberflächengebunden Substanzbibliothek ist in dieser Küvette eingesetzt und kann ähnlich wie Küvetten in einem Spektrometer ausgelesen werden.

Vorzugsweise weist die erfindungsgemäße Vorrichtung zusätzlich eine Temperatursteuerungseinheit auf, durch die gewährleistet wird, dass die Probe während der Messung temperaturstabil gelagert wird. Dies gewährleistet die Reproduzierbarkeit der Ergebnisse.

Bei einer weiteren Ausführungsform sind mehrere Reaktionsgefäße in der Vorrichtung so angeordnet, dass eine aufeinander folgende Detektion gewährleistet ist. Beispielsweise kann eine zeitlich versetzte, aufeinanderfolgende Auslesung durch Anordnung der Proben in Form von Magazinen oder einem Karussell in der erfindungsgemäßen Vorrichtung erreicht werden.

Ferner ist es bevorzugt, dass die erfindungsgemäße Vorrichtung zusätzlich einen Computer umfasst, der programmiert ist, um:

- die von der Detektionsvorrichtung aufgenommenen Signalintensitäten zu sammeln; und
- gegebenenfalls die Umwandlung der Signalintensitäten in ein analoges Bild zu gewährleisten.

Unter einem Bild wird im Rahmen der vorliegenden Erfindung eine Gruppe von Pixeln verstanden, die eine Veranschaulichung der gemessenen Signalintensitäten für einen Sonden-Array darstellt und die direkt beispielsweise an einen Bildschirm oder einen Drucker zur Aufzeichnung übermittelt werden kann.

Wie bereits vorstehend ausführlich beschrieben, lässt sich durch zeitaufgelöste Detektion während des Verstärkungsprozesses durch die Ablagerung eines Niederschlags, wie beispielsweise elementarem Silber auf als Kristallisationskeimen wirkenden Goldpartikeln, und die Berechnung der relativen Belegungsdichten aus dem Zeitverhalten nach dem erfindungsgemäßen Verfahren die dynamische Auflösung der Messdaten selbst bei Verwendung einer

8 bit-Detektionstechnik extrem erhöhen. Der Aufbau einer hierzu nötigen Vorrichtung unterscheidet sich durch die mechanische Aufnahme einer Reaktionskammer und eine modifizierte Akquisitions-Software. Die Software, beispielsweise die Software Iconoclust® (Clondiag), ist dadurch gekennzeichnet, dass sie die Verarbeitung der sukzessive aufgenommenen Aufnahmen gestattet. Hierzu werden die über den einzelnen Sondenarray-Elementen ermittelten Grauwerte zu jedem Zeitpunkt ermittelt. Für alle Array-Elemente wird die virtuelle Signalintensität in Abhängigkeit der Niederschlagbildung von der Zeit berechnet. Ausgehend von diesem Wert werden beispielsweise die Grauwerte der letzten Messung mit dem Produkt aus Rate und Messdauer in Beziehung gesetzt und damit eine Spreizung des Messbereichs erreicht. Somit ist selbst bei Verwendung von kostengünstigen 8 bit -Kameras eine ausgezeichnete Auflösung von schwachen neben starken Sonden/Target-Wechselwirkungen sowie eine genaue Quantifizierung der gebundenen Targets gewährleistet.

Das Miniaturisierungspotential eines solchen Aufbaus ist sehr hoch, so dass das gesamte System als autonomes Handgerät für den Feldeinsatz konzipiert werden kann. Ferner kann die erfindungsgemäße Vorrichtung bei einer besonders bevorzugten Ausführungsform als hochintegrierte autonome Einheit realisiert werden. Damit sind hoch sensitive Anwendungen von Mikro-Arrays wie z.B. medizinische Diagnostik, Forensik, bakterielle Screening u. dgl. unabhängig von medizinischen bzw. biologischen Laboratorien von Laien schnell durchführbar.

Das erfindungsgemäße Reaktionsgefäß umfasst somit ein Standard-Laborreaktionsgefäß bzw. Standard-Microtube mit einem an einer der Grundflächen, z. B. an der Spitze oder im Deckel eines herkömmlichen Reaktionsgefäßes wie z.B. einem handelsüblichen Eppendorff-Tube, insertierten Träger bzw. Chip bzw. Array. Diese Gestaltung erlaubt eine wesentlich leichtere Handhabbarkeit und eine höhere Reproduzierbarkeit. Lösungen können leicht in das erfindungsgemäße Reaktionsgefäß pipeltiert werden und unter Verwendung von in nahezu jedem, insbesondere in molekularbiologischen Laboren vorhandener Microtube-Ausstattung inkubiert und verarbeitet, z. B. zentrifugiert werden.

Im Folgenden werden spezielle Ausgestaltungen der erfindungsgemäßen Vorrichtung anhand von Abbildungen detailliert beschrieben:

In den Abbildungen 5 bis 12 werden verschiedene optische Prinzipien verdeutlicht, die zum Auslesen von oberflächengebundenen Substanzbibliotheken (102) herangezogen werden können. In dem Reaktionsgefäß, das in das Detektions- bzw. Lesegerät eingesetzt wird, findet eine spezifische Wechselwirkung der Probe bzw. des Targets mit Sonden einiger Bibliothekselemente der Substanzbibliothek (102) statt, wodurch sich auf einigen Flächen Wechselwirkungsprodukte bilden.

Diese Wechselwirkungsprodukte weisen einen anderen Absorptionskoeffizienten als die reinen Substanzen auf, wobei dieser Effekt durch geeignete Reaktionen noch erheblich verstärkt werden kann. Durch die in den Abbildungen 5 bis 17 dargestellten Auslesesysteme (1000) die Absorptions- oder Reflektionsverhältnisse der oberflächengebundenen Substanzbibliothek vor, während und/oder nach der Wechselwirkungsreaktion abgebildet.

In den Abbildungen 5 bis 12 wurde zur besseren Verdeutlichung des Ausleseprinzips die Darstellung des Reaktionsgefäßes (1) auf die Darstellung der oberflächengebundenen Substanzbbliothek (100) beschränkt. In den Abbildungen 13 bis 17 sind zwei konkrete Ausführungsformen zum Abbilden eines Niederschlages auf oberflächengebundenen Substanzbibliotheken (100) dargestellt. Anstelle der hier abgebildeten Lesegeräte (1000) können auch scannende Verfahren zum Auslesen der Reaktionsgefäßes (1) eingesetzt werden.

Des Weiteren erfolgt die Beschreibung der Abbildung 5 bis 17 anhand der Verwendung von DNA-Chips (100) in Verbindung mit der vorstehend beschriebenen Silberabscheidungsreaktion, ist aber selbstverständlich nicht auf diese beschränkt.

In Abbildung 5 ist die prinzipielle Anordnung der erfindungsgemäßen Vorrichtung zum Auslesen der Reaktionsgefäßes (1) dargestellt. Licht einer inkohärenten Lichtquelle (1001) leuchtet mittels einer Beleuchtungsoptik (1002) die oberflächengebundene DNA-Bibliothek auf einem Bibliothekenchip (100) aus, der sich in einem Tube (1) befindet. Mittels einer Ausleseoptik (1004) wird das Signal von einer CCD-Kamera (1005) aufgenommen.

Die Abbildung 6 zeigt die gleiche Anordnung wie die der Abbildung 5, mit der Variante, durch Einbringen von optischen Filtern (1006) in den Beleuchtungsstrahlengang den spektralen Bereich der Beleuchtung einzugrenzen. Licht einer inkohärenten Lichtquelle (1001) leuchtet mittels einer Beleuchtungsoptik (1002) die oberflächengebundene DNA-Bibliothek auf einem Bibliothekenchip (100) aus, der sich in einem Reaktionsgefäß (1) befindet. Mittels einer Ausleseoptik (1004) wird das Signal von einer CCD-Kamera (1005) aufgenommen. Die Möglichkeit, diesen Filter mittels Filterwechsler (1007) schnell zu wechseln, hat für die Auswertung den Vorteil, mögliche Fehlinformationen, die z.B. durch Verunreinigungen auftreten, eindeutig zu erkennen und zu eliminieren.

In der in Abbildung 7 gezeigten Vorrichtung wird die Punktlichtquelle (1001) durch ein Beleuchtungsarray (1008) ersetzt. Licht eines Beleuchtungsarray (1008) leuchtet mittels einer Beleuchtungsoptik (1002) die oberflächengebundene DNA-Bibliothek (102) auf einem Bibliothekenchip (100) aus, der sich in einem Reaktionsgefäß (1) befindet homogen aus. Mittels einer Ausleseoptik (1004) wird das Signal von einer CCD-Kamera (1005) aufgenommen. Vorzugsweise diffus streuende LED, matrixförmig angeordnet, ermöglichen auf kurze Entfernungen zur Probe eine homogene Beleuchtung.

In der Anordnung der erfindungsgemäßen Vorrichtung gemäß Abbildung 8 wird ein kompaktes Lesegerät (1000) dargestellt, bei der der CCD-Sensor (1005) der Kamera unmittelbar mit der Affinitätsmatrix (100) kontaktiert ist. Dieser Kontakt kann auch durch den Einsatz einer Faserplatte (1012) oder bei noch größeren Abständen durch ein Bildleitkabel hergestellt werden, wenn ein unmittelbarer Kontakt nicht möglich ist. Licht einer inkohärenten Lichtquelle (1001) leuchtet mittels einer Beleuchtungsoptik (1002) die oberflächengebundene DNA-Bibliothek auf einem Bibliothekenchip (100) aus, der sich in einem Reaktionsgefäß (1) befindet. Das Signal der Probe wird direkt von einer CCD-Kamera (1005) aufgenommen.

In dem in Abbildung 9 gezeigten Lesegerät (1000) werden die Proben im Auflicht vermessen. Im Unterschied zu den vorher beschriebenen Lesegeräten bzw. Detektionsvorrichtungen (1000) wird bei diesem Gerät die Reflektivität der DNA-Bibliothek (102) gemessen. Licht einer inkohärenten Lichtquelle (1001) leuchtet mittels einer Beleuchtungsoptik (1002) durch einen halbdurchlässigen Spiegel die oberflächengebundene DNA-Bibliothek auf einem Bibliothekenchip (100) aus, der sich in einem Reaktionsgefäß (1) befindet. Durch den halbdurchlässigen Spiegel (1009) wird das reflektierte Signal auf die Ausleseoptik (1004) umgelenkt und von dieser auf eine CCD-Kamera (1005) abgebildet.

Da der Silberniederschlag keine sehr guten Reflexionseigenschaften besitzt, ist bei Auflichtmessungen die in Abbildung 10 gezeigte Anordnung vorteilhaft. Licht einer inkohärenten Lichtquelle (1001) leuchtet mittels einer Beleuchtungsoptik (1002) durch einen halbdurchlässigen Spiegel die oberflächengebundene DNA-Bibliothek (102) auf einem Bibliothekenchip (100) aus, der sich in einem Reaktionsgefäß (1) befindet. Durch den halbdurchlässigen Spiegel (1009) wird das reflektierte Signal auf die Ausleseoptik (1004) umgelenkt und von dieser auf eine CCD-Kamera (1005) abgebildet. Der Nachteil der schlechten Reflexion der Probe wird durch Transmissionseffekte ergänzt, indem das Beleuchtungslicht über eine Spiegelschicht (1010) hinter der Probe, entweder als eigenständiger Spiegel oder als auf der Rückseite des Probenträgers aufgetragene Schicht, reflektiert wird.

Abbildung 11 zeigt die mögliche Anordnung der erfindungsgemäßen Vorrichtung in einem transparenten Probenraum, vorzugsweise in einer Küvette (1011), die durch die optisch planen Außenflächen eine gute Kopplung des Empfängers mittels Faserplatte (1012) an den Probenraum ermöglichen. Licht einer inkohärenten Lichtquelle (1001) leuchtet mittels einer Beleuchtungsoptik (1002) durch die transparente Wand der Küvette die oberflächengebundene DNA-Bibliothek (102) auf einem Bibliothekenchip (100) aus. Durch die transparente Wand der Küvette wird das Signal der Probe direkt von einer CCD-Kamera (1005) aufgenommen. Als Koppelmedium zwischen Küvette und CCD-Kamera kann eine Faserplatte (1012) oder ein Bildleitkabel verwendet werden.

Die in der Abbildung 12 gezeigte Vorrichtung stellt eine sehr kompakte Anordnung des in Abbildung 11 dargestellten Lesegerätes (1000) dar. Bei dem Einsatz von diffusen Flächenstrahlern (1013), die z.B. auf Elektrolumineszenz- oder Kaltkathodenfluoreszenz-Verfahren beruhen, lassen sich sehr kompakte Sensoren realisieren. Licht einer inkohärenten Lichtquelle (1001) in diesem Falle eines diffusen Flächenstrahlern (1013) leuchtet durch die transparente Wand der Küvette den Bibliothekenchip (100) direkt aus. Das Signal der Probe wird direkt von einer CCD-Kamera (1005) aufgenommen.

In den Abbildung 13 und 14 ist ein Ausführungsbeispiel in der Aufsicht und als Schnittzeichnung eines Lesegerätes (1000) dargestellt. Das Arbeitsprinzip entspricht dem des in Abbildung 1 dargestellten Vorrichtung. Zwischen der Affinitätsmatrix (100) und der abbildenden Optik (1004) wurde ein Umlenkspiegel eingebaut, um zu kompakten Geräteausmaßen zu kommen. Das Licht der Lichtquelle (1008) leuchtet in das Reaktionsgefäß (1) und anschließend durch den DNA-Chip (100) auf einen Umlenkspiegel. Von dort wird die Abbildung des DNA-Chips (100) über die Abbildungsoptik (1004) auf eine CCD-Kamera (1005) projiziert. Die Lichtquelle (1008) besteht aus Leuchtdioden, die in einen Schwenkarm eingebaut sind, der zum Einführen des Reaktionsgefäßes (1) in das Lesegerät (1000) zur Seite geschwenkt werden kann. Danach wird er über das Reaktionsgefäß (1) geschwenkt, um die Affinitätsmatrix (100) zu beleuchten. Dabei drückt er das Reaktionsgefäß (1) in den Reaktionsgefäßhalter (1100), so dass die DNA-Bibliothek (102) in der richtigen Abbildungsebene liegt. Eine Nachfokussierung der Abbildungsoptik (1004) wird dadurch überflüssig. Auf der Oberseite befindet sich eine Justiervertiefung (1102), in die der Deckel des Reaktionsgefäßes (1) gedrückt wird. Dadurch wird eine Verdrehung der Affinitätsmatrix (102) vermieden. Um die Bedingungen in dem Reaktionsgefäß (1) reproduzierbar zu halten, befindet sich an dem Reaktionsgefäßhalter (1100) ein Peltierelement (1200). Dadurch kann die Temperatur im Reaktionsgefäß (1) geregelt werden. Der Reaktionsgefäßhalter (1100) kann zudem mit einer Widerstandsheizung versehen werden, um auch höhere Temperaturen einstellen zu können. Alternativ kann der Reaktionsgefäßhalter (1100) auch mit umlaufenden Kühlmedien temperiert werden.

In Abbildung 15 wird ein Lesegerät (1000) dargestellt, dass sich von dem in der Abbildung 13 dadurch unterscheidet, dass der Schwenkarm (1101) durch einen Schieber (1300) ersetzte wird. Der Schieber (1300) wird über ein Linearlager (1310) über das Reaktionsgefäß (1) geschoben. Dabei drückt ein Schlitten (1320) durch vier Druckfedern (1321) das Reaktionsgefäß (1) in den Reaktionsgefäßhalter (1100). Ein Justierspalt (1322) umgreift den Deckel (3) des Reaktionsgefäßes (1) derart, dass durch die Schiebebewegung des Schiebers (1300) das Reaktionsgefäß (1) in seine richtige Position gedreht und fixiert wird. In der Abbildung 17 wird die Lage des Schiebers (1300) zum Reaktionsgefäß (1) bei geöffnetem Reader dargestellt. Der Schlitten (1300) bewegt sich entlang des dort dargestellten Pfeils und verschließt dadurch den das Reaktionsgefäß (1). Gleichzeitig wird die Lichtquelle (1008) über das Reaktionsgefäß

(1) geschoben, so dass mit dem Lesegerät (1000) gemessen werden kann. Bei geschlossenem Schieber (1300) liegt der Schlitten (1320) zwischen

Lichtquelle und Reaktionsgefäß (1). Der Schlitten weist daher eine Detektionsöffuung (1323) auf, um die Affinitätsmatrix (100) beleuchten zu können.

Die folgenden Beispiele dienen der Erläuterung der Erfindung und sollen in nicht einschränkender Weise ausgelegt werden.

Beispiele

Beispiel 1: Herstellung des Reaktionsgefäßes (1)

Es wurde ein Standard-Reaktionsröhrchen aus Polypropylen mit 1,5 ml-Nennaufnahmevolumen der Firma Eppendorf zum Umschmelzen verwendet. Dazu wurde das Reaktionsröhrchen an der Unterseite gekappt und von den Seiten her etwas Material abgedreht. Anschließend wurde es auf einen Stift gesteckt und unter Kraft auf eine heiße Form gedrückt, die den Durchbruch bzw. die Aussparung (8), die Chipauflage (6) sowie den Kleberand (7) in das Röhrchen einprägte (siehe Abbildung 1).

Beispiel 2: Nachweis und Spezifität der Hybridisierung von Nukleinsäuren im Reaktionsgefäß (1) mittels Silberdetektion

a) Testsystem

Das humane *cyp2D6*-Gen kodiert für ein humanes Cytochrom-P450. Dieses Enzym spielt eine wichtige Rolle beim Metabolismus diverser Drogen und Wirkstoffe. Mutationen im *cyp2D6*-Gen können zu Überempfindlichkeit oder Unverträglichkeit gegenüber bestimmten Medikamenten führen. Mindestens 14 solcher Mutationen (Punktmutationen und Deletionen) sind für *cyp2D6* beschrieben. Für eine Auswahl dieser Mutationen (G1749C, dT1795, G1934A, G2064A) sollte getestet werden, ob in einer zu analysierenden Probe der jeweilige Wildtyp oder die Mutation vorhanden ist.

b) Herstellung der DNA-Bibliothek (102) und Assemblierung mit dem Reaktionsgefäß (1)

Auf einer epoxidierten Glasoberfläche (Objektträgergröße: 75 mm x 25 mm) (101) wurden mit einem MicroGrid II-Arrayer (Fa. BioRobotics, Cambridge, Großbritannien) 40 aminomodifizierte Oligonukleotide (Sonden) mit einer Länge von 21-25 Nukleotiden an definierten Stellen abgelegt und kovalent immobilisiert (Bibliotheks- bzw. Array-Elemente). Die Sonden gliederten sich in Paare von Bibliothekselementen, wobei die erste jeweils den Wildtyp und die zweite die Mutation darstellt.

Die Sequenzen der Oligonukleotide waren wie folgt (Sequenz in 5' - 3'-Richtung mit 5'-$NH_2$-Modifikation):

| Name | Sequenz |
| --- | --- |
| G1749C-WT | GCTTCTCCGTGTCCACCTTGC |
| G1749C | GCTTCTCCGTCTCCACCTTGC |
| G1749C-WT-2 | GCGCTTCTCCGTGTCCACCTT |
| G1749C-2 | GCGCTTCTCCGTCTCCACCTT |
| G1749C-WT-4 | AGGCGCTTCTCCGTGTCCACC |
| G1749C-4 | AGGCGCTTCTCCGTCTCCACC |
| G1749C-WT+2 | TTCTCCGTGTCCACCTTGCGC |
| G1749C+2 | TTCTCCGTCTCCACCTTGCGC |
| G1749C-WT+4 | CTCCGTGTCCACCTTGCGCAA |
| G1749C+4 | CTCCGTCTCCACCTTGCGCAA |
| dT1795-WT | GCTGGAGCAGTGGGTGACCGA |
| dT1795 | GCTGGAGCAGGGGTGACCGAG |
| dT1795-WT-2 | TCGCTGGAGCAGTGGGTGACC |
| dT1795-2 | TCGCTGGAGCAGGGGTGACCG |

| | |
|---|---|
| dT1795-WT-4 | AGTCGCTGGAGCAGTGGGTGA |
| dT1795-4 | AGTCGCTGGAGCAGGGGTGAC |
| dT1795-WT+2 | TGGAGCAGTGGGTGACCGAGG |
| dT1795+2 | TGGAGCAGGGGTGACCGAGGA |
| dT1795-WT+4 | GAGCAGTGGGTGACCGAGGAG |
| dT1795+4 | GAGCAGGGGTGACCGAGGAGG |
| G1934A-WT | CCCACCCCCAGGACGCCCCTT |
| G1934A | CCCACCCCCAAGACGCCCCTT |
| G1934A-WT-2 | CTCCCACCCCCAGGACGCCCC |
| G1934A-2 | CTCCCACCCCCAAGACGCCCC |
| G1934A-WT-4 | ATCTCCCACCCCCAGGACGCC |
| G1934A-4 | ATCTCCCACCCCCAAGACGCC |
| G1934A-WT+2 | CACCCCCAGGACGCCCCTTTC |
| G1934A+2 | CACCCCCAAGACGCCCCTTTC |
| G1934A-WT+4 | CCCCCAGGACGCCCCTTTCGC |
| G1934A+4 | CCCCCAAGACGCCCCTTTCGC |
| G2064A-WT | TAGCTCAGGAGGGACTGAAGGAGGA |
| G2064A | TAGCTCAGGAGGAACTGAAGGAGGA |
| G2064A-WT-2 | CCTAGCTCAGGAGGGACTGAAGGAG |
| G2064A-2 | CCTAGCTCAGGAGGAACTGAAGGAG |
| G2064A-WT-4 | GACCTAGCTCAGGAGGGACTGAAGG |
| G2064A-4 | GACCTAGCTCAGGAGGAACTGAAGG |
| G2064A-WT+2 | GCTCAGGAGGGACTGAAGGAGGAGT |
| G2064A+2 | GCTCAGGAGGAACTGAAGGAGGAGT |
| G2064A-WT+4 | TCAGGAGGGACTGAAGGAGGAGTCG |
| G2064A+4 | TCAGGAGGGACTGAAGGAGGAGTCG |

Eine einzelne komplette (rechteckige) DNA-Bibliothek (102) auf der Objektträger-Oberfläche (101) bestand aus insgesamt 12 x 10 = 120 abgelegten Bibliothekselementen und 7 Markierungen zur optischen Orientierung der Abbildung des Lesegerätes (1000). Jede der 40 Oligonukleotid-Sonden wurde dabei in dreifacher Wiederholung auf der DNA-Bibliothek (102) abgelegt (zur Anordnung der Bibliothekselemente siehe Abbildung 18). Die Bibliothekselemente hatten einen Abstand von 0,2 mm, die gesamte DNA-Bibliothek (102) bedeckte eine Fläche von 2,4 mm x 2,4 mm. Insgesamt konnten auf diese Weise pro Objektträger über 100 identische DNA-Bibliotheken (102) erzeugt werden.

Das Ablegen der Bibliothekselemente auf die Objektträger erfolgte aus einer jeweils 10 µM-Lösung der Oligonukleotide in 0,1 M Phosphatpuffer/2,2%-Natriumsulfat. Anschließend wurden die Bibliothekselemente durch 30-minütiges Backen bei 60 °C kovalent mit den Epoxid-Gruppen auf der Glasoberfläche verknüpft. Es folgte ein mehrteiliger Waschprozess der in folgender Reihenfolge:

- 5 min in 600 ml $H_2O$ bidest + 600 µl Triton x100

- 2 x 2 min in 600 ml $H_2O$ bidest + 60 µl HCL (konz.)
- 30 min in 100 mM KCl-Lösung
- 1 min in $H_2O$ bidest spülen
- mit Druckluft trocknen

Nach Beendigung der Waschschritte und Trocknen der Objektträger wurden diese in 3,15 mm x 3,15 mm große Glasstücke (nachfolgend DNA-Chip (100) genannt) zerschnitten. Auf jedem der Chips (100) befand sich genau eine DNA-Bibliothek (102) der Größe 2,4 mm x 2,4 mm.
Anschließend wurden die Chips (100) in der wie in Abbildung 1 dargestellten Weise in das Reaktionsgefäß (1) eingefügt und mit Polydimethlysiloxan, das in den Kleberand (7) gegeben wurde, verklebt.

c) Vorbereitung der Targets für die Hybridisierung gegen die DNA-Bibliothek

Als zu analysierende Probe (im Folgenden Target genannt) diente eine gegen das Exon 3/4 von *cyp2D6* gerichtete biotinmarkierte PCR einer Patienten-DNA (KDL24, durch Sequenzierung als Wildtyp für die betreffenden Mutationen genotypisiert). Der verwendete PCR-Ansatz sah wie folgt aus:

- Primer 1: cyp2D6_ 3/4-f, Sequenz 5'-CACGCGCACGTGCCCGTCCCA-3', Endkonzentration 200 nM
- Primer 2: cyp2D6_ 3/4r-5'Bio, Sequenz 5'-Bio-CTCTCGCTCCGCACCTCGCGCAGA-3', Endkonzentration 200 nM
- dNTPs, Endkonzentration 200 µM
- Advantage cDNA-Polymerase-Mix (50fach, Clontech, Palo Alto, USA), Endkonzentration 1fach
- Advantage cDNA PCR reaction buffer (10fach, Clontech, Palo Alto, USA), Endkonzentration 1fach
- Template-DNA (KDL24), 80 ng
- Wasser ad 50 µl

Anschließend wurde die PCR nach folgendem Programm durchgeführt:

1 Denaturierung (10 min, 95°C)
2 Denaturierung (30 s, 95°C)
3 Annealing (30 s, 65°C)
4 Elongation (80 s, 72°C)
5 29fache Wiederholung der Schritte 2-4
6 Elongation (7 min, 72°C)
7 Kühlen (4°C, bis zur weiteren Bearbeitung)

d) Hybridisierung und Konjugation der Sondenarrays im Reaktionsgefäß (1)

Für die Hybridisierungs-Reaktion im Reaktionsgefäß (1) wurden 4 µl des hergestellten PCR-Produktes mit 146 µl 6x SSPE-Puffer (52,59 g NaCl, 8,28 g $NaH_2PO_4$ x $H_2O$, 2,22 g EDTA x $2H_2O$ in 11 $H_2O$ bidest, auf pH 7,4 mit NaOH eingestellt)/0,1 % SDS vermischt und in das fertig assemblierte Reaktionsgefäß (1) gegeben. Nach einer Denaturierung der Hybridisierungslösung (5 min 95 °C) folgte eine Inkubation unter leichtem Schütteln für 60 min bei 65 °C. Anschließend wurde die Hybridisierungslösung aus dem Reaktionsgefäß (1) abgesaugt. Es schlossen sich zwei Waschschritte an: je 10 min in 2xSSC/0,2 % SDS (500 µl bei 30 °C) und 2xSSC (500 µl bei 20°C). Danach wurde die vorbereitete Konjugationslösung (Streptavidin-Gold, British Biocell International, EM.STP5, Endkonzentration 250pg/µl in 6xSSPE/0,1% SDS) auf den Chip (100) im Reaktionsgefäß (1) gegeben und das Reaktionsgefäß (1) 15 min bei 37 °C inkubiert. Nach dem Absaugen der Konjugat-Lösung wurde der Chip (100) im Reaktionsgefäß (1) je 10 min in 2x SSC/0,2 % SDS (500 µl bei 30 °C), 2x SSC (500 µl bei 20 °C) und 0,2x SSC (500 µl bei 20°C) unter Schütteln gewaschen.

e) Detektion

Zur Silberverstärkung wurde der eingebaute Chip (100) im Reaktionsgefäß (1) mit einer Silberentwicklungslösung (British Biocell International, SEKL15, Cardiff, Großbritannien) überschichtet und das Reaktionsgefäß (1) in das Silberlesegerät (1000) eingeführt. Das Lesegerät (1000) besteht aus einer Probenaufnahme mit Temperierung, der Auslesestrecke und einem Schwenkarm (1101) mit der Beleuchtung (siehe Abbildungen 13 und 14). Die Proben wurden in Transmission mittels eines diffus leuchtenden Arrays aus mehreren LED (1008) beleuchtetet. Ein zusätzlicher Diffusor ermöglichte die homogene Beleuchtung der Proben mit Abweichungen von weniger als 10 % vom Maximum.

Ausgelesen wurde die Probe in Transmission. Dazu wurden die Proben in die vorgesehene Aufnahme gesteckt. Die Lagerichtigkeit der Proben wurde gewährleistet durch eine spezielle Ausformung der Aufnahme (1102). In diese wurde das Reaktionsgefäß (1) mit geöffnetem Verschluss eingelegt. Der Deckel des Reaktionsgefäßes (1) lässt in dieser Position keine Verdrehungen der Affinitätsmatrix (100) zu. Nur bei Lagerichtigkeit ist das Einschwenken der Beleuchtung in die Messstellung möglich. In der Messstellung fixiert der Schwenkarm gleichzeitig das Reaktionsgefäß (1) und damit die Affinitätsmatrix.

Für das Auswerteverfahren hat es sich als vorteilhaft erwiesen, dass zum Auslesen eine verzeichnungsfreie Ausleseoptik (1004) verwendet wird. Durch die Verwendung einer Optik mit großer Tiefenschärfe erübrigte sich eine Justierung der Proben zur Kamera. Das Scharfstellen war überflüssig. Je nach Dichte der Substanzen pro Flächeneinheit auf der Affinitätsmatrix ist die verwendete CCD-Kamera (1005) entweder im CCIR-Format oder noch höher auflösend. Während der Messung wurden die Proben durch eine Widerstandsheizung und ein Peltierelement (1200) temperaturstabil gehalten.

Die Silberentwicklungslösung wurde durch Mischen von gleichen Teilen Initiator- und Enhancer-Lösung hergestellt. Während der 20-minütigen Inkubation bei 25 °C (± 0,1°C) wurde der zeitliche Verlauf der Silberverstärkung durch ein Fotoserie (alle 10 s ein Bild) dokumentiert (siehe Abbildung 19)

Die optische Dichte der Bibliothekselemente aller Bilder der Zeitreihe wurde anschließend mit der Bildauswertungs-Software IconoClust® der Firma Clondiag® ermittelt. In Abbildung 20 sind die Zeitreihen für das Element G1934A+4 (Mutation) und G2064A-WT (Wildtyp) dargestellt.

Entsprechend der in der Beschreibung angegebenen Vorgehensweise zur Auswertung wurden die Messwerte $a_{G1934A+4}$ und $a_{G2064A\text{-}WT}$ berechnet. Sie betragen $a_{G1934A+4} = 4.83 * 10^{-7}$ sek$^{-2}$ und $a_{G2064A\text{-}WT} = 2.566 * 10^{-6}$ sek$^{-2}$.

Beschreibungen der Abbildungen

Abbildung 1:

In den Boden eines Reaktionsgefäßes (2) ist ein Durchbruch bzw. eine Aussparung (8) eingearbeitet mit einer Auflagefläche (6), auf die die Affinitätsmatrix (100) von außen aufgelegt werden kann.
Die Affinitätsmatrix (100) wird verklebt, indem der Klebstoff in einen dafür vorgesehenen Klebrand (7) gegeben wird.

Abbildung 2:

In den Boden eines Reaktionsgefäßes (2) ist ein Durchbruch (10) mit einer Auflagefläche (11) eingearbeitet, auf die die Affinitätsmatrix (100) von innen aufgelegt werden kann.
Die Affinitätsmatrix (100) wird verklebt, indem der Klebstoff in einen dafür vorgesehenen Klebrand (9) gegeben wird.

Abbildung 3:

Der Boden eines Reaktionsgefäßes (2) ist mit einem Klemmverbindung (201) versehen. Auf diese Klemmverbindung (201) kann ein Chipträger (200) flüssigkeitsdicht gedrückt werden. Der Chipträger weist einen Durchbruch (10) auf, in den auf einer dafür vorgesehenen Auflagefläche die Affinitätsmatrix (100) gelegt und dann verklebt werden kann.

Abbildung 4:

In der in Abbildung 4 gezeigten Ausführungsform wird die Affinitätsmatrix (100) zwischen dem Reaktionsgefäß (2) und einer Klemmhülse (300) flüssigkeitsdicht verklemmt, so dass eine Verklebung nicht unbedingt notwendig ist.

Abbildung 5:

Abbildung 5 zeigt eine prinzipielle Anordnung zum Auslesen eines Arrays im erfindungsgemäßen Reaktionsgefäß. Licht einer inkohärenten Lichtquelle (1001) leuchtet mittels einer Beleuchtungsoptik (1002) die oberflächengebundene DNA-Bibliothek auf einem Bibliothekenchip (100), der sich in einem Tube befindet, homogen aus. Mittels einer Ausleseoptik (1004) wird das Signal von einer CCD-Kamera (1005) aufgenommen.

Abbildung 6:

Abbildung 6 zeigt die gleiche Anordnung wie Abbildung 5, mit der Variante, durch Einbringen von optischen Filtern (1006) in den Beleuchtungsstrahlengang den spektralen Bereich der Beleuchtung einzugrenzen. Die Möglichkeit, diesen Filter mittels Filterwechsler (1007) schnell zu wechseln, hat für die Auswertung den Vorteil, mögliche Fehlinformationen, die z.B. durch Verunreinigungen auftreten, eindeutig zu erkennen und zu eliminieren.

Abbildung 7:

In dieser Ausführungsform wird die klassische Beleuchtung durch ein Beleuchtungsarray (1008) ersetzt. Vorzugsweise diffus streuende LED, matrixförmig angeordnet, ermöglichen auf kurze Entfernungen zur Probe eine homogene Beleuchtung.

Abbildung 8:

Abbildung 8 zeigt eine kompakte Form der Anordnung aus Abbildung 1, bei der der CCD-Sensor (1005) der Kamera unmittelbar mit der Affinitätsmatrix (100) kontaktiert ist. Dieser Kontakt kann auch durch den Einsatz einer Faserplatte (1012) oder bei noch größeren Abständen durch ein Bildleitkabel hergestellt werden, wenn ein unmittelbarer Kontakt nicht möglich ist.

Abbildung 9:

Abbildung 9 zeigt eine Anordnung zum Auslesen der Proben im Auflicht. Dabei kommt ein halbdurchlässiger Spiegel (1009) zum Einsatz.

Abbildung 10:

Da die Silberproben keine sehr guten Reflexionseigenschaften besitzen, ist eine wie in dieser Abbildung gezeigte Anordnung vorteilhaft. Hier wird der Nachteil der schlechten Reflexion durch Transmissionseffekte ergänzt, indem das Beleuchtungslicht über eine Spiegelschicht (1010), entweder als eigenständiger Spiegel oder als auf der Rückseite des Probenträgers aufgetragene Schicht, reflektiert wird.

Abbildung 11 :

Abbildung 11 zeigt eine Anordnung in einem transparenten Probenraum, vorzugsweise in einer Küvette (1011), die durch die optisch planen Außenflächen eine gute Kopplung des Empfängers mittels Faserplatte (1012) an den Probenraum ermöglichen. Bei dem Einsatz von diffusen Flächenstrahlern (1013), die z.B. auf Elektrolumineszenz- oder Kaltkathodenfluoreszenz-Verfahren beruhen, lassen sich sehr kompakte Sensoren realisieren, wie sie in Abbildung 8 dargestellt sind.

Abbildung 12:

Abbildung 12 stellt eine sehr kompakte Anordnung des in Abbildung 11 dargestellten Lesegerätes (1000) dar.

Abbildung 13:

Schnittzeichnung eines Lesegerätes, das entsprechend dem in Abbildung 7 dargestellten Prinzip arbeitet, mit dem Unterschied, dass zwischen der Affinitätsmatrix (100) und der abbildenden Optik (1004) ein Umlenkspiegel eingebaut wurde, um zu kompakten Geräteausmaßen zu kommen. Die Lichtquelle (1008) besteht aus Leuchtdioden, die in eine Schwenkarm (1101) eingebaut sind, der zum Einsetzen des Reaktionsgefäßes (1) in den Reader zur Seite geschwenkt werden kann. Danach wird er über das Reaktionsgefäß (1) geschwenkt, um die Affinitätsmatrix (100) zu beleuchten.

Abbildung 14:

Abbildung 14 entspricht der Abbildung 13, nur als Aufsicht (von der Bedienerseite). Auf der Oberseite befindet sich eine Justierverkiefung (1102), in die der Deckel des Reaktionsgefäßes (1) gedrückt wird. Dadurch wird eine Verdrehung der Affinitätsmatrix vermieden.

Abbildung 15:

Es wird ein Lesegerät (1000) dargestellt, dass sich von dem in der Abbildung 13 dadurch unterscheidet, dass

der Schwenkarm (1101) durch einen Schieber (1300) ersetzt wird. Der Schieber (1300) wird über ein Linearlager (1310) über das Reaktionsgefäß (1) geschoben. Dabei drückt ein Schlitten (1320) das Reaktionsgefäß (1) in den Reaktionsgefäß-Halter (1100).

Abbildung 16:

Der Schlitten (1320) des Schiebers (1300) wird in der Aufsicht gezeigt. Der Schlitten (1320) wird durch vier Druckfedern (1321) auf das Reaktionsgefäß (1) gedrückt. Ein Justierspalt (1322) umgreift den Deckel (3) des Reaktionsgefäßes (1) derart, dass das Reaktionsgefäß (1) in seine richtige Position gedreht und fixiert wird.

Abbildung 17:

In der Darstellung wird im Querschnitt die Lage des Schiebers (1300) zum Reaktionsgefäß (1) bei geöffnetem Reader dargestellt. Der Schlitten (1300) bewegt sich entlang des Pfeils und verschließt dadurch den Reader. Der Schieber besteht aus den Druckfedern (1321), die den Schlitten (1320) herunterdrücken, der eine Detektionsöffnung (1323) aufweist, durch die das Licht der Lichtquelle (1001) strahlen kann.

Abbildung 18:

Layout des Arrays (102) der Größe 2,4 mm x 2,4 mm; Angegeben sind jeweils die Namen der 3fach redundanten Sonden. "M" = Markierung.

Abbildung 19:

Ausgewähltes Zeitreihenbild (nach 15-minütiger Silber-Entwicklungszeit) der Hybridisierung einer biotinmarkierten Wildtyp-PCR (Exon 3/4, KDL24) im erfindungsgemäßen Reaktionsgefäß. Das Layout des Chips ist in Abbildung 1 dargestellt.

Abbildung 20:

Darstellung des zeitlichen Verlaufs der Schwärzung zweier Spots durch Silberabscheidung. Ein Spot weißt die perfekte Komplementärsequenz der Target-DNA auf (Wildtyp *G2064A-WT*), der andere unterscheidet sich um eine Base (Mutation *G1934A+4*). Die zugehörigen Regressionsfunktionen sind ebenfalls dargestellt.

Abbildung 21:

Foto zweier Standard - Reaktionsgefäße aus Polypropylen mit 1,5 ml Füllvolumen.

Legende

| | |
|---|---|
| 1 | Reaktionsgefäß |
| 2 | Reaktionsgefäß mit einem Durchbruch (Aussparung), der als Einfassung zur Aufnahme von Affinitätsmatrizen ausgeformt ist. |
| 3 | Deckel |
| 4 | Lasche |
| 5 | Dichtung |
| 6 | Chipauflage außen |
| 7 | Kleberand außen |
| 8 | Flüssigkeitsdurchbruch |
| 9 | Kleberand innen |
| 10 | Sichtdurchbruch |
| 11 | Chipauflage innen |

| | |
|---|---|
| 100 | Bibliothekenchip, Affinitätsmatrix bzw. DNA-Chip |
| 101 | Substrat |
| 102 | oberflächengebundene Substanzbibliothek |

200 Chipträger
201 Klemmverbindung

300 Klemmhülse
301 Dichtflächen
1000 Lesegerät
1001 Lichtquelle
1002 Beleuchtungsoptik
1004 Ausleseoptik
1005 Sensor, vorzugsweise eine CCD-Kamera
1006 Optischer Transmissionsfilter
1007 Filterwechsler
1008 Beleuchtungsarray, vorzugsweise eine Anordnung von LED
1009 Halbdurchlässiger Spiegel
1010 Spiegel bzw. Verspiegelung des Trägers der DNA-Bibliothek
1011 Küvette
1012 Faserplatte
1013 Diffuser Flächenstrahler
1014 Umlenkspiegel

1100 Reaktionsgefäß-Halter
1101 Schwenkarm
1102 Justiervertiefung

1200 Peltierelement

1300 Schieber
1310 Linearlager
1320 Schlitten
1321 Druckfeder
1322 Justierspalt
1323 Detektionsöffnung
1324 Andruckfläche

**Patentansprüche**

1. Reaktionsgefäß zum Nachweis der spezifischen Wechselwirkung zwischen molekularen Target- und Sondenmolekülen,
wobei es ein Tube ist, das eine typische Form und Größe aufweist und das ein Füllvolumen von 100µl bis 2,5 ml aufweist, wobei auf einer seiner Grundflächen ein Trägerelement (100) mit darauf auf vorbestimmten Bereichen immobilisierten Sondenmolekülen angeordnet ist.

2. Reaktionsgefäß nach Anspruch 1,
**dadurch gekennzeichnet, dass** die Grundfläche des Laborreaktionsgefäßes eine Aussparung zur Aufnahme des Trägerelements aufweist.

3. Reaktionsgefäß nach Anspruch 1 oder 2,
**dadurch gekennzeichnet, dass** das Trägerelement im Bereich einer Detektionsfläche optisch durchlässig ist.

4. Reaktionsgefäß nach einem der vorangehenden Ansprüche,
**dadurch gekennzeichnet, dass** die der Detektionsfläche gegenüberliegende Grundfläche optisch durchlässig ist.

5. Reaktionsgefäß nach einem der vorangehenden Ansprüche,
**dadurch gekennzeichnet, dass** das Material für das Trägerelement Glas und/oder Silizium ist und vorzugsweise ausgewählt ist aus der Gruppe bestehend aus Borofloat 33, Quarzglas, einkristallinem $CaF_2$ und einkristallinem Silizium.

6. Reaktionsgefäß nach einem der vorangehenden Ansprüche,
**dadurch gekennzeichnet, dass** das Material für das Gefäß ausgewählt ist aus der Gruppe bestehend aus Glas, Glaskeramik, kunststoffbeschichtetem Glas, Kunststoffen wie Polypropylen, Polyethylen, Polystyrol, Polycarbonat, PVC, Polymethylmethacrylat, Silikonkunststoff, Kautschuk, Polytetrafluorethylen und/oder Nylon, und Metallen wie nicht rostenden Stählen, Platin und/oder Aluminium.

7. Reaktionsgefäß nach einem der vorangehenden Ansprüche,
**dadurch gekennzeichnet, dass** es ein Füllvolumen von 1,5 ml aufweist.

8. Reaktionsgefäß nach einem der vorangehenden Ansprüche,
**dadurch gekennzeichnet, dass** es sich bei immobilisierten Sondenmolekülen um eine Substanzbibliothek ausgewählt aus der Gruppe bestehend aus Proteinbibliotheken, Peptidbibliotheken und Nukleinsäurebibliotheken handelt.

9. Reaktionsgefäß nach Anspruch 8,
**dadurch gekennzeichnet, dass** die Proteinbibliothek eine Antikörperbibliothek, eine Rezeptorproteinbibliothek und/oder eine Membranproteinbibliothek ist.

10. Reaktionsgefäß nach Anspruch 8,
**dadurch gekennzeichnet, dass** die Peptidbibliothek eine Bibliothek von Rezeptorliganden, eine Bibliothek pharmakologisch aktiver Peptide und/oder eine Bibliothek von Peptidhormonen ist.

11. Reaktionsgefäß nach Anspruch 8,
**dadurch gekennzeichnet, dass** die Nukleinsäurebibliothek eine DNA-Molekülbibliothek und/oder eine RNA-Molekülbibliothek ist.

12. Vorrichtung zum Nachweis der spezifischen Wechselwirkung zwischen molekularen Target- und Sondenmolekülen, umfassend:

a) mindestens ein Reaktionsgefäß nach einem der Ansprüche 1 bis 11; und
b) eine Detektionsvorrichtung zum Nachweis der spezifischen Wechselwirkung.

13. Vorrichtung nach Anspruch 12,
**dadurch gekennzeichnet, dass** die Detektionsvorrichtung eine Kamera und ggf. eine Ausleseoptik umfasst.

14. Vorrichtung nach Anspruch 13,
**dadurch gekennzeichnet, dass** die Kamera eine CCD- oder CMOS-Kamera ist.

15. Vorrichtung nach einem der Ansprüche 12 bis 14,
**dadurch gekennzeichnet, dass** sie zusätzlich eine Lichtquelle umfasst.

16. Vorrichtung nach Anspruch 15,
**dadurch gekennzeichnet, dass** die Lichtquelle eine homogene Beleuchtung des Trägers gewährleistet

17. Vorrichtung nach Anspruch 15 oder 16,
**dadurch gekennzeichnet, dass** die Lichtquelle ein Beleuchtungsarray ist, welches mehrere diffus strahlende Lichtquellen umfasst, die durch Überlagerung die homogene Beleuchtung des Trägers gewährleisten.

18. Vorrichtung nach einem der Ansprüche 15 bis 17,
**dadurch gekennzeichnet, dass** die Lichtquelle ausgewählt ist aus der Gruppe, bestehend aus Lasern, Lichtemittierenden Dioden (LED), Flächenstrahlern und Hochdrucklampen.

19. Vorrichtung nach einem der Ansprüche 12 bis 18,
**dadurch gekennzeichnet, dass** der Deckel des Reaktionsgefäßes strukturiert ist, um eine homogene Beleuchtung zu gewährleisten.

20. Vorrichtung nach einem der Ansprüche 12 bis 19,
**dadurch gekennzeichnet, dass** sie zusätzlich optische Filter und ggf. einen Filterwechsler umfasst.

**21.** Vorrichtung nach einem der Ansprüche 12 bis 20,
**dadurch gekennzeichnet, dass** sie zusätzlich einen halbdurchlässigen Spiegel zwischen Lichtquelle und Träger umfasst.

**22.** Vorrichtung nach einem der Ansprüche 12 bis 21,
**dadurch gekennzeichnet, dass** das Reaktionsgefäß in unmittelbarem Kontakt mit der Detektionsvorrichtung ist.

**23.** Vorrichtung nach einem der Ansprüche 12 bis 22,
**dadurch gekennzeichnet, dass** mehrere Reaktionsgefäße so angeordnet sind, dass eine aufeinanderfolgende Detektion gewährleistet ist.

**24.** Vorrichtung nach einem der Ansprüche 12 bis 23,
**dadurch gekennzeichnet, dass** sie zusätzlich eine Temperatursteuerungseinheit umfasst.

**25.** Vorrichtung nach einem der Ansprüche 12 bis 24,
**dadurch gekennzeichnet, dass** sie zusätzlich einen Computer umfasst, der programmiert ist, um:

- die von der Detektionsvorrichtung aufgenommenen Signalintensitäten zu sammeln; und
- ggf. die Umwandlung der virtuellen Signalintensitäten in ein analoges Bild zu gewährleisten.

**26.** Verfahren zum Nachweis der spezifischen Wechselwirkung zwischen molekularen Target- und Sondenmolekülen, umfassend die folgenden Schritte:

a) Bereitstellung eines Reaktionsgefäßes nach einem der Ansprüche 1 bis 11 bzw. einer Vorrichtung nach einem der Ansprüche 12 bis 25;
b) Wechselwirkung der Targets mit den auf vorbestimmten Bereichen bzw. Array-Elementen angeordneten Sonden; und
c) Detektion der Wechselwirkung.

**27.** Verfahren nach Anspruch 26,
**dadurch gekennzeichnet, dass** zur Detektion der Wechselwirkung eine Reaktion durchgeführt wird, die zu einem Niederschlag an den Array-Elementen führt, an denen eine Wechselwirkung erfolgt ist.

**28.** Verfahren nach Anspruch 27,
**dadurch gekennzeichnet, dass** in Schritt c) der zeitliche Verlauf der Niederschlagsbildung an den Array-Elementen in Form von Signalintensitäten detektiert wird.

**29.** Verfahren nach Anspruch 27 oder 28,
**dadurch gekennzeichnet, dass** die Reaktion, die zur Bildung eines Niederschlags an den Array-Elementen führt, die Umwandlung eines löslichen Substrats in einen metallischen Niederschlag ist.

**30.** Verfahren nach Anspruch 29,
**dadurch gekennzeichnet, dass** die zur Bildung eines Niederschlags an den Array-Elementen führende Reaktion die chemische Reduktion einer Silberverbindung, vorzugsweise Silbernitrat, Silberlactat, Silberacetat oder Silbertartrat, zu elementarem Silber ist.

**31.** Verfahren nach Anspruch 30,
**dadurch gekennzeichnet, dass** das Reduktionsmittel ausgewählt wird aus der Gruppe bestehend aus Formaldehyd und Hydrochinon.

**32.** Verfahren nach einem der Ansprüche 29 bis 31,
**dadurch gekennzeichnet, dass** die Umwandlung eines löslichen Substrats in einen metallischen Niederschlag in Gegenwart von mit den Targets gekoppelten Metallclustern bzw. kolloidalen Metallpartikeln erfolgt.

**33.** Verfahren nach Anspruch 32,
**dadurch gekennzeichnet, dass** die Umwandlung eines löslichen Substrats in einen metallischen Niederschlag in Gegenwart von Goldclustern oder kolloidalen Goldpartikeln erfolgt.

34. Verfahren nach einem der Ansprüche 29 bis 33, **dadurch gekennzeichnet, dass** die Kopplung der Metallcluster bzw. kolloidalen Metallpartikel an die Targets direkt oder über an die Targets gekoppelte Ankermoleküle erfolgt.

35. Verfahren nach einem der Ansprüche 26 bis 34, **dadurch gekennzeichnet, dass** die Wechselwirkung zwischen dem Target und der Sonde eine Hybridisierung zwischen zwei Nukleotidsequenzen ist.

36. Verfahren nach einem der Ansprüche 26 bis 35, **dadurch gekennzeichnet, dass** die Detektion der Gegenwart eines Niederschlags auf einem Array-Element durch Reflexion, Absorption oder Diffusion eines Lichtstrahls, vorzugsweise eines Laserstrahls oder einer Leuchtdiode, durch den Niederschlag erfolgt.

37. Verwendung einer Vorrichtung nach einem der Ansprüche 1 bis 11 bzw. einer Vorrichtung nach einem der Ansprüche 12 bis 25 zur Durchführung von Mikro-Array basierten Tests.

## Claims

1. A reaction vessel for detecting the specific interaction between molecular target and probe molecules, wherein it is a tube which has a typical shape and size and which has a filling volume in the range of from 100 µl to 2.5 ml, wherein a supporting element with probe molecules immobilised thereon on predetermined regions is arranged on one of its base surfaces.

2. The reaction vessel according to claim 1, **characterised in that** the base surface of the laboratory reaction vessel has a recess for receiving the supporting element.

3. The reaction vessel according to claim 1 or 2, **characterised in that** the supporting element is optically transparent in the area of a detection surface.

4. The reaction vessel according to any one of the preceding claims, **characterised in that** the base surface opposite to the detection surface is optically transparent.

5. The reaction vessel according to any one of the preceding claims, **characterised in that** the material for the supporting element is glass and/or silicon and is preferably selected from the group consisting of Borofloat 33, quartz glass, mono-crystalline $CaF_2$ and mono-crystalline silicon.

6. The reaction vessel according to any one of the preceding claims, **characterised in that** the material for the vessel is selected from the group consisting of glass, glass-ceramics, plastic-coated glass, plastics such as polypropylene, polyethylene, polystyrene, polycarbonate, PVC, polymethylmethacrylate, silicone plastic, rubber, polytetrafluoroethylene and/or nylon, and metals such as stainless steels, platinum and/or aluminium.

7. The reaction vessel according to any one of the preceding claims, **characterised in that** it has a filling volume of 1.5 ml.

8. The reaction vessel according to any one of the preceding claims, **characterised in that** the immobilised probe molecules are a substance library selected from the group consisting of protein libraries, peptide libraries and nucleic acid libraries.

9. The reaction vessel according to claim 8, **characterised in that** the protein library is an antibody library, a receptor protein library and/or a membrane protein library.

10. The reaction vessel according to claim 8, **characterised in that** the peptide library is a library of receptor ligands, a library of pharmacologically active peptides and/or a library of peptide hormones.

11. The reaction vessel according to claim 8, **characterised in that** the nucleic acid library is a DNA molecule library and/or an RNA molecule library.

**12.** A device for detecting the specific interaction between molecular target and probe molecules, comprising:

a) at least one reaction vessel according to any one of claims 1 to 11; and
b) a detection device for detecting the specific interaction.

**13.** The device according to 12, **characterised in that** the detection device comprises a camera and, optionally, read-out optics.

**14.** The device according to claim 13, **characterised in that** the camera is a CCD or CMOS camera.

**15.** The device according to any one of claims 12 to 14, **characterised in that** it additionally comprises a light source.

**16.** The device according to claim 15, **characterised in that** the light source ensures homogeneous illumination of the support.

**17.** The device according to claim 15 or claim 16, **characterised in that** the light source is an illuminating array which comprises a plurality of diffusely emitting light sources which ensure homogeneous illumination of the support by spectral interference.

**18.** The device according to any one of claims 15 to 17, **characterised in that** the light source is selected from the group consisting of lasers, light-emitting diodes (LED), surface emitters and high-pressure lamps.

**19.** The device according to any one of claims 12 to 18, **characterised in that** the lid of the reaction vessel is structured to ensure a homogeneous illumination.

**20.** The device according to any one of claims 12 to 19, **characterised in that** it additionally comprises optical filters, and, optionally, filter changers.

**21.** The device according to any one of claims 12 to 20, **characterised in that** it additionally comprises a semitransparent mirror positioned between light source and support.

**22.** The device according to any one of claims 12 to 21, **characterised in that** the reaction vessel is in direct contact with the detection device.

**23.** The device according to any one of claims 12 to 22, **characterised in that** a plurality of reaction vessels are arranged such that successive detection is ensured.

**24.** The device according to any one of claims 12 to 23, **characterised in that** it additionally comprises a temperature control unit.

**25.** The device according to any one of claims 12 to 24, **characterised in that** it additionally comprises a computer which is programmed for

- collecting the signal intensities recorded by the detection device; and
- optionally, ensuring conversion of the virtual signal intensities into an analogue image.

**26.** A method for detecting the specific interaction between molecular target and probe molecules comprising the following steps:

a) providing a reaction vessel according to any one of claims 1 to 11 or a device according to any one of claims 12 to 25;
b) interacting of the target with the probes arranged on predetermined regions or array elements; and
c) detecting the interaction.

**27.** The method according to claim 26, **characterised in that** for detecting the interaction a reaction is carried out which results in a precipitate on the array elements on which an interaction occurs.

**28.** The method according to claim 27, **characterised in that** in step c) the time profile of the formation of the precipitate

is detected on the array elements in the form of signal intensities.

**29.** The method according to claim 27 or claim 28, **characterised in that** the reaction which results in the formation of a precipitate on the array elements is the conversion of a soluble substrate into a metallic precipitate.

**30.** The method according to claim 29, **characterised in that** the reaction which results in the formation of a precipitate on the array elements is the chemical reduction of a silver compound, preferably silver nitrate, silver lactate, silver acetate or silver tartrate to form elemental silver.

**31.** The method according to claim 30, **characterised in that** the reducing agent is selected from the group consisting of formaldehyde and hydroquinone.

**32.** The method according to any one of claims 29 to 31, **characterised in that** the conversion of a soluble substrate into a metallic precipitate takes place in the presence of metal clusters or colloidal metal particles coupled to the targets.

**33.** The method according to claim 32, **characterised in that** the conversion of a soluble substrate into a metallic precipitate takes place in the presence of gold clusters or colloidal gold particles.

**34.** The method according to any one of claims 29 to 33, **characterised in that** the coupling of the metal clusters or colloidal metal particles to the targets takes place directly or via anchor molecules coupled to the targets.

**35.** The method according to any one of claims 26 to 34, **characterised in that** the interaction between the target and the probe is a hybridisation between two nucleotide sequences.

**36.** The method according to any one of claims 26 to 35, **characterised in that** the detection of the presence of a precipitate on an array element is effected by reflection, absorption or diffusion of a light beam, preferably a laser beam or a light-emitting diode, by the precipitate.

**37.** Use of a device according to any one of claims 1 to 11 or a device according to any one of claims 12 to 25 for carrying out micro-array-based tests.

## Revendications

**1.** Récipient réacteur pour prouver l'interaction spécifique entre des molécules moléculaires cible et de sonde, dans lequel il s'agit d'un tube présentant une forme et une taille typiques et présentant une capacité de remplissage de 100 µl à 2,5 ml, dans lequel un élément support (100) est disposé sur l'une de ses surfaces de base, comportant des molécules de sonde immobilisées dans des zones prédéfinies.

**2.** Récipient réacteur selon la revendication 1,
**caractérisé en ce que** la surface de base du récipient réacteur de laboratoire présente un évidement pour recevoir l'élément support.

**3.** Récipient réacteur selon la revendication 1 ou 2,
**caractérisé en ce que** l'élément support est optiquement perméable dans la zone d'une surface de détection.

**4.** Récipient réacteur selon l'une quelconque des revendications précédentes,
**caractérisé en ce que** la surface de base figurant en face de la surface de détection est optiquement transparente.

**5.** Récipient réacteur selon l'une quelconque des revendications précédentes,
**caractérisé en ce que** le matériau de l'élément support est du verre et/ou du silicium et, de préférence, sélectionné dans le groupe se composant de Borofloat 33, de verre de quartz, de $CaF_2$ monocristallin et de silicium monocristallin.

**6.** Récipient réacteur selon l'une quelconque des revendications précédentes,
**caractérisé en ce que** le matériau du récipient est sélectionné dans le groupe se composant du verre, de la céramique de verre, de verre recouvert de matière plastique, de matières plastiques tels que le polypropylène, le

polyéthylène, le polystyrol, le polycarbonate, le PVC, le polyméthylméthacrylate, de matières plastiques à base de silicone, le caoutchouc, le polytétrafluoréthylène et/ou le Nylon et des métaux tels que les aciers inoxydables, le platine et/ou l'aluminium.

**7.** Récipient réacteur selon l'une quelconque des revendications précédentes,
**caractérisé en ce qu'**il présente un volume de remplissage de 1,5 ml.

**8.** Récipient réacteur selon l'une quelconque des revendications précédentes,
**caractérisé en ce que** dans le cas des molécules de sonde immobilisées, il s'agit d'une bibliothèque de substances sélectionnée dans le groupe se composant de bibliothèques de protéines, de bibliothèques de peptides et de bibliothèques d'acides nucléiques.

**9.** Récipient réacteur selon la revendication 8,
**caractérisé en ce que** la bibliothèque de protéines est une bibliothèque d'anticorps, une bibliothèque de protéines de récepteur et/ou une bibliothèque de protéines de membrane.

**10.** Récipient réacteur selon la revendication 8,
**caractérisé en ce que** la bibliothèque de peptides est une bibliothèque de ligands de récepteur, une bibliothèque de peptides pharmacologiquement actifs et/ou une bibliothèque d'hormones de peptide.

**11.** Récipient réacteur selon la revendication 8,
**caractérisé en ce que** la bibliothèque d'acides nucléiques est une bibliothèque de molécules d'ADN et/ou une bibliothèque de molécules d'ARN.

**12.** Dispositif pour prouver l'interaction spécifique entre des molécules moléculaires cible et de sonde, comprenant :

a) au moins un récipient réacteur selon l'une quelconque de revendications 1 à 11 ; et
b) un dispositif de détection pour prouver l'interaction spécifique.

**13.** Dispositif selon la revendication 12,
**caractérisé en ce que** le dispositif de détection comprend une caméra et, le cas échéant, une optique de filtrage.

**14.** Dispositif selon la revendication 13,
**caractérisé en ce que** la caméra est une caméra CCD ou CMOS.

**15.** Dispositif selon l'une quelconque des revendications 12 à 14,
**caractérisé en ce qu'**il comprend en outre une source lumineuse.

**16.** Dispositif selon la revendication 15,
**caractérisé en ce que** la source lumineuse assure une illumination homogène du support.

**17.** Dispositif selon la revendication 15 ou 16,
**caractérisé en ce que** la source lumineuse est un réseau d'illumination qui comprend plusieurs sources lumineuses de rayonnement diffus qui par superposition assurent l'illumination homogène du support.

**18.** Dispositif selon l'une quelconque des revendications 15 à 17,
**caractérisé en ce que** la source lumineuse est sélectionnée dans le groupe se composant de lasers, de diodes électroluminescentes (DEL), de projecteurs plats et de lampes haute pression.

**19.** Dispositif selon l'une quelconque des revendications 12 à 18,
**caractérisé en ce que** le couvercle du récipient réacteur est structuré pour assurer une illumination homogène.

**20.** Dispositif selon l'une quelconque des revendications 12 à 19,
**caractérisé en ce qu'**il comprend en outre des filtres optiques et, le cas échéant, un changeur de filtre.

**21.** Dispositif selon l'une quelconque des revendications 12 à 20,
**caractérisé en ce qu'**il comprend en outre un miroir semi-transparent entre source lumineuse et support.

**22.** Dispositif selon l'une quelconque des revendications 12 à 21, **caractérisé en ce que** le récipient réacteur est en contact immédiat avec le dispositif de détection.

**23.** Dispositif selon l'une quelconque des revendications 12 à 22, **caractérisé en ce que** plusieurs récipients réacteur sont disposés de manière telle qu'une détection successive soit assurée.

**24.** Dispositif selon l'une quelconque des revendications 12 à 23, **caractérisé en ce qu'**il comprend en outre une unité de commande de température.

**25.** Dispositif selon l'une quelconque des revendications 12 à 24, **caractérisé en ce qu'**il comprend en outre un ordinateur, qui est programmé pour :

- collecter les intensités du signal enregistrées par le dispositif de détection ; et
- le cas échéant, assurer la conversion des intensités virtuelles du signal en une image analogique.

**26.** Procédé pour prouver l'interaction spécifique entre des molécules moléculaires cible et de sonde, comprenant les étapes suivantes :

a) fourniture d'un récipient réacteur selon l'une quelconque des revendications 1 à 11 ou d'un dispositif selon l'une quelconque des revendications 12 à 25 ;
b) interaction des cibles avec les sondes disposées dans des zones prédéterminées ou des éléments en réseau ; et
c) détection de l'interaction.

**27.** Procédé selon la revendication 26, **caractérisé en ce qu'**une réaction est réalisée pour détecter l'interaction qui conduit à une précipitation aux éléments de réseau auxquels une interaction s'est produite.

**28.** Procédé selon la revendication 27, **caractérisé en ce que** dans l'étape c) le déroulement temporel de la formation d'un précipité aux éléments de réseau est détecté sous la forme d'intensités de signal.

**29.** Procédé selon la revendication 27 ou 28, **caractérisé en ce que** la réaction qui conduit à la formation d'un précipité aux éléments de réseau est la transformation d'un substrat soluble en un précipité métallique.

**30.** Procédé selon la revendication 29, **caractérisé en ce que** la réaction conduisant à la formation d'un précipité aux éléments de réseau est la réduction chimique d'un composé d'argent, de préférence de nitrate d'argent, de lactate d'argent, d'acétate d'argent ou de tartrate d'argent, en argent élémentaire.

**31.** Procédé selon la revendication 30, **caractérisé en ce que** le réducteur est sélectionné dans le groupe se composant de formaldéhyde et d'hydroquinone.

**32.** Procédé selon l'une quelconque des revendications 29 à 31, **caractérisé en ce que** la transformation d'un substrat soluble en un précipité métallique s'effectue en présence de clusters métalliques ou de particules de métal colloïdales couplés aux cibles.

**33.** Procédé selon la revendication 32, **caractérisé en ce que** la transformation d'un substrat soluble en un précipité métallique s'effectue en présence de clusters d'or ou de particules d'or colloïdales.

**34.** Procédé selon l'une quelconque des revendications 29 à 33, **caractérisé en ce que** le couplage des clusters métalliques ou des particules de métal colloïdales avec les cibles s'effectue directement ou par des molécules d'ancrage couplées aux cibles.

**35.** Procédé selon l'une quelconque des revendications 26 à 34,
**caractérisé en ce que** l'interaction entre la cible et la sonde est une hybridation entre deux séquences de nucléotides.

**36.** Procédé selon l'une quelconque des revendications 26 à 35,
**caractérisé en ce que** la détection de la présence d'un précipité sur un élément de réseau s'effectue par réflexion, absorption ou diffusion d'un rayon lumineux, de préférence d'un rayon laser ou d'une diode lumineuse, par le précipité.

**37.** Utilisation d'un dispositif selon l'une quelconque des revendications 1 à 11 ou
d'un dispositif selon l'une quelconque des revendications 12 à 25 pour réaliser des tests reposant sur un micro-réseau.

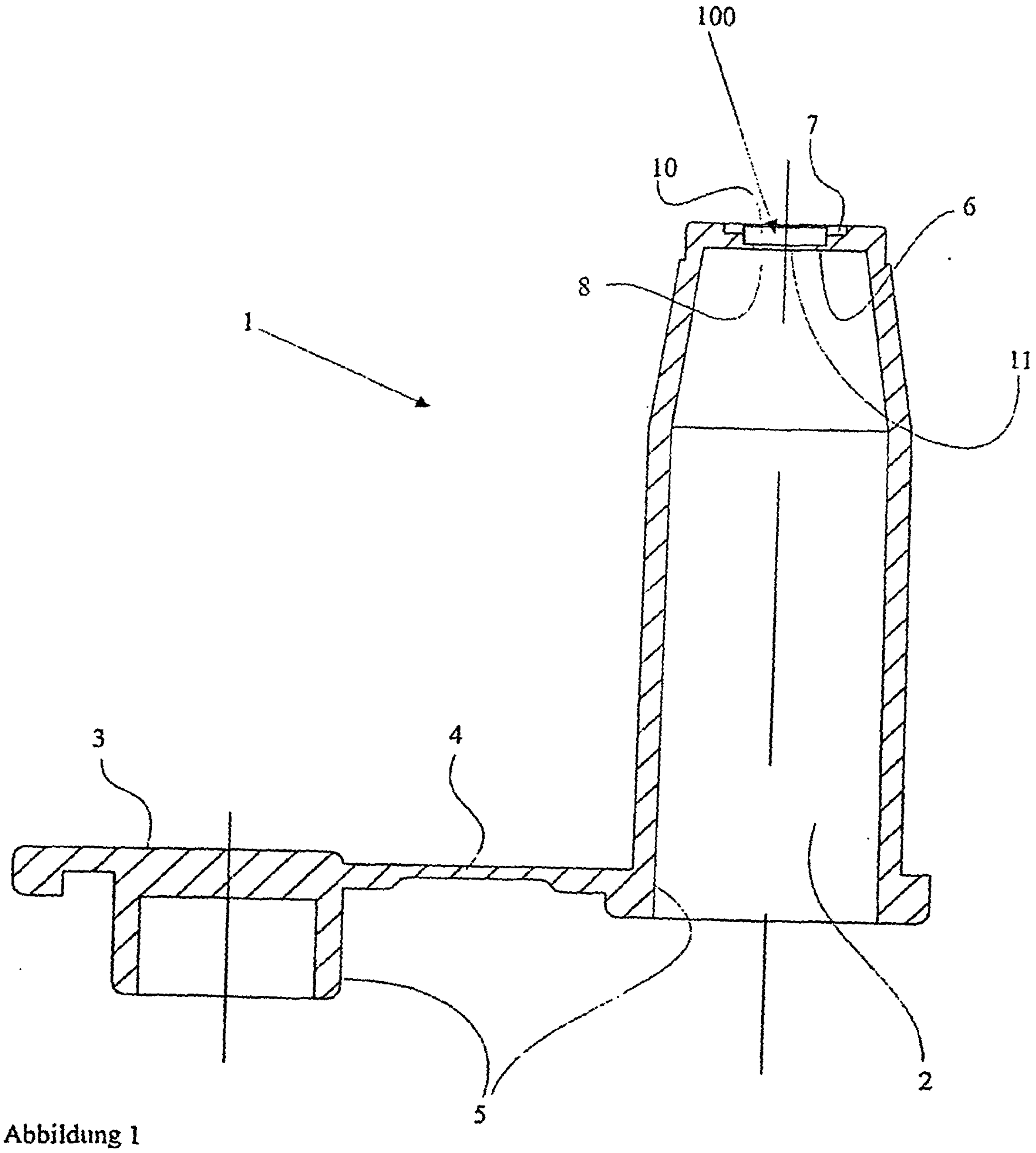


Abbildung 1

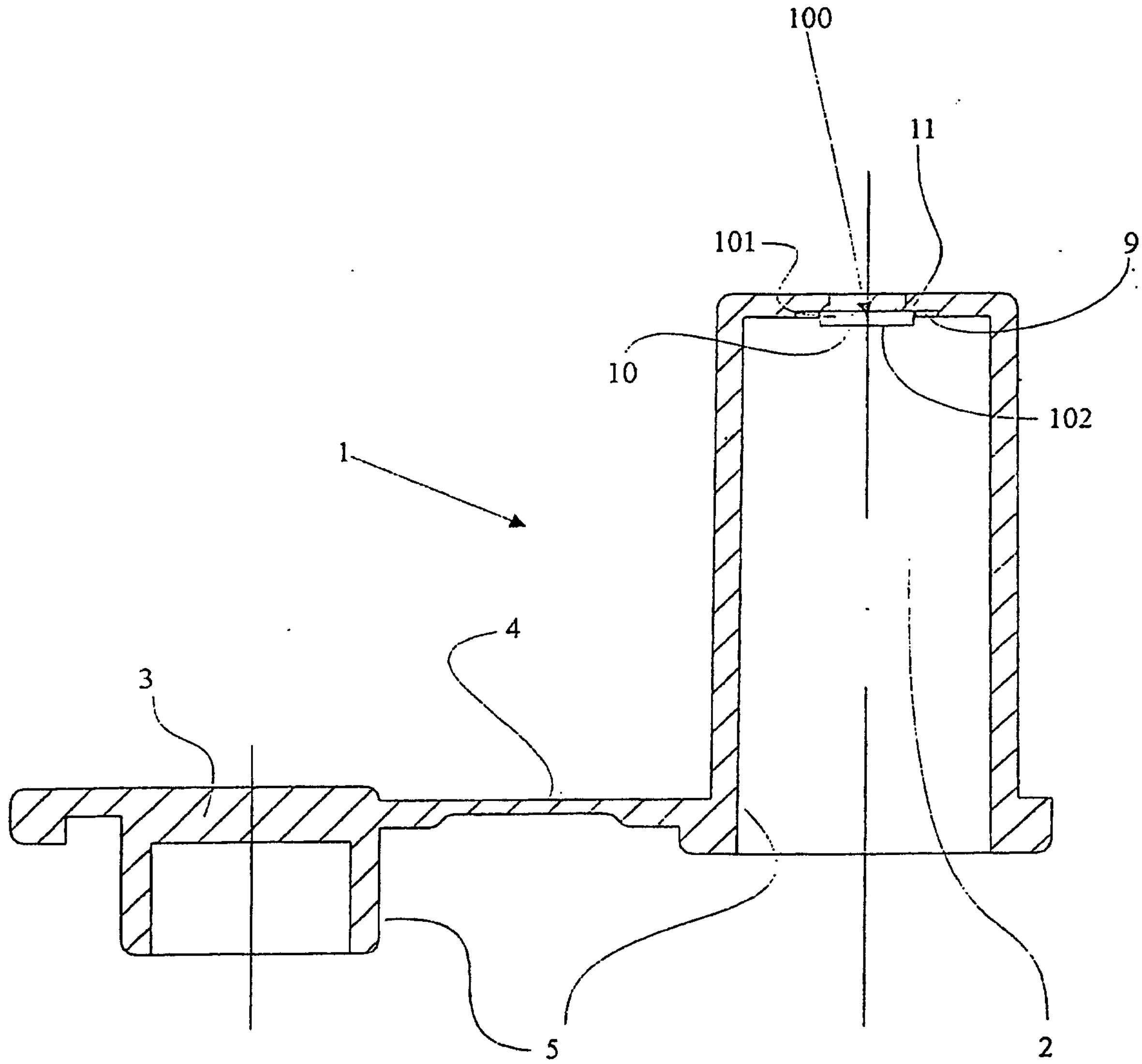


Abbildung 2

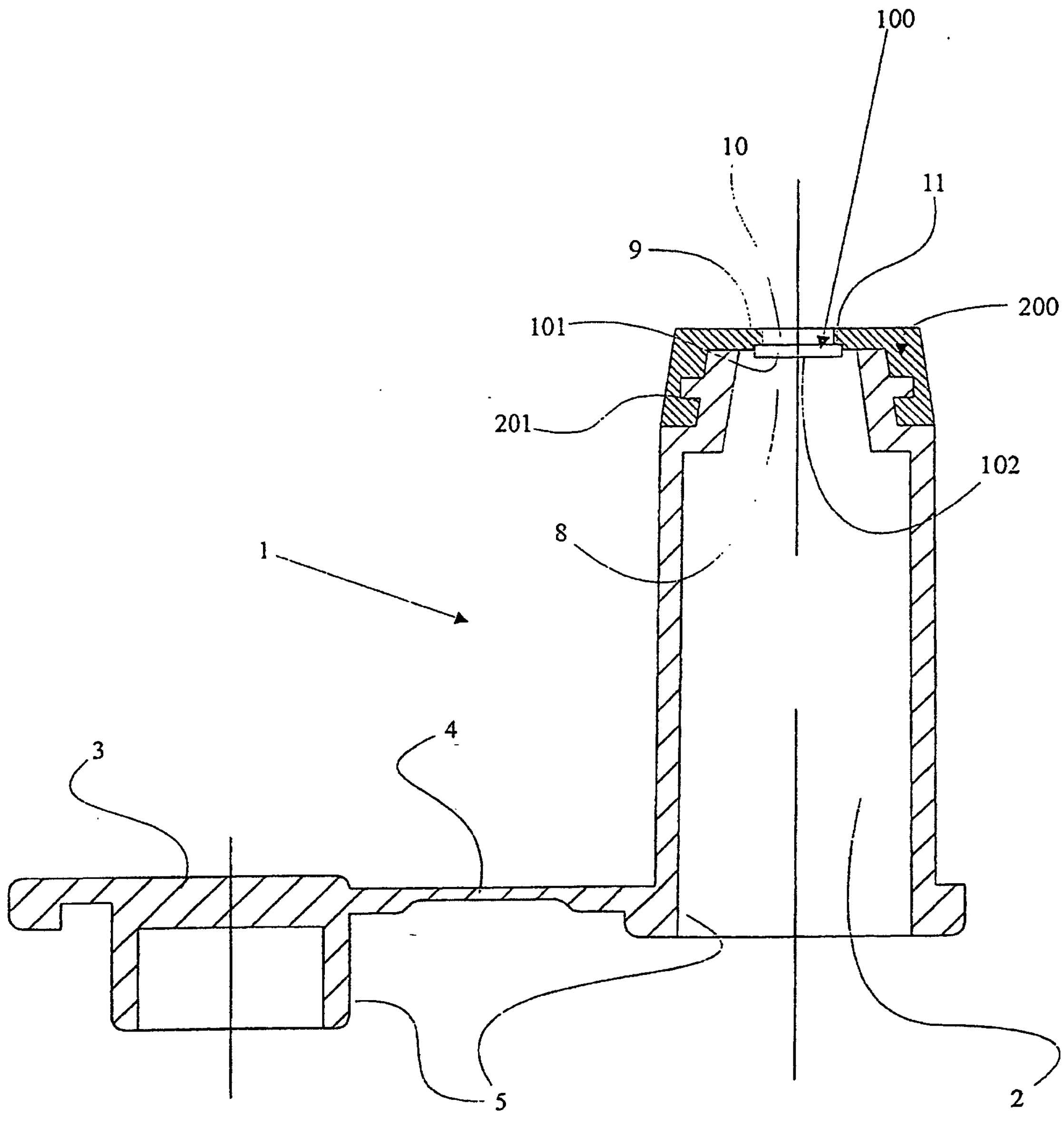


Abbildung 3

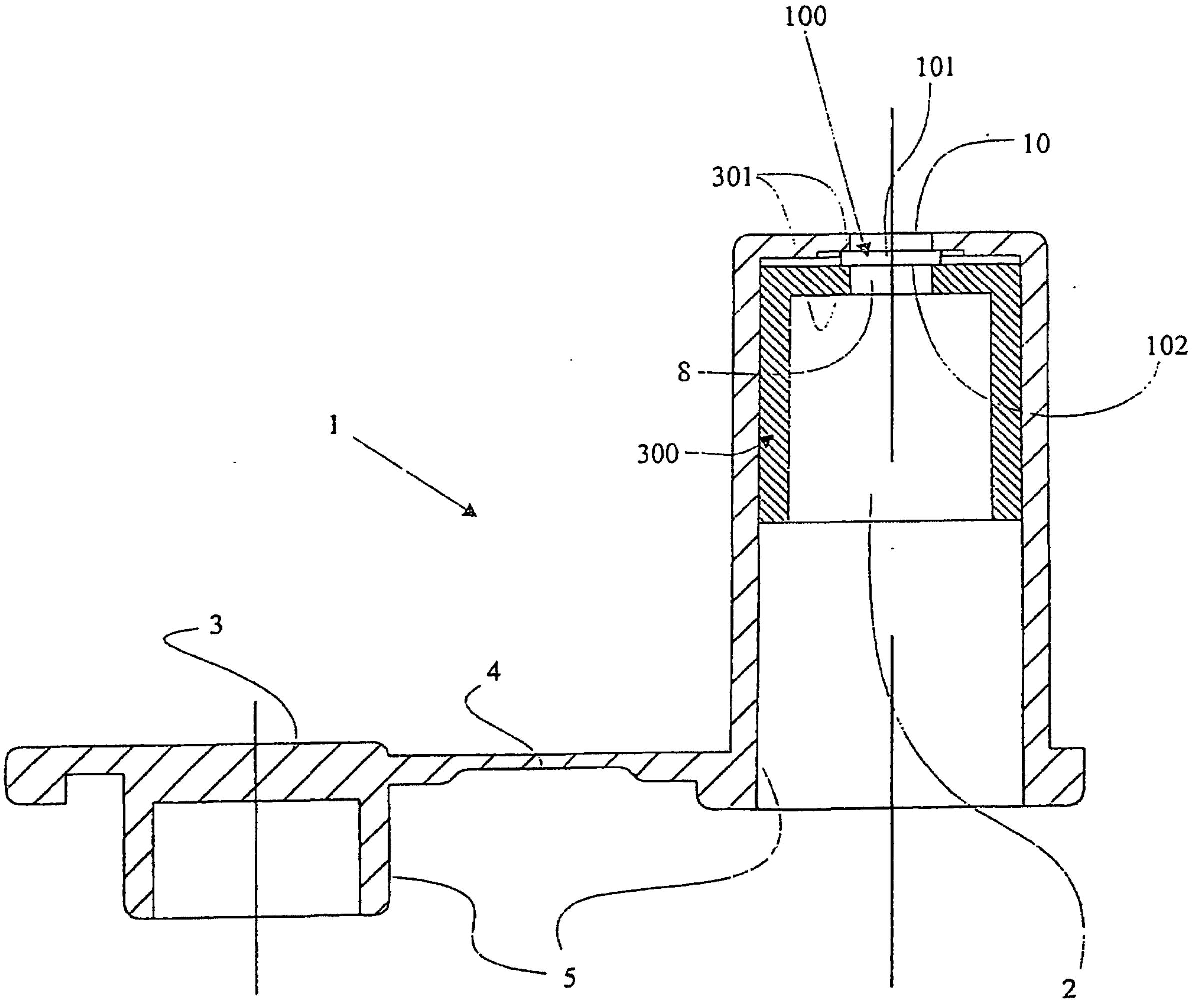


Abbildung 4

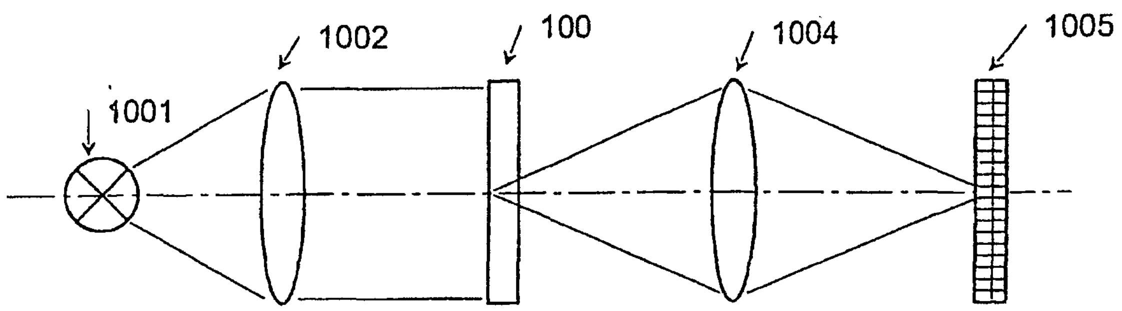


Abbildung 5

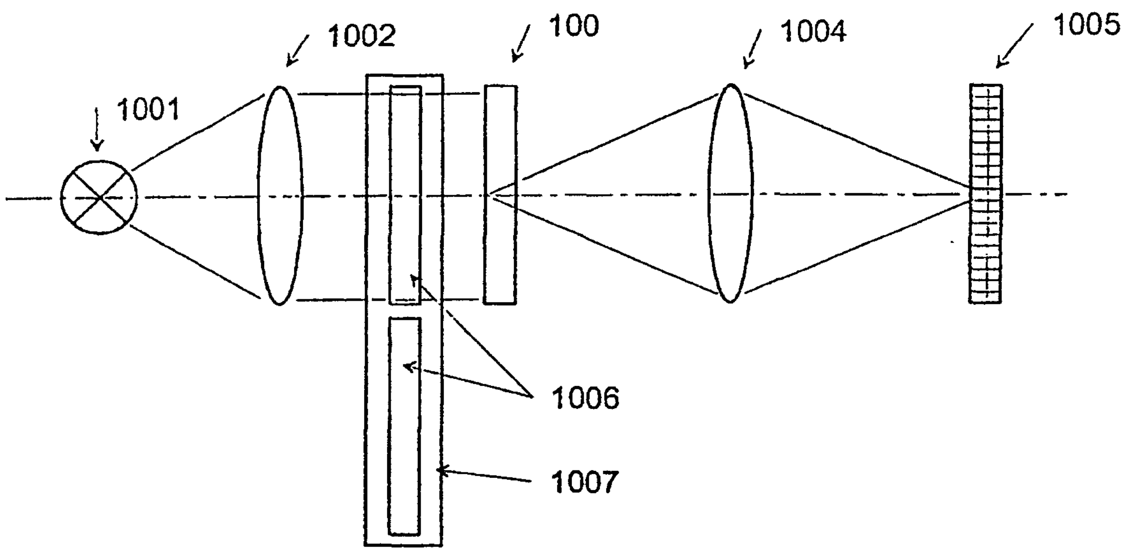


Abbildung 6

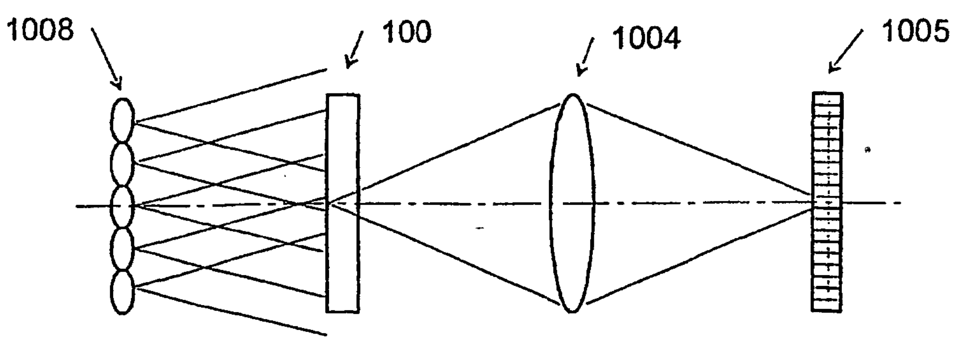


Abbildung 7

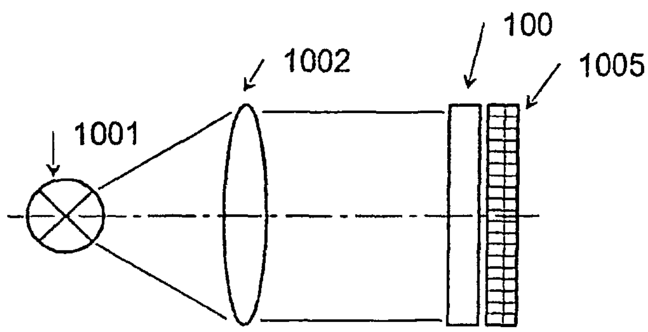


Abbildung 8

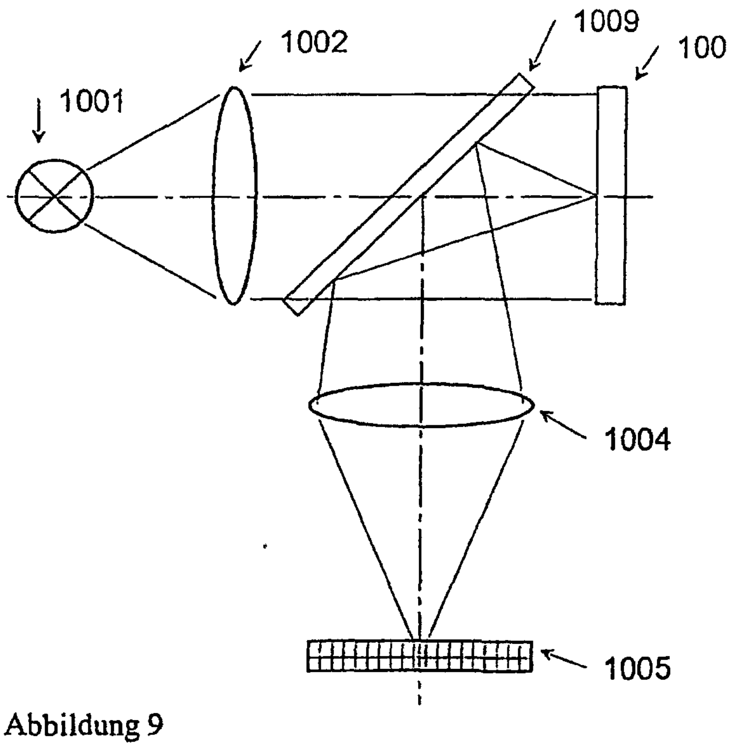


Abbildung 9

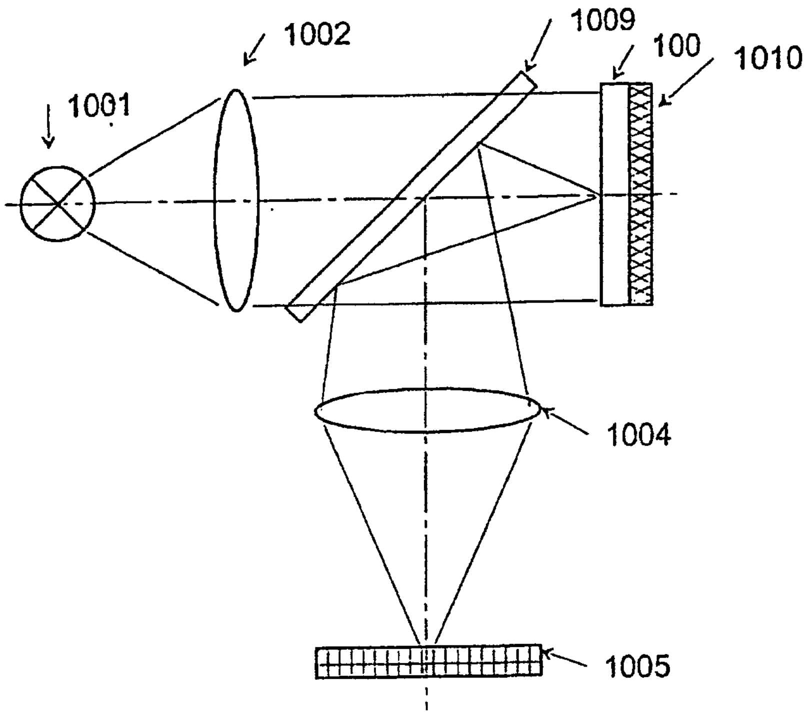


Abbildung 10

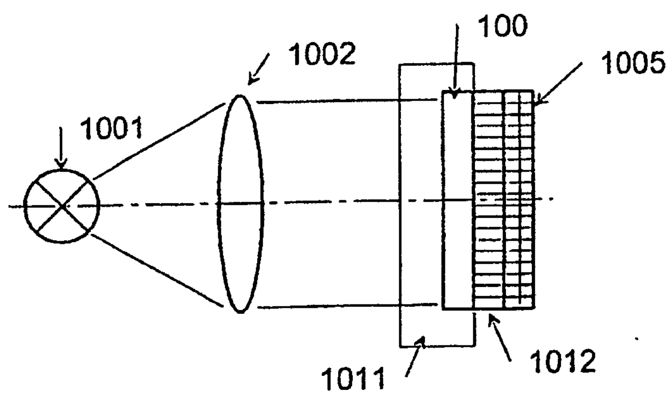


Abbildung 11

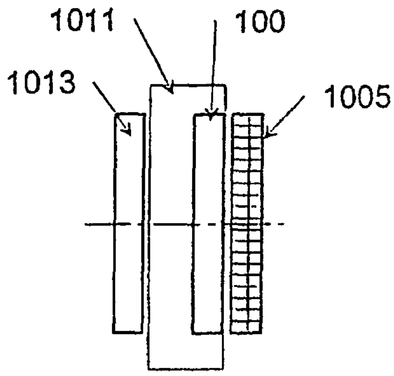


Abbildung 12

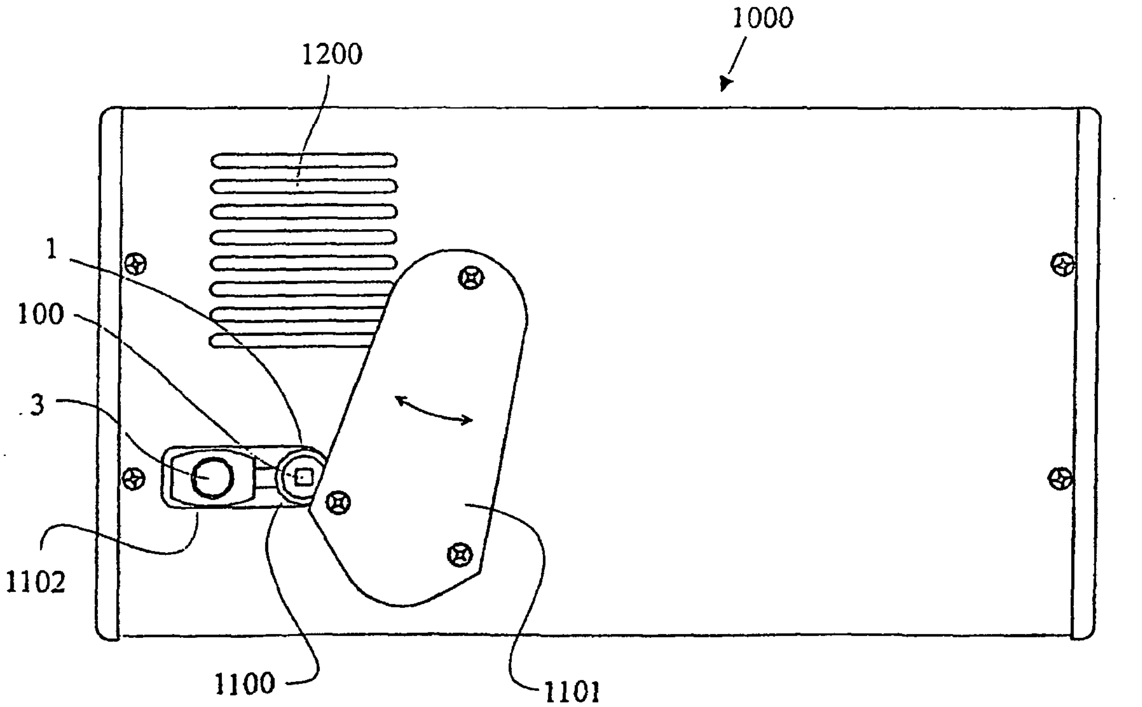


Abbildung 13

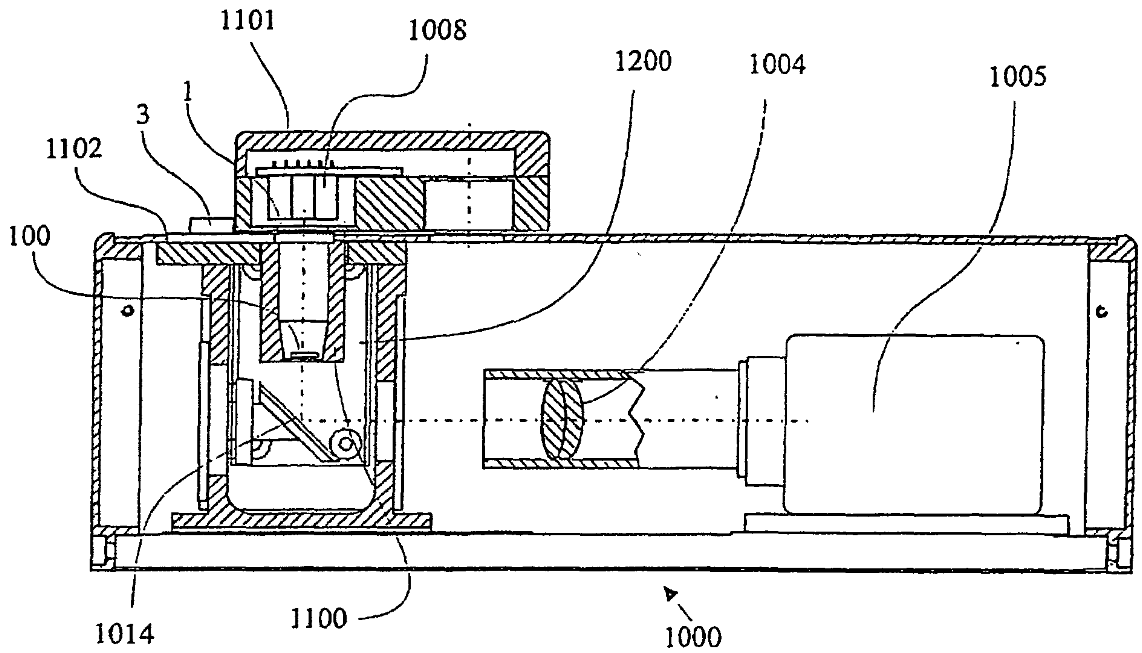


Abbildung 14

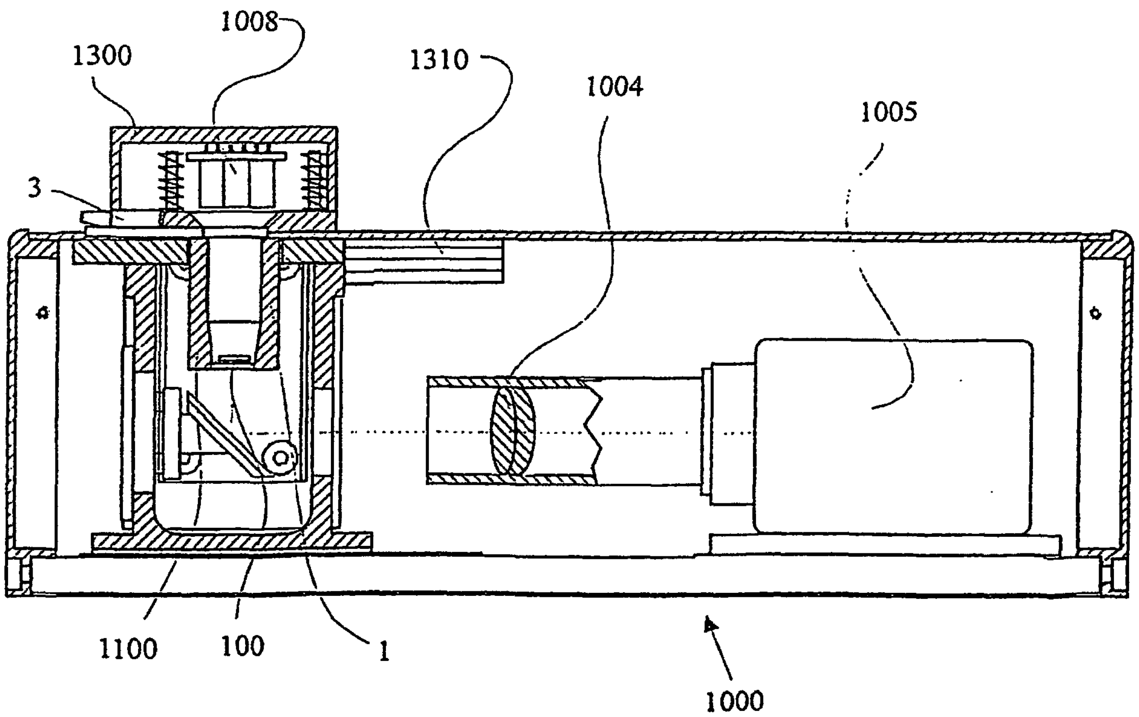


Abbildung 15

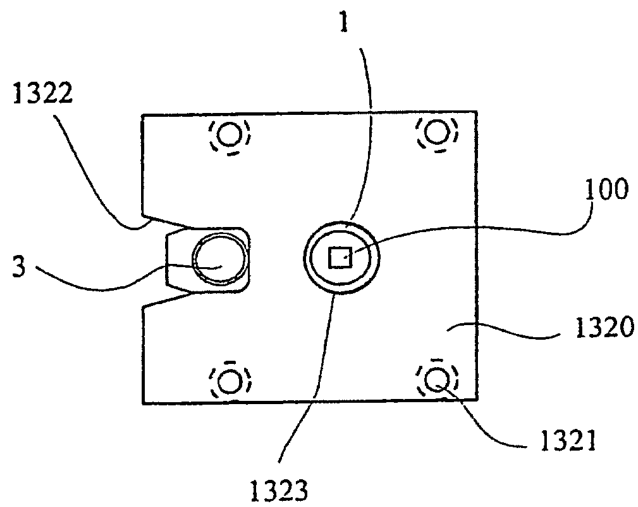


Abbildung 16

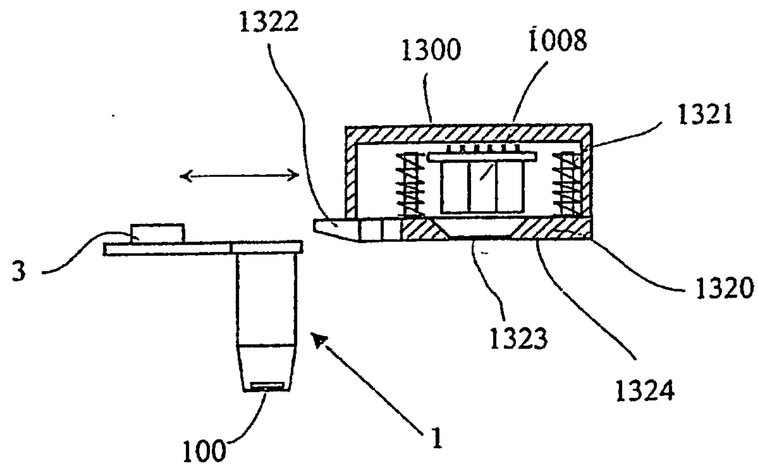


Abbildung 17

M M · M M M

| | | | |
|---|---|---|---|
| G2064A-WT+2 | G2064A+2 | G2064A-WT+4 | G2064A+4 |
| G2064A-WT-2 | G2064A-2 | G2064A-WT-4 | G2064A-4 |
| G1934A-WT+4 | G1934A+4 | G2064A-WT | G2064A |
| G1934A-WT-4 | G1934A-4 | G1934A-WT+2 | G1934A+2 |
| G1934A-WT | G1934A | G1934A-WT-2 | G1934A-2 |
| dT1795-WT+2 | dT1795+2 | dT1795-WT+4 | dT1795+4 |
| dT1795-WT-2 | dT1795-2 | dT1795-WT-4 | dT1795-4 |
| G1749C-WT+4 | G1749C+4 | dT1795-WT | dT1795 |
| G1749C-WT-4 | G1749C-4 | G1749C-WT+2 | G1749C+2 |
| G1749C-WT | G1749C | G1749C-WT-2 | G1749C-2 |

M M

Abbildung 18

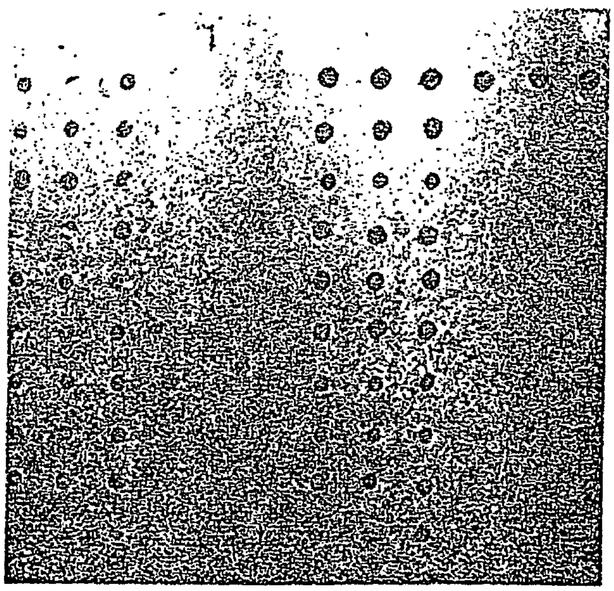

Abbildung 19

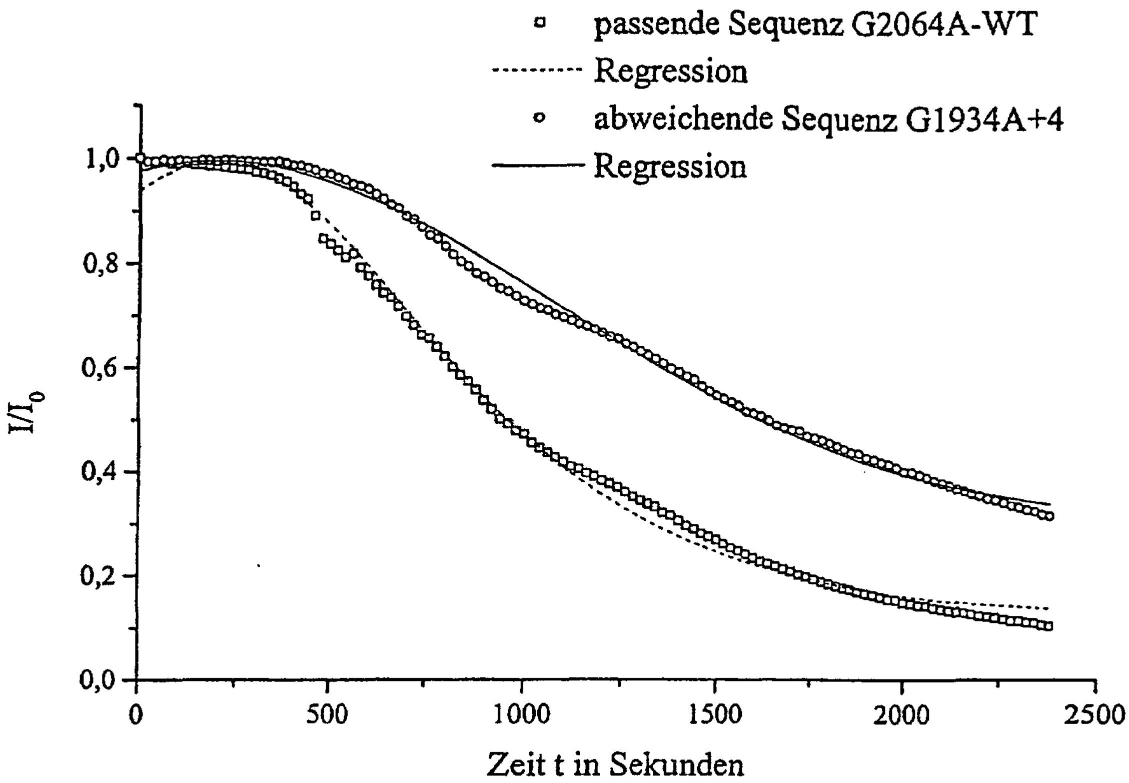


Abbildung 20

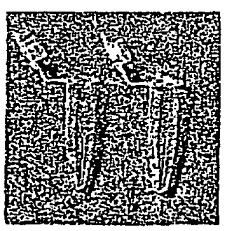

Abbildung 21